# EUROPEAN PATENT APPLICATION

(11) **EP 3 778 640 A1**
(43) Date of publication of application: **17.02.2021**
(21) Application number: 20184006.3
(22) Date of filing: 29.04.2016
(51) Int. Cl.: C07K 16/28, A61P 35/00, A61K 39/395

(54) **MASKED ANTI-CD3 ANTIBODIES AND METHODS OF USE**

(30) Priority: 01.05.2015 US 201562155723 P
(62) Divisional of application: 16724772.5
(71) Applicant: Genentech, Inc., South San Francisco, CA 94080-4990 (US)
(72) Inventor: DENNIS, Mark, S., San Carlos, CA 94070 (US)
(74) Representative: Mewburn Ellis LLP

(57) **Abstract**

The invention provides masked anti-cluster of differentiation 3 (CDS) antibodies and methods of using the same.

SEQUENCE LISTING

<110>  GENENTECH, INC.

<120>  MASKED ANTI-CD3 ANTIBODIES AND METHODS OF USE

<130>  007698434

<140>  Divisional of 16724772.5
<140>  2016-04-29

<150>  16724772.5
<150>  2016-04-29

<150>  US 62/155,723
<151>  2015-05-01

<160>  112

<170>  PatentIn version 3.5

<210>  1
<211>  27
<212>  PRT
<213>  Artificial Sequence

<220>
<223>  Synthetic Construct

<400>  1

Gln Asp Gly Asn Glu Glu Met Gly Gly Ile Thr Gln Thr Pro Tyr Lys
1               5                   10                  15

Val Ser Ile Ser Gly Thr Thr Val Ile Leu Thr
            20                  25

<210>  2
<211>  5
<212>  PRT
<213>  Artificial Sequence

<220>
<223>  Synthetic Construct

<400>  2

Asn Tyr Tyr Ile His
1               5

<210>  3
<211>  17
<212>  PRT
<213>  Artificial Sequence

<220>
<223>  Synthetic Construct

<400>  3

Trp Ile Tyr Pro Gly Asp Gly Asn Thr Lys Tyr Asn Glu Lys Phe Lys
1               5                   10                  15

Gly

<210>  4
<211>  10
<212>  PRT
<213>  Artificial Sequence

<220>
<223>  Synthetic Construct

<400>  4

Asp Ser Tyr Ser Asn Tyr Tyr Phe Asp Tyr
1               5                   10

<210>  5
<211>  17
<212>  PRT
<213>  Artificial Sequence

<220>
<223>  Synthetic Construct

<400>  5

Lys Ser Ser Gln Ser Leu Leu Asn Ser Arg Thr Arg Lys Asn Tyr Leu
1               5                   10                  15

Ala

<210>  6
<211>  7
<212>  PRT
<213>  Artificial Sequence

<220>
<223>  Synthetic Construct

<400>  6

Trp Ala Ser Thr Arg Glu Ser
1               5

<210>  7
<211>  8
<212>  PRT
<213>  Artificial Sequence

<220>
<223>  Synthetic Construct

<400>  7

Thr Gln Ser Phe Ile Leu Arg Thr
1               5

<210>  8
<211>  119
<212>  PRT
<213>  Artificial Sequence

<220>
<223>  Synthetic Construct

<400>  8

Glu Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asn Tyr
            20                  25                  30

Tyr Ile His Trp Val Arg Gln Ala Pro Gly Gln Gly Leu Glu Trp Ile
        35                  40                  45

Gly Trp Ile Tyr Pro Gly Asp Gly Asn Thr Lys Tyr Asn Glu Lys Phe
    50                  55                  60

Lys Gly Arg Ala Thr Leu Thr Ala Asp Thr Ser Thr Ser Thr Ala Tyr
65                  70                  75                  80

Leu Glu Leu Ser Ser Leu Arg Ser Glu Asp Thr Ala Val Tyr Tyr Cys
                85                  90                  95

Ala Arg Asp Ser Tyr Ser Asn Tyr Tyr Phe Asp Tyr Trp Gly Gln Gly
            100                 105                 110

Thr Leu Val Thr Val Ser Ser
        115

<210>  9
<211>  112
<212>  PRT
<213>  Artificial Sequence

<220>
<223>  Synthetic Construct

<400>  9

Asp Ile Val Met Thr Gln Ser Pro Asp Ser Leu Ala Val Ser Leu Gly
1               5                   10                  15

Glu Arg Ala Thr Ile Asn Cys Lys Ser Ser Gln Ser Leu Leu Asn Ser
            20                  25                  30

Arg Thr Arg Lys Asn Tyr Leu Ala Trp Tyr Gln Gln Lys Pro Gly Gln
        35                  40                  45

Pro Pro Lys Leu Leu Ile Tyr Trp Ala Ser Thr Arg Glu Ser Gly Val
    50                  55                  60

Pro Asp Arg Phe Ser Gly Ser Gly Ser Gly Thr Asp Phe Thr Leu Thr
65                  70                  75                  80

Ile Ser Ser Leu Gln Ala Glu Asp Val Ala Val Tyr Tyr Cys Thr Gln
                85                  90                  95

Ser Phe Ile Leu Arg Thr Phe Gly Gln Gly Thr Lys Val Glu Ile Lys
            100                 105                 110

<210>  10
<211>  5
<212>  PRT
<213>  Artificial Sequence

<220>
<223>  Synthetic Construct

<400>  10

Ser Tyr Tyr Ile His
1               5

<210>  11
<211>  17
<212>  PRT
<213>  Artificial Sequence

<220>
<223>  Synthetic Construct

<400>  11

Trp Ile Tyr Pro Glu Asn Asp Asn Thr Lys Tyr Asn Glu Lys Phe Lys
1               5                   10                  15

Asp

<210>  12
<211>  10
<212>  PRT
<213>  Artificial Sequence

<220>
<223>  Synthetic Construct

<220>
<221>  MOD_RES
<222>  (1)..(1)
<223>  X is selected from the group consisting of D, T, and S

<220>
<221>  MOD_RES
<222>  (2)..(2)
<223>  X is selected from the group consisting of G, A, and S

<220>
<221>  MOD_RES
<222>  (5)..(5)
<223>  X is R or N

<220>
<221>  MOD_RES
<222>  (6)..(6)
<223>  X is Y or A

<220>
<221>  MOD_RES
<222>  (7)..(7)
<223>  X is Y or A

<400>  12

Xaa Xaa Tyr Ser Xaa Xaa Xaa Phe Asp Tyr
1               5                   10


<210>  13
<211>  17
<212>  PRT
<213>  Artificial Sequence

<220>
<223>  Synthetic Construct

<400>  13

Lys Ser Ser Gln Ser Leu Leu Asn Ser Arg Thr Arg Lys Asn Tyr Leu
1               5                   10                  15


Ala




<210>  14
<211>  7
<212>  PRT
<213>  Artificial Sequence

<220>
<223>  Synthetic Construct

<400>  14

Trp Thr Ser Thr Arg Lys Ser
1               5

<210>  15
<211>  8
<212>  PRT
<213>  Artificial Sequence

<220>
<223>  Synthetic Construct


<220>
<221>  MOD_RES
<222>  (1)..(1)
<223>  X is K or T

<220>
<221>  MOD_RES
<222>  (2)..(2)
<223>  X is Q or A

<220>
<221>  MOD_RES
<222>  (4)..(4)
<223>  X is F or A

<220>
<221>  MOD_RES
<222>  (5)..(5)
<223>  X is I or A

<400>  15

Xaa Xaa Ser Xaa Xaa Leu Arg Thr
1               5


<210>  16
<211>  10
<212>  PRT
<213>  Artificial Sequence

<220>
<223>  Synthetic Construct

<400>  16

Asp Gly Tyr Ser Arg Tyr Tyr Phe Asp Tyr
1               5                   10


<210>  17
<211>  8
<212>  PRT
<213>  Artificial Sequence

<220>
<223>  Synthetic Construct

<400>  17

Lys Gln Ser Phe Ile Leu Arg Thr
1               5

<210>  18
<211>  119
<212>  PRT
<213>  Artificial Sequence

<220>
<223>  Synthetic Construct

<400>  18

Glu Val Gln Leu Val Gln Ser Gly Ala Glu Val Lys Lys Pro Gly Ala
1               5                   10                  15

Ser Val Lys Val Ser Cys Lys Ala Ser Gly Phe Thr Phe Thr Ser Tyr
            20                  25                  30

Tyr Ile His Trp Val Arg Gln Ala Pro Gly Gln Gly Leu Glu Trp Ile
        35                  40                  45

Gly Trp Ile Tyr Pro Glu Asn Asp Asn Thr Lys Tyr Asn Glu Lys Phe
    50                  55                  60

Lys Asp Arg Val Thr Ile Thr Ala Asp Thr Ser Thr Ser Thr Ala Tyr
65                  70                  75                  80

Leu Glu Leu Ser Ser Leu Arg Ser Glu Asp Thr Ala Val Tyr Tyr Cys
                85                  90                  95

Ala Arg Asp Gly Tyr Ser Arg Tyr Tyr Phe Asp Tyr Trp Gly Gln Gly
            100                 105                 110

Thr Leu Val Thr Val Ser Ser
        115


<210>  19
<211>  112
<212>  PRT
<213>  Artificial Sequence

<220>
<223>  Synthetic Construct

<400>  19

Asp Ile Val Met Thr Gln Ser Pro Asp Ser Leu Ala Val Ser Leu Gly
1               5                   10                  15

Glu Arg Ala Thr Ile Asn Cys Lys Ser Ser Gln Ser Leu Leu Asn Ser
            20                  25                  30

Arg Thr Arg Lys Asn Tyr Leu Ala Trp Tyr Gln Gln Lys Pro Gly Gln
        35                  40                  45

Ser Pro Lys Leu Leu Ile Tyr Trp Thr Ser Thr Arg Lys Ser Gly Val
    50                  55                  60

Pro Asp Arg Phe Ser Gly Ser Gly Ser Gly Thr Asp Phe Thr Leu Thr
65                  70                  75                  80

Ile Ser Ser Leu Gln Ala Glu Asp Val Ala Val Tyr Tyr Cys Lys Gln
                85                  90                  95

Ser Phe Ile Leu Arg Thr Phe Gly Gln Gly Thr Lys Val Glu Ile Lys
            100                 105                 110

<210>  20
<211>  10
<212>  PRT
<213>  Artificial Sequence

<220>
<223>  Synthetic Construct

<400>  20

Gly Tyr Thr Phe Thr Ser Tyr Asn Met His
1               5                   10

<210>  21
<211>  17
<212>  PRT
<213>  Artificial Sequence

<220>
<223>  Synthetic Construct

<400>  21

Ala Ile Tyr Pro Gly Asn Gly Asp Thr Ser Tyr Asn Gln Lys Phe Lys
1               5                   10                  15

Gly

<210>  22
<211>  13
<212>  PRT
<213>  Artificial Sequence

<220>
<223>  Synthetic Construct

<400>  22

Val Val Tyr Tyr Ser Asn Ser Tyr Trp Tyr Phe Asp Val
1               5                   10

<210>  23
<211>  10
<212>  PRT
<213>  Artificial Sequence

<220>
<223>  Synthetic Construct

<400>  23

Arg Ala Ser Ser Ser Val Ser Tyr Met His
1               5                   10


<210>  24
<211>  7
<212>  PRT
<213>  Artificial Sequence

<220>
<223>  Synthetic Construct

<400>  24

Ala Pro Ser Asn Leu Ala Ser
1               5


<210>  25
<211>  9
<212>  PRT
<213>  Artificial Sequence

<220>
<223>  Synthetic Construct

<400>  25

Gln Gln Trp Ser Phe Asn Pro Pro Thr
1               5


<210>  26
<211>  122
<212>  PRT
<213>  Artificial Sequence

<220>
<223>  Synthetic Construct

<400>  26

<210>  27
<211>  106
<212>  PRT
<213>  Artificial Sequence

<220>
<223>  Synthetic Construct

<400>  27

<210>  28
<211>  30
<212>  PRT
<213>  Artificial Sequence

<220>
<223>  Synthetic Construct

<400>  28

Glu Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr
            20                  25                  30

<210>  29
<211>  14
<212>  PRT
<213>  Artificial Sequence

<220>
<223>  Synthetic Construct

<400>  29

Trp Val Arg Gln Ala Pro Gly Gln Gly Leu Glu Trp Ile Gly
1               5                   10

<210>  30
<211>  32
<212>  PRT
<213>  Artificial Sequence

<220>
<223>  Synthetic Construct

<400>  30

Arg Ala Thr Leu Thr Ala Asp Thr Ser Thr Ser Thr Ala Tyr Leu Glu
1               5                   10                  15

Leu Ser Ser Leu Arg Ser Glu Asp Thr Ala Val Tyr Tyr Cys Ala Arg
            20                  25                  30

<210>  31
<211>  11
<212>  PRT
<213>  Artificial Sequence

<220>
<223>  Synthetic Construct

<400>  31

Trp Gly Gln Gly Thr Leu Val Thr Val Ser Ser
1               5                   10

<210>  32
<211>  23
<212>  PRT

<213>  Artificial Sequence

<220>
<223>  Synthetic Construct

<400>  32

Asp Ile Val Met Thr Gln Ser Pro Asp Ser Leu Ala Val Ser Leu Gly
1               5                   10                  15

Glu Arg Ala Thr Ile Asn Cys
            20

<210>  33
<211>  15
<212>  PRT
<213>  Artificial Sequence

<220>
<223>  Synthetic Construct

<400>  33

Trp Tyr Gln Gln Lys Pro Gly Gln Pro Pro Lys Leu Leu Ile Tyr
1               5                   10                  15

<210>  34
<211>  32
<212>  PRT
<213>  Artificial Sequence

<220>
<223>  Synthetic Construct

<400>  34

Gly Val Pro Asp Arg Phe Ser Gly Ser Gly Ser Gly Thr Asp Phe Thr
1               5                   10                  15

Leu Thr Ile Ser Ser Leu Gln Ala Glu Asp Val Ala Val Tyr Tyr Cys
            20                  25                  30

<210>  35
<211>  10
<212>  PRT
<213>  Artificial Sequence

<220>
<223>  Synthetic Construct

<400>  35

Phe Gly Gln Gly Thr Lys Val Glu Ile Lys
1               5                   10

<210>  36
<211>  30

<212>  PRT
<213>  Artificial Sequence

<220>
<223>  Synthetic Construct

<400>  36

Glu Val Gln Leu Val Gln Ser Gly Ala Glu Val Lys Lys Pro Gly Ala
1               5                   10                  15

Ser Val Lys Val Ser Cys Lys Ala Ser Gly Phe Thr Phe Thr
            20                  25                  30

<210>  37
<211>  14
<212>  PRT
<213>  Artificial Sequence

<220>
<223>  Synthetic Construct

<400>  37

Trp Val Arg Gln Ala Pro Gly Gln Gly Leu Glu Trp Ile Gly
1               5                   10

<210>  38
<211>  32
<212>  PRT
<213>  Artificial Sequence

<220>
<223>  Synthetic Construct

<400>  38

Arg Val Thr Ile Thr Ala Asp Thr Ser Thr Ser Thr Ala Tyr Leu Glu
1               5                   10                  15

Leu Ser Ser Leu Arg Ser Glu Asp Thr Ala Val Tyr Tyr Cys Ala Arg
            20                  25                  30

<210>  39
<211>  11
<212>  PRT
<213>  Artificial Sequence

<220>
<223>  Synthetic Construct

<400>  39

Trp Gly Gln Gly Thr Leu Val Thr Val Ser Ser
1               5                   10

<210>  40

<211>  23
<212>  PRT
<213>  Artificial Sequence

<220>
<223>  Synthetic Construct

<400>  40

Asp Ile Val Met Thr Gln Ser Pro Asp Ser Leu Ala Val Ser Leu Gly
1               5                   10                  15

Glu Arg Ala Thr Ile Asn Cys
            20

<210>  41
<211>  15
<212>  PRT
<213>  Artificial Sequence

<220>
<223>  Synthetic Construct

<400>  41

Trp Tyr Gln Gln Lys Pro Gly Gln Ser Pro Lys Leu Leu Ile Tyr
1               5                   10                  15

<210>  42
<211>  32
<212>  PRT
<213>  Artificial Sequence

<220>
<223>  Synthetic Construct

<400>  42

Gly Val Pro Asp Arg Phe Ser Gly Ser Gly Ser Gly Thr Asp Phe Thr
1               5                   10                  15

Leu Thr Ile Ser Ser Leu Gln Ala Glu Asp Val Ala Val Tyr Tyr Cys
            20                  25                  30

<210>  43
<211>  10
<212>  PRT
<213>  Artificial Sequence

<220>
<223>  Synthetic Construct

<400>  43

Phe Gly Gln Gly Thr Lys Val Glu Ile Lys
1               5                   10

<210>  44
<211>  25
<212>  PRT
<213>  Artificial Sequence

<220>
<223>  Synthetic Construct

<400>  44

<210>  45
<211>  14
<212>  PRT
<213>  Artificial Sequence

<220>
<223>  Synthetic Construct

<400>  45

Trp Val Arg Gln Ala Pro Gly Lys Gly Leu Glu Trp Val Gly
1               5                   10

<210>  46
<211>  32
<212>  PRT
<213>  Artificial Sequence

<220>
<223>  Synthetic Construct

<400>  46

Arg Phe Thr Ile Ser Val Asp Lys Ser Lys Asn Thr Leu Tyr Leu Gln
1               5                   10                  15

Met Asn Ser Leu Arg Ala Glu Asp Thr Ala Val Tyr Tyr Cys Ala Arg
            20                  25                  30

<210>  47
<211>  11
<212>  PRT
<213>  Artificial Sequence

<220>
<223>  Synthetic Construct

<400>  47

Trp Gly Gln Gly Thr Leu Val Thr Val Ser Ser
1               5                   10

<210>  48
<211>  23
<212>  PRT
<213>  Artificial Sequence

<220>
<223>  Synthetic Construct

<400>  48

Asp Ile Gln Met Thr Gln Ser Pro Ser Ser Leu Ser Ala Ser Val Gly
1               5                   10                  15

Asp Arg Val Thr Ile Thr Cys
            20

<210>  49
<211>  15
<212>  PRT
<213>  Artificial Sequence

<220>
<223>  Synthetic Construct

<400>  49

Trp Tyr Gln Gln Lys Pro Gly Lys Ala Pro Lys Pro Leu Ile Tyr
1               5                   10                  15

<210>  50
<211>  32
<212>  PRT
<213>  Artificial Sequence

<220>
<223>  Synthetic Construct

<400>  50

Gly Val Pro Ser Arg Phe Ser Gly Ser Gly Ser Gly Thr Asp Phe Thr
1               5                   10                  15

Leu Thr Ile Ser Ser Leu Gln Pro Glu Asp Phe Ala Thr Tyr Tyr Cys
            20                  25                  30

<210>  51
<211>  10
<212>  PRT
<213>  Artificial Sequence

<220>
<223>  Synthetic Construct

<400>  51

Phe Gly Gln Gly Thr Lys Val Glu Ile Lys
1               5                   10

<210>  52
<211>  9
<212>  PRT
<213>  Artificial Sequence

<220>
<223>  Synthetic Construct

<400>  52

Gln Asp Gly Leu Val Pro Arg Gly Ser
1               5


<210>  53
<211>  11
<212>  PRT
<213>  Artificial Sequence

<220>
<223>  Synthetic Construct

<400>  53

Gln Asp Gly Asn Glu Leu Val Pro Arg Gly Ser
1               5                   10


<210>  54
<211>  13
<212>  PRT
<213>  Artificial Sequence

<220>
<223>  Synthetic Construct

<400>  54

Gln Asp Gly Asn Glu Glu Met Leu Val Pro Arg Gly Ser
1               5                   10


<210>  55
<211>  15
<212>  PRT
<213>  Artificial Sequence

<220>
<223>  Synthetic Construct

<400>  55

Gln Asp Gly Asn Glu Glu Met Gly Gly Leu Val Pro Arg Gly Ser
1               5                   10                  15


<210>  56
<211>  16
<212>  PRT
<213>  Artificial Sequence

<220>
<223>  Synthetic Construct

<400>  56

Gln Asp Gly Asn Glu Glu Met Gly Gly Ile Leu Val Pro Arg Gly Ser
1               5                   10                  15


<210>  57
<211>  17
<212>  PRT
<213>  Artificial Sequence

<220>
<223>  Synthetic Construct

<400>  57

Gln Asp Gly Asn Glu Glu Met Gly Gly Ile Thr Leu Val Pro Arg Gly
1               5                   10                  15


Ser




<210>  58
<211>  18
<212>  PRT
<213>  Artificial Sequence

<220>
<223>  Synthetic Construct

<400>  58

Gln Asp Gly Asn Glu Glu Met Gly Gly Ile Thr Gln Leu Val Pro Arg
1               5                   10                  15


Gly Ser




<210>  59
<211>  19
<212>  PRT
<213>  Artificial Sequence

<220>
<223>  Synthetic Construct

<400>  59

Gln Asp Gly Asn Glu Glu Met Gly Gly Ile Thr Gln Thr Leu Val Pro
1               5                   10                  15


Arg Gly Ser

<210>  60
<211>  20
<212>  PRT
<213>  Artificial Sequence

<220>
<223>  Synthetic Construct

<400>  60

Gln Asp Gly Asn Glu Glu Met Gly Gly Ile Thr Gln Thr Pro Leu Val
1               5                   10                  15


Pro Arg Gly Ser
            20


<210>  61
<211>  21
<212>  PRT
<213>  Artificial Sequence

<220>
<223>  Synthetic Construct

<400>  61

Gln Asp Gly Asn Glu Glu Met Gly Gly Ile Thr Gln Thr Pro Tyr Leu
1               5                   10                  15


Val Pro Arg Gly Ser
            20


<210>  62
<211>  27
<212>  PRT
<213>  Artificial Sequence

<220>
<223>  Synthetic Construct

<400>  62

Gln Asp Gly Asn Glu Glu Met Gly Gly Ile Thr Gln Thr Pro Tyr Lys
1               5                   10                  15


Val Ser Ile Ser Gly Leu Val Pro Arg Gly Ser
            20                  25


<210>  63
<211>  31
<212>  PRT
<213>  Artificial Sequence

<220>
<223>  Synthetic Construct

<400>  63

Gln Asp Gly Asn Glu Glu Met Gly Gly Ile Thr Gln Thr Pro Tyr Lys
1               5                   10                  15

Val Ser Ile Ser Gly Thr Thr Val Ile Leu Val Pro Arg Gly Ser
            20                  25                  30

<210>  64
<211>  33
<212>  PRT
<213>  Artificial Sequence

<220>
<223>  Synthetic Construct

<400>  64

Gln Asp Gly Asn Glu Glu Met Gly Gly Ile Thr Gln Thr Pro Tyr Lys
1               5                   10                  15

Val Ser Ile Ser Gly Thr Thr Val Ile Leu Thr Leu Val Pro Arg Gly
            20                  25                  30

Ser

<210>  65
<211>  7
<212>  PRT
<213>  Artificial Sequence

<220>
<223>  Synthetic Construct

<400>  65

Gln Leu Val Pro Arg Gly Ser
1               5

<210>  66
<211>  8
<212>  PRT
<213>  Artificial Sequence

<220>
<223>  Synthetic Construct

<400>  66

Gln Asp Leu Val Pro Arg Gly Ser
1               5

<210>  67
<211>  9
<212>  PRT
<213>  Artificial Sequence

<220>
<223>  Synthetic Construct

<400>  67

Gln Asp Gly Leu Val Pro Arg Gly Ser
1               5


<210>  68
<211>  10
<212>  PRT
<213>  Artificial Sequence

<220>
<223>  Synthetic Construct

<400>  68

Gln Asp Gly Asn Leu Val Pro Arg Gly Ser
1               5                   10


<210>  69
<211>  11
<212>  PRT
<213>  Artificial Sequence

<220>
<223>  Synthetic Construct

<400>  69

Gln Asp Gly Asn Glu Leu Val Pro Arg Gly Ser
1               5                   10


<210>  70
<211>  12
<212>  PRT
<213>  Artificial Sequence

<220>
<223>  Synthetic Construct

<400>  70

Gln Asp Gly Asn Glu Glu Leu Val Pro Arg Gly Ser
1               5                   10


<210>  71
<211>  13
<212>  PRT
<213>  Artificial Sequence

<220>
<223>  Synthetic Construct

<400>  71

Gln Asp Gly Asn Glu Glu Met Leu Val Pro Arg Gly Ser

1                   5                        10


<210>  72
<211>  14
<212>  PRT
<213>  Artificial Sequence

<220>
<223>  Synthetic Construct

<400>  72

Gln Asp Gly Asn Glu Glu Met Gly Leu Val Pro Arg Gly Ser
1                   5                        10


<210>  73
<211>  15
<212>  PRT
<213>  Artificial Sequence

<220>
<223>  Synthetic Construct

<400>  73

Gln Asp Gly Asn Glu Glu Met Gly Gly Leu Val Pro Arg Gly Ser
1                   5                        10                       15


<210>  74
<211>  16
<212>  PRT
<213>  Artificial Sequence

<220>
<223>  Synthetic Construct

<400>  74

Gln Asp Gly Asn Glu Glu Met Gly Gly Ile Leu Val Pro Arg Gly Ser
1                   5                        10                       15


<210>  75
<211>  17
<212>  PRT
<213>  Artificial Sequence

<220>
<223>  Synthetic Construct

<400>  75

Gln Asp Gly Asn Glu Glu Met Gly Gly Ile Thr Leu Val Pro Arg Gly
1                   5                        10                       15


Ser

<210>  76
<211>  18
<212>  PRT
<213>  Artificial Sequence

<220>
<223>  Synthetic Construct

<400>  76

Gln Asp Gly Asn Glu Glu Met Gly Gly Ile Thr Gln Leu Val Pro Arg
1               5                   10                  15

Gly Ser

<210>  77
<211>  19
<212>  PRT
<213>  Artificial Sequence

<220>
<223>  Synthetic Construct

<400>  77

Gln Asp Gly Asn Glu Glu Met Gly Gly Ile Thr Gln Thr Leu Val Pro
1               5                   10                  15

Arg Gly Ser

<210>  78
<211>  20
<212>  PRT
<213>  Artificial Sequence

<220>
<223>  Synthetic Construct

<400>  78

Gln Asp Gly Asn Glu Glu Met Gly Gly Ile Thr Gln Thr Pro Leu Val
1               5                   10                  15

Pro Arg Gly Ser
            20

<210>  79
<211>  27
<212>  PRT
<213>  Artificial Sequence

<220>
<223>  Synthetic Construct

<400>  79

Gln Asp Gly Ser Gly Gly Ser Gly Gly Ser Gly Gly Ser Gly Gly Ser
1               5                   10                  15

Gly Gly Ser Gly Gly Leu Val Pro Arg Gly Ser
            20                  25

<210>  80
<211>  27
<212>  PRT
<213>  Artificial Sequence

<220>
<223>  Synthetic Construct

<400>  80

Gln Asp Gly Asn Ser Gly Gly Ser Gly Gly Ser Gly Gly Ser Gly Gly
1               5                   10                  15

Ser Gly Gly Ser Gly Leu Val Pro Arg Gly Ser
            20                  25

<210>  81
<211>  27
<212>  PRT
<213>  Artificial Sequence

<220>
<223>  Synthetic Construct

<400>  81

Gln Asp Gly Asn Glu Ser Gly Gly Ser Gly Gly Ser Gly Gly Ser Gly
1               5                   10                  15

Gly Ser Gly Gly Ser Leu Val Pro Arg Gly Ser
            20                  25

<210>  82
<211>  27
<212>  PRT
<213>  Artificial Sequence

<220>
<223>  Synthetic Construct

<400>  82

Gln Asp Gly Asn Glu Glu Ser Gly Gly Ser Gly Gly Ser Gly Gly Ser
1               5                   10                  15

Gly Gly Ser Gly Gly Leu Val Pro Arg Gly Ser
            20                  25

<210>  83
<211>  27
<212>  PRT
<213>  Artificial Sequence

<220>
<223>  Synthetic Construct

<400>  83

Gln Asp Gly Asn Glu Glu Met Ser Gly Gly Ser Gly Gly Ser Gly Gly
1               5                   10                  15

Ser Gly Gly Ser Gly Leu Val Pro Arg Gly Ser
            20                  25

<210>  84
<211>  27
<212>  PRT
<213>  Artificial Sequence

<220>
<223>  Synthetic Construct

<400>  84

Gln Asp Gly Asn Glu Glu Met Gly Ser Gly Gly Ser Gly Gly Ser Gly
1               5                   10                  15

Gly Ser Gly Gly Ser Leu Val Pro Arg Gly Ser
            20                  25

<210>  85
<211>  27
<212>  PRT
<213>  Artificial Sequence

<220>
<223>  Synthetic Construct

<400>  85

Gln Asp Gly Asn Glu Glu Met Gly Gly Ser Gly Gly Ser Gly Gly Ser
1               5                   10                  15

Gly Gly Ser Gly Gly Leu Val Pro Arg Gly Ser
            20                  25

<210>  86
<211>  27
<212>  PRT
<213>  Artificial Sequence

<220>
<223>  Synthetic Construct

<400>  86

Gln Asp Gly Asn Glu Glu Met Gly Gly Ile Gly Gly Ser Gly Gly Ser
1               5                   10                  15

Gly Gly Ser Gly Gly Leu Val Pro Arg Gly Ser
            20                  25

<210>  87
<211>  27
<212>  PRT
<213>  Artificial Sequence

<220>
<223>  Synthetic Construct

<400>  87

Gln Asp Gly Asn Glu Glu Met Gly Gly Ile Thr Gly Gly Ser Gly Gly
1               5                   10                  15

Ser Gly Gly Ser Gly Leu Val Pro Arg Gly Ser
            20                  25

<210>  88
<211>  27
<212>  PRT
<213>  Artificial Sequence

<220>
<223>  Synthetic Construct

<400>  88

Gln Asp Gly Asn Glu Glu Met Gly Gly Ile Thr Gln Gly Ser Gly Gly
1               5                   10                  15

Ser Gly Gly Ser Gly Leu Val Pro Arg Gly Ser
            20                  25

<210>  89
<211>  12
<212>  PRT
<213>  Artificial Sequence

<220>
<223>  Synthetic Construct

<400>  89

Gln Asp Gly Asn Glu Glu Met Ser Ser Gly Gly Ser
1               5                   10

<210>  90
<211>  13
<212>  PRT
<213>  Artificial Sequence

<220>
<223>  Synthetic Construct

<400>  90

Gln Asp Gly Asn Glu Glu Met Ser Ser Gly Gly Gly Ser
1               5                   10


<210>  91
<211>  14
<212>  PRT
<213>  Artificial Sequence

<220>
<223>  Synthetic Construct

<400>  91

Gln Asp Gly Asn Glu Glu Met Ser Ser Gly Gly Ser Gly Gly
1               5                   10


<210>  92
<211>  15
<212>  PRT
<213>  Artificial Sequence

<220>
<223>  Synthetic Construct

<400>  92

Gln Asp Gly Asn Glu Glu Met Ser Ser Gly Gly Ser Gly Gly Ser
1               5                   10                  15


<210>  93
<211>  16
<212>  PRT
<213>  Artificial Sequence

<220>
<223>  Synthetic Construct

<400>  93

Gln Asp Gly Asn Glu Glu Met Ser Ser Gly Gly Ser Gly Gly Ser Gly
1               5                   10                  15


<210>  94
<211>  17
<212>  PRT
<213>  Artificial Sequence

<220>
<223>  Synthetic Construct

<400>  94

Gln Asp Gly Asn Glu Glu Met Ser Ser Gly Gly Ser Gly Gly Ser Gly

1                   5                   10                  15

Gly

<210>  95
<211>  12
<212>  PRT
<213>  Artificial Sequence

<220>
<223>  Synthetic Construct

<400>  95

Gln Asp Gly Ser Gly Gly Gly Ser Gly Gly Gly Ser
1               5                   10

<210>  96
<211>  9
<212>  PRT
<213>  Artificial Sequence

<220>
<223>  Synthetic Construct

<400>  96

Gln Asp Gly Asn Ser Gly Gly Gly Ser
1               5

<210>  97
<211>  10
<212>  PRT
<213>  Artificial Sequence

<220>
<223>  Synthetic Construct

<400>  97

Gln Asp Gly Asn Ser Gly Gly Gly Ser Gly
1               5                   10

<210>  98
<211>  12
<212>  PRT
<213>  Artificial Sequence

<220>
<223>  Synthetic Construct

<400>  98

Gln Asp Gly Asn Glu Ser Gly Gly Gly Ser Gly Gly
1               5                   10

<210>  99
<211>  12
<212>  PRT
<213>  Artificial Sequence

<220>
<223>  Synthetic Construct

<400>  99

Gln Asp Gly Asn Glu Glu Ser Gly Gly Gly Ser Gly
1               5                   10


<210>  100
<211>  12
<212>  PRT
<213>  Artificial Sequence

<220>
<223>  Synthetic Construct

<400>  100

Gln Asp Gly Asn Glu Glu Met Gly Gly Ser Gly Gly
1               5                   10


<210>  101
<211>  14
<212>  PRT
<213>  Artificial Sequence

<220>
<223>  Synthetic Construct

<400>  101

Gln Asp Gly Asn Glu Glu Met Gly Gly Ser Gly Gly Ser Gly
1               5                   10


<210>  102
<211>  15
<212>  PRT
<213>  Artificial Sequence

<220>
<223>  Synthetic Construct

<400>  102

Gln Asp Gly Asn Glu Glu Met Gly Gly Ser Gly Gly Ser Gly Gly
1               5                   10                  15


<210>  103
<211>  16
<212>  PRT
<213>  Artificial Sequence

<220>
<223>  Synthetic Construct

<400>  103

Gln Asp Gly Asn Glu Glu Met Gly Gly Ser Gly Gly Ser Gly Gly Ser
1               5                   10                  15


<210>  104
<211>  8
<212>  PRT
<213>  Artificial Sequence

<220>
<223>  Synthetic Construct

<400>  104

Lys Gln Ser Phe Ala Leu Arg Thr
1               5


<210>  105
<211>  8
<212>  PRT
<213>  Artificial Sequence

<220>
<223>  Synthetic Construct

<400>  105

Lys Ala Ser Phe Ile Leu Arg Thr
1               5


<210>  106
<211>  8
<212>  PRT
<213>  Artificial Sequence

<220>
<223>  Synthetic Construct

<400>  106

Lys Gln Ser Ala Ile Leu Arg Thr
1               5


<210>  107
<211>  119
<212>  PRT
<213>  Artificial Sequence

<220>
<223>  Synthetic Construct

<400>  107

Glu Val Gln Leu Val Gln Ser Gly Ala Glu Val Lys Lys Pro Gly Ala
1               5                   10                  15

Ser Val Lys Val Ser Cys Lys Ala Ser Gly Phe Thr Phe Thr Ser Tyr
            20                  25                  30

Tyr Ile His Trp Val Arg Gln Ala Pro Gly Gln Gly Leu Glu Trp Ile
        35                  40                  45

Gly Trp Ile Tyr Pro Glu Asn Asp Asn Thr Lys Tyr Asn Glu Lys Phe
    50                  55                  60

Lys Asp Arg Val Thr Ile Thr Ala Asp Thr Ser Thr Ser Thr Ala Tyr
65                  70                  75                  80

Leu Glu Leu Ser Ser Leu Arg Ser Glu Asp Thr Ala Val Tyr Tyr Cys
                85                  90                  95

Ala Arg Asp Gly Tyr Ser Arg Tyr Tyr Phe Asp Tyr Trp Gly Gln Gly
            100                 105                 110

Thr Leu Val Thr Val Ser Ser
        115

<210> 108
<211> 112
<212> PRT
<213> Artificial Sequence

<220>
<223> Synthetic Construct

<400> 108

Asp Ile Val Met Thr Gln Ser Pro Asp Ser Leu Ala Val Ser Leu Gly
1               5                   10                  15

Glu Arg Ala Thr Ile Asn Cys Lys Ser Ser Gln Ser Leu Leu Asn Ser
            20                  25                  30

Arg Thr Arg Lys Asn Tyr Leu Ala Trp Tyr Gln Gln Lys Pro Gly Gln
        35                  40                  45

Ser Pro Lys Leu Leu Ile Tyr Trp Thr Ser Thr Arg Lys Ser Gly Val
    50                  55                  60

Pro Asp Arg Phe Ser Gly Ser Gly Ser Gly Thr Asp Phe Thr Leu Thr
65                  70                  75                  80

Ile Ser Ser Leu Gln Ala Glu Asp Val Ala Val Tyr Tyr Cys Lys Gln
                85                  90                  95

Ser Phe Ala Leu Arg Thr Phe Gly Gln Gly Thr Lys Val Glu Ile Lys

            100                 105                 110

<210> 109
<211> 119
<212> PRT
<213> Artificial Sequence

<220>
<223> Synthetic Construct

<400> 109

Glu Val Gln Leu Val Gln Ser Gly Ala Glu Val Lys Lys Pro Gly Ala
1               5                   10                  15

Ser Val Lys Val Ser Cys Lys Ala Ser Gly Phe Thr Phe Thr Ser Tyr
            20                  25                  30

Tyr Ile His Trp Val Arg Gln Ala Pro Gly Gln Gly Leu Glu Trp Ile
        35                  40                  45

Gly Trp Ile Tyr Pro Glu Asn Asp Asn Thr Lys Tyr Asn Glu Lys Phe
    50                  55                  60

Lys Asp Arg Val Thr Ile Thr Ala Asp Thr Ser Thr Ser Thr Ala Tyr
65                  70                  75                  80

Leu Glu Leu Ser Ser Leu Arg Ser Glu Asp Thr Ala Val Tyr Tyr Cys
                85                  90                  95

Ala Arg Asp Gly Tyr Ser Arg Tyr Tyr Phe Asp Tyr Trp Gly Gln Gly
            100                 105                 110

Thr Leu Val Thr Val Ser Ser
        115

<210> 110
<211> 112
<212> PRT
<213> Artificial Sequence

<220>
<223> Synthetic Construct

<400> 110

Asp Ile Val Met Thr Gln Ser Pro Asp Ser Leu Ala Val Ser Leu Gly
1               5                   10                  15

Glu Arg Ala Thr Ile Asn Cys Lys Ser Ser Gln Ser Leu Leu Asn Ser
            20                  25                  30

Arg Thr Arg Lys Asn Tyr Leu Ala Trp Tyr Gln Gln Lys Pro Gly Gln
        35                  40                  45

Ser Pro Lys Leu Leu Ile Tyr Trp Thr Ser Thr Arg Lys Ser Gly Val
    50                  55                  60

Pro Asp Arg Phe Ser Gly Ser Gly Ser Gly Thr Asp Phe Thr Leu Thr
65                  70                  75                  80

Ile Ser Ser Leu Gln Ala Glu Asp Val Ala Val Tyr Tyr Cys Lys Ala
                85                  90                  95

Ser Phe Ile Leu Arg Thr Phe Gly Gln Gly Thr Lys Val Glu Ile Lys
            100                 105                 110

<210>  111
<211>  119
<212>  PRT
<213>  Artificial Sequence

<220>
<223>  Synthetic Construct

<400>  111

Glu Val Gln Leu Val Gln Ser Gly Ala Glu Val Lys Lys Pro Gly Ala
1               5                   10                  15

Ser Val Lys Val Ser Cys Lys Ala Ser Gly Phe Thr Phe Thr Ser Tyr
            20                  25                  30

Tyr Ile His Trp Val Arg Gln Ala Pro Gly Gln Gly Leu Glu Trp Ile
        35                  40                  45

Gly Trp Ile Tyr Pro Glu Asn Asp Asn Thr Lys Tyr Asn Glu Lys Phe
    50                  55                  60

Lys Asp Arg Val Thr Ile Thr Ala Asp Thr Ser Thr Ser Thr Ala Tyr
65                  70                  75                  80

Leu Glu Leu Ser Ser Leu Arg Ser Glu Asp Thr Ala Val Tyr Tyr Cys
                85                  90                  95

Ala Arg Asp Gly Tyr Ser Arg Tyr Tyr Phe Asp Tyr Trp Gly Gln Gly
            100                 105                 110

Thr Leu Val Thr Val Ser Ser
        115

<210>  112

<211>  112
<212>  PRT
<213>  Artificial Sequence

<220>
<223>  Synthetic Construct

<400>  112

Asp Ile Val Met Thr Gln Ser Pro Asp Ser Leu Ala Val Ser Leu Gly
1               5                   10                  15

Glu Arg Ala Thr Ile Asn Cys Lys Ser Ser Gln Ser Leu Leu Asn Ser
            20                  25                  30

Arg Thr Arg Lys Asn Tyr Leu Ala Trp Tyr Gln Gln Lys Pro Gly Gln
        35                  40                  45

Ser Pro Lys Leu Leu Ile Tyr Trp Thr Ser Thr Arg Lys Ser Gly Val
    50                  55                  60

Pro Asp Arg Phe Ser Gly Ser Gly Ser Gly Thr Asp Phe Thr Leu Thr
65                  70                  75                  80

Ile Ser Ser Leu Gln Ala Glu Asp Val Ala Val Tyr Tyr Cys Lys Gln
                85                  90                  95

Ser Ala Ile Leu Arg Thr Phe Gly Gln Gly Thr Lys Val Glu Ile Lys
            100                 105                 110

## Description

### SEQUENCE LISTING

The instant application contains a Sequence Listing which has been submitted electronically in ASCII format and is hereby incorporated by reference in its entirety. Said ASCII copy, created on April 25, 2016, is named 50474-063WO2_Sequence_Listing_4_25_16_ST25 and is 39,917 bytes in size.

### FIELD OF THE INVENTION

The present invention relates to masked anti-cluster of differentiation 3 (CD3) antibodies and methods of using the same.

### BACKGROUND

Cell proliferative disorders, such as cancer, are characterized by the uncontrolled growth of cell subpopulations. They are the leading cause of death in the developed world and the second leading cause of death in developing countries, with over 12 million new cancer cases diagnosed and 7 million cancer deaths occurring each year. The American Cancer Society estimates that greater than half a million Americans will die of cancer in 2015, accounting for nearly one out of every four deaths in the country. As the elderly population has grown, the incidence of cancer has concurrently risen, as the probability of developing cancer is more than two-fold higher after the age of seventy. Cancer care thus represents a significant and ever-increasing societal burden.

Longstanding approaches to cancer treatment include chemotherapy, radiation therapy, and surgery to remove solid tumors, Recently, T cell-targeting therapeutic antibodies have been developed. These therapeutic antibodies include bispecific antibodies that are capable of simultaneously binding cell surface antigens on T cells and tumor cells, thereby enabling the bound T cells to contribute to the destruction of the tumor cells. However, the development of such a T cell-dependent bispecific (TDB) antibody can carry an inherent risk for the development of adverse immune-mediated effects. Although certain unwanted effects, such as Fcγ receptor-mediated depletion of T cells, can be minimized by rendering the TDB antibodies effectorless, there is an unmet need in the field for the development of alternative TDB antibodies that also account for the kinetics of T cell engagement and activation.

### SUMMARY

The present invention relates to masked anti-cluster of differentiation 3 (CD3) antibodies and methods of using the same.

In one aspect, the invention features an anti-cluster of differentiation 3 (CD3) antibody, wherein the anti-CD3 antibody comprises (a) a binding domain and (b) a polypeptide mask, wherein the polypeptide mask comprises a masking moiety (MM) comprising the amino acid sequence of at least amino acid residues 1-3 of SEQ ID NO: 1 (e.g., a polypeptide mask comprising a MM comprising amino acid residues 1-3, 1-4, 1-5, 1-6, 1-7, 1-8, 1-9, 1-10, 1-11, 1-12, 1-13. 1-14, 1-15, 1-16. 1-17, 1-18, 1-19, 1-20, 1-21, 1-22. 1-23, 1-24, 1-25, 1-26, or 1-27 of SEQ ID NO: 1), or an N-terminal cyclicized glutamine derivative thereof (e.g., a polypeptide mask comprising a MM comprising 5-oxopyrrolidine-2-carboxylic acid (PCA)). For example, in some embodiments, the MM comprises at least the first three amino acid residues of SEQ ID NO: 1, except that the residue at position 1 is an N-terminal cyclicized glutamine (PCA) residue instead of a glutamine residue (e.g., the MM comprises the amino acid sequence PCA-D-G). In some embodiments, the binding domain comprises a heavy chain variable (VH) domain and a light chain variable (VL) domain and the polypeptide mask is joined to the VH domain or the VL domain. In some embodiments, the MM is extended at the C-terminus by all or a portion of the remaining sequence of SEQ ID NO: 1. For example, in some embodiments, the MM comprises the amino acid sequence of at least amino acid residues 1-5 of SEQ ID NO: 1, or an N-terminal cyclicized glutamine derivative thereof (e.g., a polypeptide mask comprising a MM comprising at least the first five amino acid residues of SEQ ID NO: 1, except that the residue at position 1 is PCA instead of a glutamine). In other embodiments, the MM comprises the amino acid sequence of at least amino acid residues 1-6 of SEQ ID NO: 1, or an N-terminal cyclicized glutamine derivative thereof (e.g., a polypeptide mask comprising a MM comprising at least the first six amino acid residues of SEQ ID NO: 1, except that the residue at position 1 is PCA instead of glutamine). In some embodiments, the anti-CD3 antibody and MM are positioned relative to each other in an N-terminal to C-terminal direction as (MM)-(anti-CD3 antibody).

In some embodiments, the polypeptide mask further comprises a cleavable moiety (CM). In some embodiments, the CM is capable of being cleaved by an enzyme, In some embodiments, the enzyme is selected from the group consisting of matrix metalloprotease (MMP)-2, MMP-9, legumain asparaginyl endopeptidase, thrombin, fibroblast activation protease (FAP), MMP-1, MMP-3, MMP-7, MMP-8, MMP-12, MMP-13, MMP-14, membrane type 1 matrix metalloprotease (MT1-MMP), plasmin, transmembrane protease, serine (TMPRSS-3/4), cathepsin A, cathepsin B, cathepsin D, cathepsin E, cathepsin F, cathepsin H, cathepsin K, cathepsin L, cathepsin L2, cathepsin O, cathepsin S, caspase 1, caspase 2, caspase 3, caspase 4, caspase 5, caspase 6, caspase 7, caspase 8, caspase 9, caspase 10, caspase 11, caspase 12, caspase 13, caspase 14, human neutrophil elastase, urokinase/urokinase-type plasminogen activator (uPA), a disintegrin and metalloprotease (ADAM)10, ADAM12, ADAM17, ADAM with thrombospondin motifs (ADAMTS), ADAMTS5, beta secretase (BACE), granzyme A, granzyme B, guanidinobenzoatase, hepsin, matriptase, matriptase 2, meprin, neprilysin, prostate-specific membrane antigen (PSMA), tumor necrosis factor-converting enzyme (TACE), kallikrein-related peptidase (KLK)3, KLK5, KLK7, KLK11, NS3/4 protease of hepatitis C virus (HCV-NS3/4), tissue plasminogen activator (tPA), caipain, calpain 2, glutamate carboxypeptidase II, plasma kallikrein, AMSH-like protease, AMSH, γ-secretase component, antiplasmin cleaving enzyme (APCE), decysin 1, apoptosis-related cysteine peptidase, and N-acetylated alpha-linked acidic dipeptidase-like 1. For example, in some embodiments, the enzyme is MMP-2, MMP-9, legumain asparaginyl endopeptidase, thrombin, or FAP. In some embodiments, the CM comprises an acid-labile linker that is capable of being cleaved in an acidic pH environment. In some embodiments, the acid-labile linker comprises a hydrazone, an imino, an ester, or an amido group. In some embodiments, the acidic pH environment is found in the lysosome of a cell. In another embodiment, the acidic pH environment is found in a tumor microenvironment. In some embodiments wherein the anti-CD3 antibody, MM, and CM are positioned relative to each other in an N-terminal to C-terminal direction as (MM)-(CM)-(anti-CD3 antibody).

In some embodiments, the polypeptide mask further comprises a linker moiety (LM). In some embodiments, the LM is between 5 to 24 amino acids in length (e.g., the LM is 5, 6, 7, 8, 9, 10, 11, 12, 13, 14, 15, 16, 17, 18, 19, 20, 21, 22, 23, or 24 amino acids in length). In some embodiments, the LM is between 5 to 15 amino acids in length (e.g., the LM is 5, 6, 7, 8, 9, 10, 11, 12, 13, 14, or 15 amino acids in length). In some embodiments, the LM comprises glycine (G) and serine (S) residues. In some embodiments, the LM comprises GS repeats. In some embodiments, the anti-CD3 antibody, MM, and LM are positioned relative to each other in an N-terminal to C-terminal direction as (MM)-(LM)-(anti-CD3 antibody). In some embodiments, the polypeptide mask comprises a cleavable moiety and a linker moiety, and wherein the anti-CD3 antibody, MM, CM, and LM are positioned relative to each other in an N-terminal to C-terminal direction as (MM)-(LM)-(CM)-(anti-CD3 antibody)or (MM)-(CM)-(LM)-(anti-CD3 antibody).

In some embodiments, the binding domain comprises one or more (e.g., one, two, three, four, five, or six) of the following six hypervariable regions (HVRs): (a) an HVR-H1 comprising the amino acid sequence of SEQ ID NO: 2; (b) an HVR-H2 comprising the amino acid sequence of SEQ ID NO: 3; (c) an HVR-H3 comprising the amino acid sequence of SEQ ID NO: 4; (d) an HVR-L1 comprising the amino acid sequence of SEQ ID NO: 5; (e) an HVR-L2 comprising the amino acid sequence of SEQ ID NO: 6: and (f) an HVR-L3 comprising the amino acid sequence of SEQ ID NO: 7. In some embodiments, the binding domain comprises (a) a heavy chain variable (VH) domain comprising an amino acid sequence having at least 95% sequence identity to the amino acid sequence of SEQ ID NO: 8; (b) a light chain variable (VL) domain comprising an amino acid sequence having at least 95% sequence identity to the amino acid sequence of SEQ ID NO: 9; or (c) a VH domain as in (a) and a VL domain as in (b). In some embodiments, the VH domain comprises the amino acid sequence of SEQ ID NO: 8. In some embodiments, the VL domain comprises the amino acid sequence of SEQ ID NO: 9. In some embodiments, the VH domain comprises the amino acid sequence of SEQ ID NO: 8, and the VL domain comprises the amino acid sequence of SEQ ID NO: 9.

In some embodiments, the binding domain comprises one or more (e.g., one, two, three, four, five, or six) of the following six hypervariable regions (HVRs): (a) an HVR-H1 comprising the amino acid sequence of SEQ ID NO: 10; (b) an HVR-H2 comprising the amino acid sequence of SEQ ID NO: 11; (c) an HVR-H3 comprising the amino acid sequence of X₁X₂YSX₃X₄X₅FDY, wherein X₁ is selected from the group consisting of D, T, and S; X₂ is selected from the group consisting of G, A, and S; X₃ is R or N; X₄ is Y or A; and X₅ is Y or A (SEQ ID NO: 12); (d) an HVR-L1 comprising the amino acid sequence of SEQ ID NO: 13; (e) an HVR-L2 comprising the amino acid sequence of SEQ ID NO: 14; and (f) an HVR-L3 comprising the amino acid sequence of X₁X₂SX₃X₄LRT, wherein X₁ is K or T; X₂ is Q or A; X₃ is F or A; and X₄ is I or A (SEQ ID NO: 15). For example, in some embodiments, the binding domain comprises the following six HVRs: (a) an HVR-H1 comprising the amino acid sequence of SEQ ID NO: 10; (b) an HVR-H2 comprising the amino acid sequence of SEQ ID NO: 11; (c) an HVR-H3 comprising the amino acid sequence of SEQ ID NO: 16; (d) an HVR-L1 comprising the amino acid sequence of SEQ ID NO: 13; (e) an HVR-L2 comprising the amino acid sequence of SEQ ID NO: 14; and (f) an HVR-L3 comprising the amino acid sequence of SEQ ID NO: 17. In some embodiments, the binding domain comprises (a) a VH domain comprising an amino acid sequence having at least 95% sequence identity to the amino acid sequence of SEQ ID NO: 18; (b) a VL domain comprising an amino acid sequence having at least 95% sequence identity to the amino acid sequence of SEQ ID NO: 19; or (c) a VH domain as in (a) and a VL domain as in (b). In some embodiments, the VH domain comprises the amino acid sequence of SEQ ID NO: 18. In some embodiments, the VL domain comprises the amino acid sequence of SEQ ID NO: 19. In some embodiments, the VH domain comprises the amino acid sequence of SEQ ID NO: 18, and the VL domain comprises the amino acid sequence of SEQ ID NO: 19.

In other embodiments, the binding domain comprises one or more (e.g., one, two, three, four, five, or six) of the following six HVRs: (a) an HVR-H1 comprising the amino acid sequence of SEQ ID NO: 10; (b) an HVR-H2 comprising the amino acid sequence of SEQ ID NO: 11; (c) an HVR-H3 comprising the amino acid sequence of SEQ ID NO: 16; (d) an HVR-L1 comprising the amino acid sequence of SEQ ID NO: 13; (e) an HVR-L2 comprising the amino acid sequence of SEQ ID NO: 14; and (f) an HVR-L3 comprising the amino acid sequence of SEQ ID NO: 104, In some embodiments, the binding domain comprises (a) a VH domain comprising an amino acid sequence having at least 95% sequence identity to the amino acid sequence of SEQ ID NO: 107; (b) a VL domain comprising an amino acid sequence having at least 95% sequence identity to the amino acid sequence of SEQ ID NO: 108; or (c) a VH domain as in (a) and a VL domain as in (b). In some embodiments, the VH domain comprises the amino acid sequence of SEQ ID NO: 107. In some embodiments, the VL domain comprises the amino acid sequence of SEQ ID NO: 108. In some embodiments, the VH domain comprises the amino acid sequence of SEQ ID NO: 107, and the VL domain comprises the amino acid sequence of SEQ ID NO: 108.

In some embodiments, the binding domain comprises one or more (e.g., one, two, three, four, five, or six) of the following six HVRs: (a) an HVR-H1 comprising the amino acid sequence of SEQ ID NO: 10; (b) an HVR-H2 comprising the amino acid sequence of SEQ ID NO: 11; (c) an HVR-H3 comprising the amino acid sequence of SEQ ID NO: 16; (d) an HVR-L1 comprising the amino acid sequence of SEQ ID NO: 13; (e) an HVR-L2 comprising the amino acid sequence of SEQ ID NO: 14; and (f) an HVR-L3 comprising the amino acid sequence of SEQ ID NO: 105. In some embodiments, the binding domain comprises (a) a VH domain comprising an amino acid sequence having at least 95% sequence identity to the amino acid sequence of SEQ ID NO: 109; (b) a VL domain comprising an amino acid sequence having at least 95% sequence identity to the amino acid sequence of SEQ ID NO: 110; or (c) a VH domain as in (a) and a VL domain as in (b). In some embodiments, the VH domain comprises the amino acid sequence of SEQ ID NO: 109, In some embodiments, the VL domain comprises the amino acid sequence of SEQ ID NO: 110. In some embodiments, the VH domain comprises the amino acid sequence of SEQ ID NO: 109, and the VL domain comprises the amino acid sequence of SEQ ID NO: 110.

In other embodiments, the binding domain comprises one or more (e.g., one, two, three, four, five, or six) of the following six HVRs: (a) an HVR-H1 comprising the amino acid sequence of SEQ ID NO: 10; (b) an HVR-H2 comprising the amino acid sequence of SEQ ID NO: 11; (c) an HVR-H3 comprising the amino acid sequence of SEQ ID NO: 16; (d) an HVR-L1 comprising the amino acid sequence of SEQ ID NO: 13; (e) an HVR-L2 comprising the amino acid sequence of SEQ ID NO: 14; and (f) an HVR-L3 comprising the amino acid sequence of SEQ ID NO: 106. In some embodiments, the binding domain comprises (a) a VH domain comprising an amino acid sequence having at least 95% sequence identity to the amino acid sequence of SEQ ID NO: 111; (b) a VL domain comprising an amino acid sequence having at least 95% sequence identity to the amino acid sequence of SEQ ID NO: 112; or (c) a VH domain as in (a) and a VL domain as in (b). In some embodiments, the VH domain comprises the amino acid sequence of SEQ ID NO: 111. In some embodiments, the VL domain comprises the amino acid sequence of SEQ ID NO: 112. In some embodiments, the VH domain comprises the amino acid sequence of SEQ ID NO: 111, and the VL domain comprises the amino acid sequence of SEQ ID NO: 112.

Additional anti-CD3 antibodies include anti-CD3 binding domains disclosed in U.S.SN. 14/574,132 (U.S. Pub. No. 2015-0166661), which is incorporated herein by reference in its entirety, and a polypeptide mask having any of the aforementioned additional features (e.g., MM, CM, LM). For example, a masked anti-CD3 antibody may include HVRs as in any of the referenced anti-CD3 antibodies, and further include any of the referenced acceptor framework regions (FRs) in addition to a polypeptide mask including, for example, a MM, CM, and/or LM. In some embodiments, a masked anti-CD3 antibody may include a VH domain and/or a VL domain as in any of the referenced anti-CD3 antibodies, and further include a polypeptide mask including, for example, a MM, CM, and/or LM. For instance, a masked anti-CD3 antibody may include anti-CD3 antibody SP34 (Pessano et al. The EMBO Journal. 4: 337-344,1985) and a polypeptide mask having, for example, a MM, CM, and/or LM.

In some embodiments, the polypeptide mask inhibits the ability of the anti-CD3 antibody to bind to a human CD3 polypeptide by at least 10% (e.g., 10%, 11 %, 1 2%, 13%, 14%, 15%, 16%, 17%, 18%, 19%. 20%, 21%, 22%, 23%, 24%, 25%, 26%, 27%. 28%. or 29%), at least 30% (e.g., 30%, 31%, 32%, 33%, 34%, 35%, 36%, 37%, 38%, 39%, 40%, 41%, 42%, 43%. 44%, 45%, 46%, 47%, 48%, 49%, 50%, 51%. 52%, 53%, 54%, 55%, 56%, 57%, 58%, or 59%), at least 60% (e.g., 60%, 61%, 62%, 63%, 64%, 65%. 66%, 67%, 68%, 69%, 70%, 71%, 72%, 73%, 74%, 75%, 76%, 77%, 78%, or 79%), at least 80% (e.g., 80%, 81%, 82%, 83%, 84%, 85%, 86%, 87%, 88%, or 89%), or at least 90% (e.g., 90%, 91%, 92%, 93%, 94%, 95%, 96%, 97%, 98%, or 99%). In some embodiments, the polypeptide mask completely inhibits the ability of the anti-CD3 antibody to bind to a human CD3 polypeptide (i.e., 100% inhibition). In some embodiments, the human CD3 polypeptide is a human CD3ε polypeptide.

In some embodiments, the anti-CD3 antibody comprises an aglycosylation site mutation. In some embodiments, the aglycosylation site mutation is a substitution mutation. In some embodiments, the substitution mutation is at amino acid residue N297, L234, L235, and/or D265 (EU numbering). In some embodiments, the substitution mutation is selected from the group consisting of N297G, N297A, L234A, L235A, and D265A. In some embodiments, the substitution mutation is an N297G mutation. In some embodiments, the aglycosylation site mutation reduces effector function of the anti-CD3 antibody.

In some embodiments, the anti-CD3 antibody is monoclonal, human, humanized, or chimeric. In some embodiments, the anti-CD3 antibody is an antibody fragment that binds CD3. In some embodiments, the antibody fragment is selected from the group consisting of Fab, Fab', Fab'-SH, Fv, scFv, TaFv, (Fab')₂, diabody, bsDb, scDb, DART, BiTE, and V_{H}H fragments. In some embodiments, the anti-CD3 antibody is a full-length antibody. In some embodiments, the anti-CD3 antibody is an IgG antibody. In some embodiments, the anti-CD3 antibody is a monospecific antibody.

In other embodiments, the anti-CD3 antibody is a multispecific antibody. In some embodiments, the multispecific antibody is a bispecific antibody. In some embodiments, the bispecific antibody comprises a second binding domain that binds to a second biological molecule, wherein the second biological molecule is a cell surface antigen, In some embodiments, the cell surface antigen is a tumor antigen. In some embodiments, the tumor antigen is selected from the group consisting of CD20; FcRH5 (Fc Receptor-like 5); HER2; LYPD1; Ly6G6D (lymphocyte antigen 6 complex, locus G61); Ly6-D, MEGT1); PMEL17 (silver homolog; SILV; D12S53E; PMEL17; (SI); (SIL); ME20; gp100); Ly6E (lymphocyte antigen 6 complex, locus E; Ly67, RIG-E, SCA-2, TSA-1): CD19; CD33; CD22 (B-cell receptor CD22-B isoform); CD79a (CD79A, CD79a, immunoglobulin-associated alpha; BMPR1B (bone morphogenetic protein receptor-type IB); CD79b (CD79B, CD790, 1 Gb (immunoglobulin-associated beta), B29); EDAR (Ectodysplasin A Receptor); GFRA1 (GDNF-Ra1); MRP4 (Multidrug Resistance Protein 4); RET; STEAP1 (six transmembrane epithelial antigen of prostate); TENB2 (putative transmembrane proteoglycan); E16 (LAT1, SLC7A5); 0772P (CA125, MUC16); MPF (MPF, MSLN, SMR, megakaryocyte potentiating factor, mesothelin); Napi3b (NAPI-3B, NPTIIb, SLC34A2, solute carrier family 34 (sodium phosphate), member 2, type II sodium-dependent phosphate transporter 3b); Sema 5b; PSCA hlg (2700050C12Rik, C530008O16Rik, RIKEN cDNA 2700050C12, RIKEN cDNA 2700050C12 gene); ETBR (Endothelin type B receptor); MSG783 (RNF124, hypothetical protein FLJ20315); STEAP2; TrpM4 (BR22450, FLJ20041, TRPM4, TRPM4B, transient receptor potential cation channel, subfamily M, member 4); CRIPTO (CR, CR1, CRGF, CRIPTO, TOGF1, teratocarcinoma-derived growth factor); CD21 (CR2 (Complement receptor 2) or C3DR (C3d/Epstein Barr virus receptor) or Hs.73792); FcRH2 (IFGP4, IRTA4, SPAP1A (SH2 domain containing phosphatase anchor protein 1a), SPAP1B, SPAP1C); NCA; MDP; IL20Rα; Brevican; EphB2R; ASLG659; PSCA; GEDA; BAFF-R (B cell-activating factor receptor, BLyS receptor 3, BR3); CXCR5 (Burkitt's lymphoma receptor 1; HLA-DOB (Beta subunit of MHC class II molecule); P2X5 (Purinergic receptor P2X ligand-gated ion channel 5; CD72 (B-cell differentiation antigen CD72, Lyb-2); LY64 (Lymphocyte antigen 64 (RP105), type I membrane protein of the leucine rich repeat (LRR) family); FcRH1 (Fc receptor-like protein 1); IRTA2 (Immunoglobulin superfamily receptor translocation associated 2); TMEFF1; TMEM46 (shisa homolog 2 (Xenopus laevis); SHISA2); LGR5 (leucine-rich repeat-containing G protein-coupled receptor 5; GPR49, GPR67); LY6K (lymphocyte antigen 6 complex, locus K; LY6K; HSJ001348; FLJ35226); GPR19 (G protein-coupled receptor 19; Mm 4787); GPR54 (KISS1 receptor; KISS1R; GPR54; HOT7T175; AXOR12); ASPHD1 (aspartate beta-hydroxylase domain containing 1; LOC253982); Tyrosinase (TYR; OCAIA; OCA1A; tyrosinase; SHEP3); TMEM118 (ring finger protein, transmembrane 2; RNFT2; FLJ14627); GPR172A (G protein-coupled receptor 172A; GPCR41; FLJ11856; D15Ertd747e); GPC3 (Glypican 3); CLL1 (C-Type Lectin-like molecule 1); B7-H4 (B7x; B7S1); RNF43 (Ring finger protein 43); CD70; CXORF61 (Chromosome X open reading frame 61); and SLC53D3. In some embodiments, the tumor antigen is selected from the group consisting of CD20, FcRH5, HER2, LYPD1, LY6G6D, PMEL17, LY6E, CD19, CD33, CD22, CD79A, CD79B, EDAR, GFRA1, MRP4, RET, Steap1, and TenB2.

In some embodiments wherein the tumor antigen is CD20, the second binding domain comprises the following six HVRs: (a) an HVR-H1 comprising the amino acid sequence of SEQ ID NO: 20: (b) an HVR-H2 comprising the amino acid sequence of SEQ ID NO: 21; (c) an HVR-H3 comprising the amino acid sequence of SEQ ID NO: 22; (d) an HVR-L1 comprising the amino acid sequence of SEQ ID NO: 23; (e) an HVR-L2 comprising the amino acid sequence of SEQ ID NO: 24; and (f) an HVR-L3 comprising the amino acid sequence of SEQ ID NO: 25. In some embodiments, the binding domain comprises (a) a heavy chain variable (VH) domain comprising an amino acid sequence having at least 95% sequence identity to the amino acid sequence of SEQ ID NO: 26; (b) a light chain variable (VL) domain comprising an amino acid sequence having at least 95% sequence identity to the amino acid sequence of SEQ ID NO: 27; or (c) a VH domain as in (a) and a VL domain as in (b). In some embodiments, the VH domain comprises the amino acid sequence of SEQ ID NO: 26. In some embodiments, the VL domain comprises the amino acid sequence of SEQ ID NO: 27. In some embodiments, the VH domain comprises the amino acid sequence of SEQ ID NO: 26 and the VL domain comprises the amino acid sequence of SEQ ID NO: 27.

In some embodiments, the anti-CD3 antibody comprises one or more heavy chain constant domains, wherein the one or more heavy chain constant domains are selected from a first CH1 (CH1*₁*) domain, a first CH2 (CH2*₁*) domain, a first CH3 (CH3*₁*) domain, a second CH1 (CH1*₂*) domain, second CH2 (CH2*₂*) domain, and a second CH3 (CH3*₂*) domain. In some embodiments, at least one of the one or more heavy chain constant domains is paired with another heavy chain constant domain. In some embodiments, the CH3*₁* and CH3*₂* domains each comprise a protuberance or cavity, and wherein the protuberance or cavity in the CH3*₁* domain is positionable in the cavity or protuberance, respectively, in the CH3*₂* domain. In some embodiments, the CH2*₁* and CH2*₂* domains each comprise a protuberance or cavity, and wherein the protuberance or cavity in the CH2*₁* domain is positionable in the cavity or protuberance, respectively, in the CH2*₂* domain. In some embodiments, the CH2*₁* and CH2*₂* domains meet at an interface between said protuberance and cavity.

In another aspect, the invention features an isolated nucleic acid that encodes any of the anti-CD3 antibodies disclosed herein. The nucleic acid may be comprised in a vector (e.g., an expression vector) for expressing the antibody.

In another aspect, the invention features host cells comprising the preceding nucleic acids and/or vectors. In some embodiments, the host cell is a mammalian cell (e.g., a Chinese hamster ovary (CHO) cell). In other embodiments, the host cell is a prokaryotic cell (e.g., an *E. coli* cell). A method of producing any one of the preceding anti-CD3 antibodies is also provided, the method comprising culturing the host cell that produces the anti-CD3 antibody. In some embodiments, the method further comprises recovering the anti-CD3 antibody from the host cell or the culture medium.

In some embodiments, the invention features an immunoconjugate comprising any one of the preceding anti-CD3 antibodies conjugated to a cytotoxic agent.

Also provided is a composition comprising any one of the preceding anti-CD3 antibodies or immunoconjugates. In some embodiments, the composition further comprises a pharmaceutically acceptable carrier, excipient, or diluent. In some embodiments, the composition is a pharmaceutical composition. In some embodiments, the composition further comprises an additional therapeutic agent.

A further aspect of the invention is a method of treating or delaying the progression of a cell proliferative disorder or an autoimmune disorder in a subject in need thereof, the method comprising administering to the subject an effective amount any one of the preceding anti-CD3 antibodies. In another aspect, the invention features a method of enhancing immune function in a subject having a cell proliferative disorder or an autoimmune disorder, the method comprising administering to the subject any one of the preceding anti-CD3 antibodies. In some embodiments, the anti-CD3 antibody binds to (a) a CD3 molecule located on an immune effector cell and (b) a second biological molecule located on a target cell other than the immune effector cell. In some embodiments, the anti-CD3 antibody binds to the second biological molecule prior to the CD3 molecule. In some embodiments, the anti-CD3 antibody accumulates at the surface of the target cell, for example, because of a decreased association constant (Kₐ) of the anti-CD3 antibody towards CD3 molecules located on the immune effector cell. In some embodiments, the anti-CD3 antibody is capable of providing a cytotoxic effect on the target cell (e.g., via the activated immune effector cell). In some embodiments, the anti-CD3 antibody is capable of providing an apoptotic effect on the target cell (e.g., via the activated immune effector cell). In some embodiments, the anti-CD3 antibody is capable of providing a cytotoxic effect and/or an apoptotic effect on the target cell. In some embodiments, the cytotoxic effect and/or the apoptotic effect on the target cell is independent of activation of the immune effector cell. In other embodiments, the cytotoxic effect and/or the apoptotic effect on the target cell is dependent of activation of the immune effector cell. In some embodiments, the anti-CD3 antibody is administered to the subject in a dosage of about 0.01 mg/kg to about 30 mg/kg. In some embodiments, the anti-CD3 antibody is administered to the subject in a dosage of about 0.1 mg/kg to about 30 mg/kg. In some embodiments, the anti-CD3 antibody is administered to the subject in a dosage of about 1 mg/kg to about 30 mg/kg. In some embodiments, the anti-CD3 antibody is administered subcutaneously, intravenously, intramuscularly, topically, orally, transdermally, intraperitoneally, intraorbitally, by implantation, by inhalation, intrathecally, intraventricularly, or intranasally. In some embodiments, the anti-CD3 antibody is administered subcutaneously. In some embodiments, the anti-CD3 antibody is administered intravenously.

In some embodiments, the method further comprises administering to the subject a PD-1 axis binding antagonist or an additional therapeutic agent. In some embodiments, the additional therapeutic agent is administered prior to or subsequent to the administration of the anti-CD3 antibody. In some embodiments, the additional therapeutic agent is administered concurrently with the anti-CD3 antibody. In some embodiments, the PD-1 axis binding antagonist is selected from the group consisting of a PD-1 binding antagonist, a PD-L1 binding antagonist, and a PD-L2 binding antagonist. In some embodiments, the PD-1 axis binding antagonist is a PD-1 binding antagonist. In some embodiments, the PD-1 binding antagonist is selected from the group consisting of MDX-1106 (nivolumab), MK-3475 (lambrolizumab), CT-011 (pidilizumab), and AMP-224, In other embodiments, the PD-1 axis binding antagonist is a PD-L1 binding antagonist. In some embodiments, the PD-L1 binding antagonist is selected from the group consisting of: YW243.55.S70, MPDL3280A, MDX-1105, and MEDt4736. In other embodiments, the PD-1 axis binding antagonist is a PD-L2 binding antagonist. In some embodiments, the PD-L2 binding antagonist is an antibody or an immunoadhesin.

In some embodiments, the method further comprises administering to the subject a glucocorticoid. In some embodiments, the glucocorticoid is selected from the group consisting of dexamethasone, hydrocortisone, cortisone, prednisolone, prednisone, methylprednisone, triamcinolone, paramethasone, betamethasone, fludrocortisone, and pharmaceutical acceptable esters, salts, and complexes thereof. In some embodiments, the glucocorticoid is dexamethasone. In some embodiments, the glucocorticoid is a pharmaceutically acceptable ester, salt, or complex of dexamethasone.

In some embodiments, the method further comprises administering to the subject rituximab.

In any of the preceding methods, the cell proliferative disorder can be cancer. In some embodiments, the cancer is selected from the group consisting of breast cancer, bladder cancer, colorectal cancer, non-small cell lung cancer, non-Hodgkin's lymphoma (NHL), B cell lymphoma, B cell leukemia, multiple myeloma, renal cancer, prostate cancer, liver cancer, head and neck cancer, melanoma, ovarian cancer, mesothelioma, and glioblastoma. In some embodiments, the B cell leukemia is chronic lymphoid leukemia (CLL).

In any of the preceding methods, the autoimmune disorder can be selected from the group consisting of rheumatoid arthritis, juvenile rheumatoid arthritis, systemic lupus erythematosus (SLE), Wegener's disease, inflammatory bowel disease, idiopathic thrombocytopenic purpura (ITP), thrombotic thrombocytopenic purpura (TTP), autoimmune thrombocytopenia, multiple sclerosis, psoriasis, IgA nephropathy, IgM polyneuropathies, myasthenia gravis, vasculitis, diabetes mellitus, Reynaud's syndrome, Sjögren's syndrome, glomerulonephritis, Neuromyelitis Optica (NMO), and IgG neuropathy.

In another aspect, the invention features a kit comprising: (a) a composition comprising any one of the preceding anti-CD3 antibodies and (b) a package insert comprising instructions for administering the composition to a subject to treat or delay progression of a cell proliferative disorder.

In any of the preceding methods, the subject can be a human.

### Brief Description of the Drawings

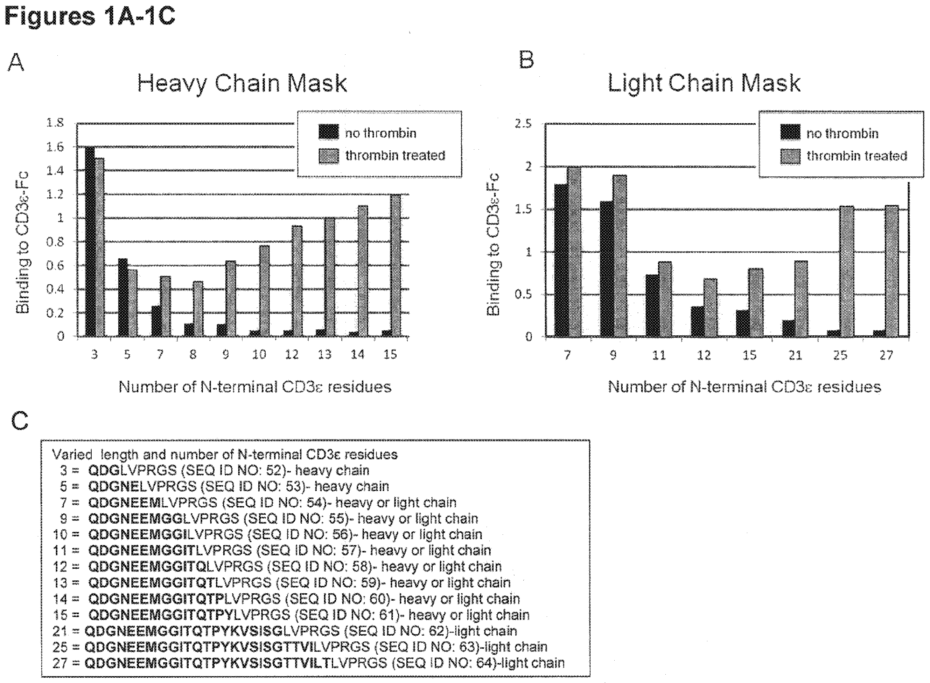

FIGURE 1A is a graph showing the relative binding of the indicated cleavable moiety (CM)-containing masked anti-CD3 SP34 variants to CD3ε¹⁻²⁷-Fc (CD3ε-Fc), before or after cleavage with thrombin, as assessed by phage ELISA. The masked SP34 variants included polypeptide masks having a masking moiety (MM) including an N-terminal CD3ε peptide of varied length (ranging from the first 3 to the first 15 amino acid residues of human CD3ε), joined to the N-terminus of the heavy chain variable (VH) region of SP34 via an intervening CM including a thrombin cleavage site. The measured binding signals were normalized for display, as assessed by binding to a gD tag displayed on the C-terminus of the light chain of SP34.
FIGURE 1B is a graph showing the relative binding of the indicated CM-containing masked anti-CD3 SP34 variants to CD3ε-Fc, before or after cleavage with thrombin, as assessed by phage ELISA. The masked SP34 variants included polypeptide masks having a MM including an N-terminal CD3ε. peptide of varied length (ranging from the first 7 to the first 27 amino acid residues of human CD3ε), joined to the N-terminus of the light chain variable (VL) region of SP34 via an intervening CM including a thrombin cleavage site. The measured binding signals were normalized for display, as assessed by binding to a gD tag displayed on the C-terminus of the light chain of SP34.
FIGURE 1C shows the amino acid sequences of each of the CM-containing polypeptide masks that were joined to the N-terminus of the VH or VL region of the anti-CD3 SP34 variants tested in Figures 1A and 1B.

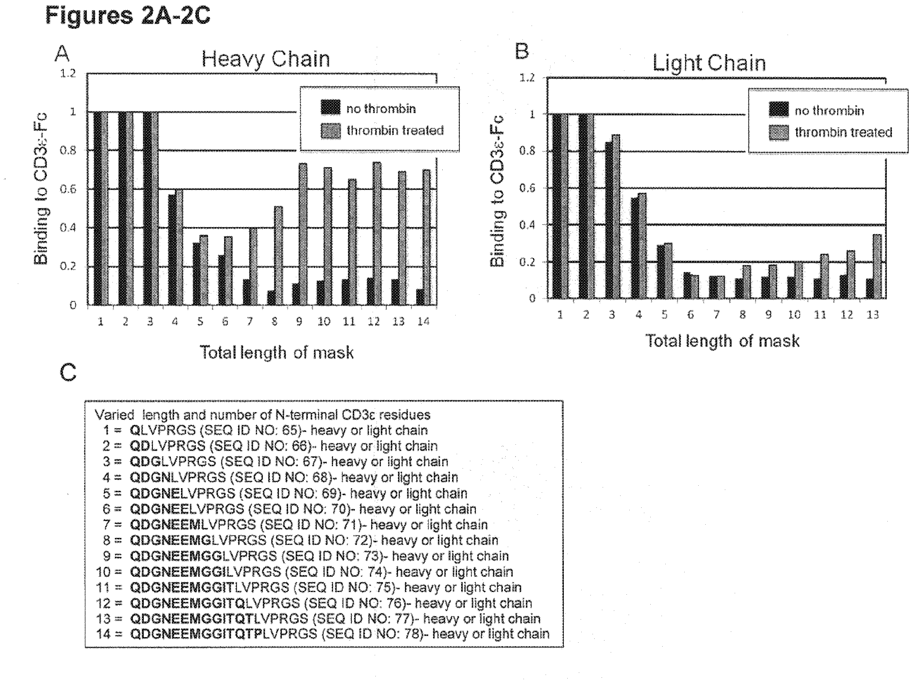

FIGURE 2A is a graph showing the relative binding of the indicated CM-containing masked anti-CD3 antibody variants to CD3ε-Fc, before or after cleavage with thrombin, as assessed by phage ELISA. The masked anti-CD3 antibody variants included polypeptide masks having a MM including an N-terminal CD3ε peptide of varied length (ranging from the first amino acid residue to the first 14 amino acid residues of human CD3ε), joined to the N-terminus of the VH region of the anti-CD3 antibody via an intervening CM including a thrombin cleavage site. The measured binding signals were normalized for display, as assessed by binding to a gD tag displayed on the C-terminus of the light chain of the anti-CD3 antibody.
FIGURE 2B is a graph showing the relative binding of the indicated CM-containing masked anti-CD3 antibody variants to CD3ε-Fc, before or after cleavage with thrombin, as assessed by phage ELISA. The masked anti-CD3 antibody variants included polypeptide masks having a MM including an N-terminal CD3ε peptide of varied length (ranging from the first amino acid residue to the first 13 amino acid residues of human CD3ε), joined to the N-terminus of the VL region of the anti-CD3 antibody via an intervening CM including a thrombin cleavage site. The measured binding signals were normalized for display, as assessed by binding to a gD tag displayed on the C-terminus of the light chain of the anti-CD3 antibody.
FIGURE 2C shows the amino acid sequences of each of the CM-containing polypeptide masks that were joined to the N-terminus of the VH or VL region of the anti-CD3 antibody variants tested in Figures 2A and 2B.

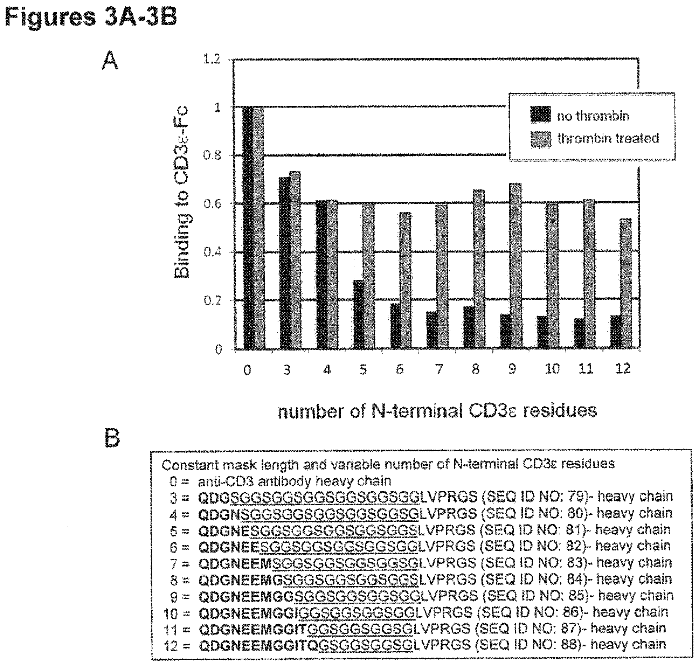

FIGURE 3A is a graph showing the relative binding of the indicated masked anti-CD3 antibody variants to CD3ε-Fc, before or after cleavage with thrombin, as assessed by phage ELISA. The masked anti-CD3 antibody variants included polypeptide masks, each having a MM and a linker moiety (LM) of varied length (MM ranging from the first amino acid residue to the first 11 amino acid residues of human CD3ε; LM ranging from 10-20 amino acid residues), joined to the N-terminus of the VH region of the anti-CD3 antibody via an CM including a thrombin cleavage site. The measured binding signals were normalized for display, as assessed by binding to a gD tag displayed on the C-terminus of the light chain of the anti-CD3 antibody.
FIGURE 3B shows the amino acid sequences of each of the CM- and LM-containing polypeptide masks that were joined to the N-terminus of the VH region of the anti-CD3 antibody variants tested in Figures 3A.

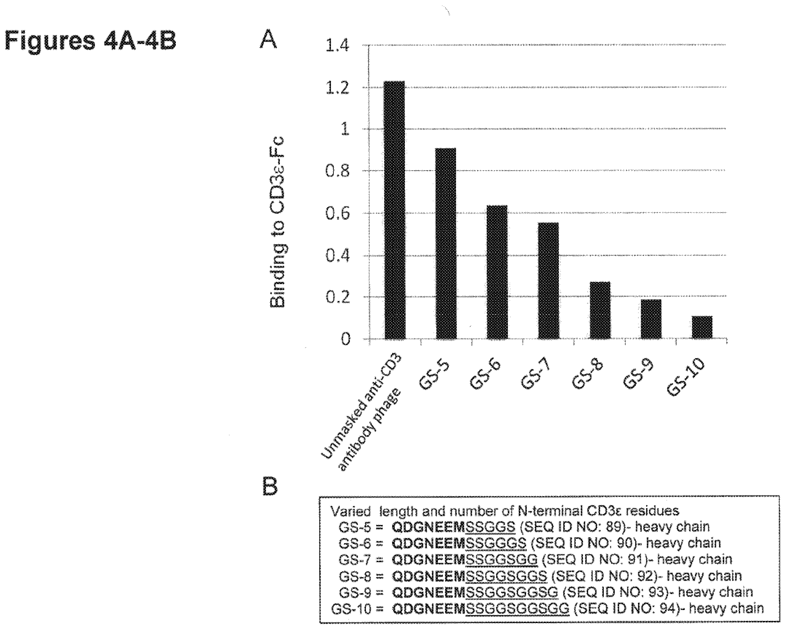

FIGURE 4A is a graph showing the relative binding of the indicated masked anti-CD3 antibody variants to CD3ε-Fc, as assessed by phage ELISA. The masked anti-CD3 antibody variants included "fixed" polypeptide masks, each having a MM including the first seven amino acid residues of human CD3ε and a LM of varied length (ranging from 5-10 amino acid residues), joined to the N-terminus of the VH region of the anti-CD3 antibody via the LM. The measured binding signals were normalized for display, as assessed by binding to a gD tag displayed on the C-terminus of the light chain of the anti-CD3 antibody.
FIGURE 4B shows the amino acid sequences of each of the fixed polypeptide masks that were joined to the N-terminus of the VH region of the anti-CD3 antibody variants tested in Figures 4A.

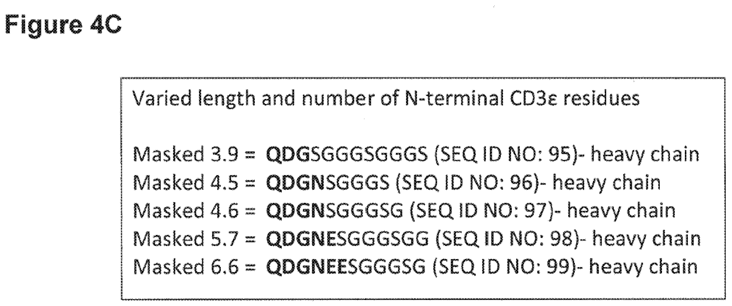

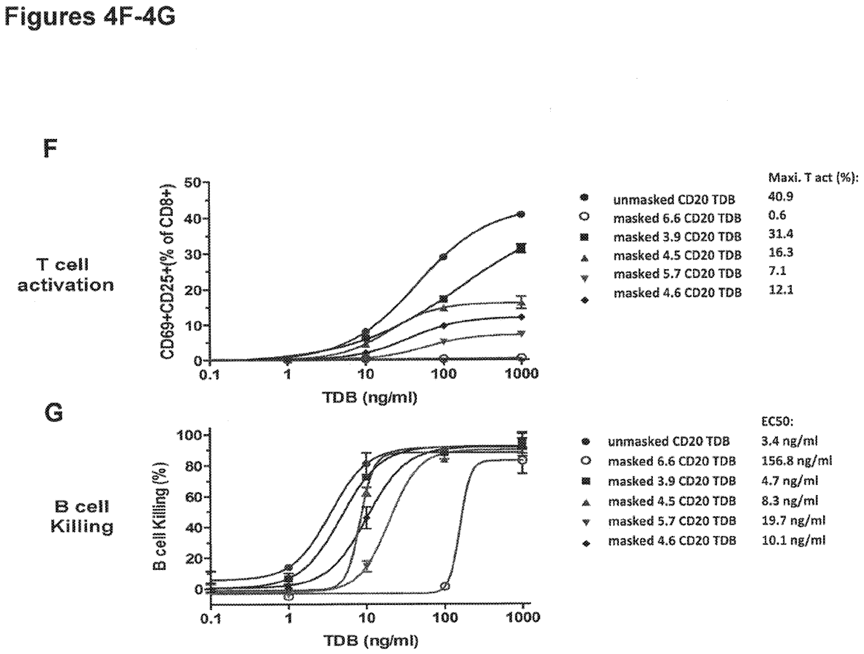

FIGURE 4C shows the amino acid sequences of each of the fixed polypeptide masks having varied MM length that were joined to the N-terminus of the VH region of the anti-CD3 arm of the CD20 TDBs tested in Figures 4F and 4G.

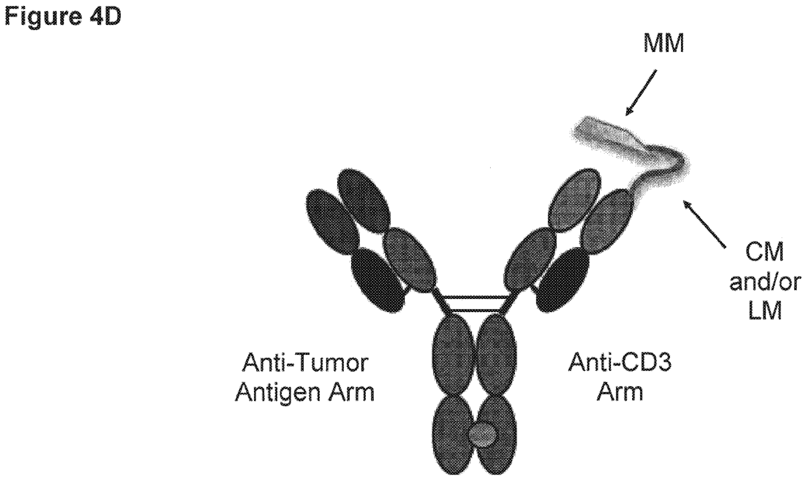

FIGURE 4D depicts a schematic generalization of a masked T cell-dependent bispecific (TDB) antibody having an anti-tumor antigen arm (e.g., anti-CD20 arm), an anti-CD3 arm, and a polypeptide mask including a MM joined to the anti-CD3 arm of the TDB via a CM, LM, or both CM and LM. The polypeptide mask of the depicted masked TDB is joined to the VL domain of the anti-CD3 arm, but it should be understood that the polypeptide mask may alternatively be joined to the VH region of the anti-CD3 arm,

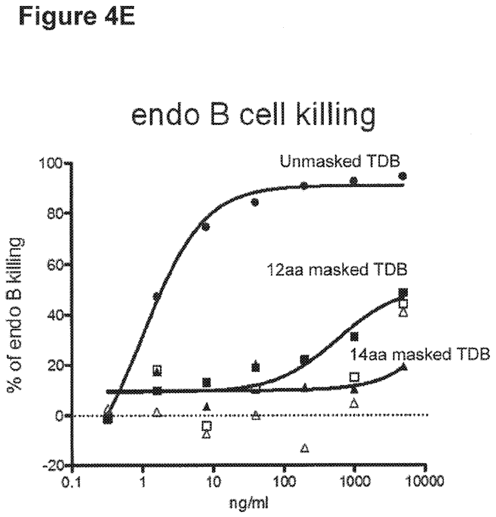

FIGURE 4E is a graph showing the percentage of endogenous B cell killing relative to a non-TDB treated control, after 48 hours of incubation of various CD3/CD20 TDBs (unmasked, 12aa masked, and 14 aa masked TDBs) at different concentrations with 200,000 human PBMCs (isolated from Donor #P0000033694) per well, as measured by FACS analysis. At the end of each assay, live B cells were gated out as PI-CD19⁺ or PI-CD20⁺ B cells, and absolute cell count was obtained by FITC beads added to the reaction mixture as an internal counting control.
FIGURE 4F is a graph showing the percentage of CD8⁺ T cell activation, as measured by the percentage of CD69 and CD25 surface expression, after ∼20 hours of incubation of various CD3/CD20 TDBs (unmasked, masked 6.6, masked 3.9, masked 4.5, masked 5.7, and masked 4.6 CD20 TDBs) at different concentrations with ∼200,000 human PBMCs (isolated from Donor #P0000033694) per well, as measured by FACS analysis. The extent of T cell activation was determined by comparing the percentage of the CD69⁺/CD25⁺ population of CD8⁺ T cells.
FIGURE 4G is a graph showing the percentage of endogenous B cell killing relative to a non-TDB treated control, after 48 hours of incubation of various CD3/CD20 TDBs (unmasked, masked 6.6, masked 3.9, masked 4.5, masked 5.7, and masked 4.6 CD20 TDBs) at different concentrations with 200,000 human PBMCs (isolated from Donor #P0000033694) per well. as measured by FACS analysis. At the end of each assay, live B cells were gated out as PI-CD19⁺ or PI-CD20⁺ B cells, and absolute cell count was obtained by FITC beads added to the reaction mixture as an internal counting control. The EC50 values in ng/ml are also shown in tabular form to quantitatively evaluate the efficacy of B cell killing for each CD20 TDB tested.

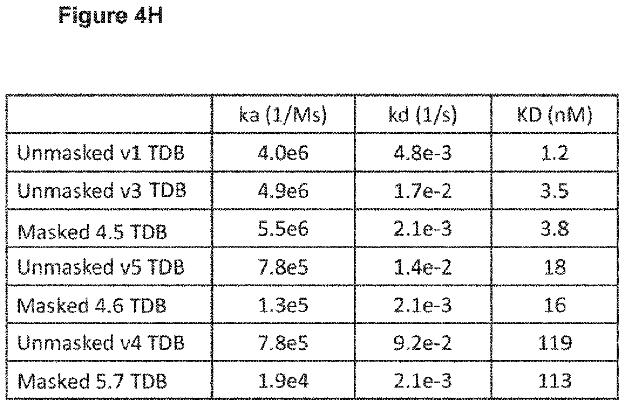

FIGURE 4H is a table indicating the results of a binding affinity assay for affinity variants of unmasked CD3/CD20 TDBs (unmasked v1, unmasked v3, unmasked v4, and unmasked v5) and masked CD3/CD20 TDBs (masked 4.5, masked 4.6, and masked 5.7) provided in the first column. For each TDB, the calculated association rate (kₐ), dissociation rate (k_{d}), and overall binding affinity (K_{D}) for a single chain CD3εγ heterodimer recombinant antigen is provided.

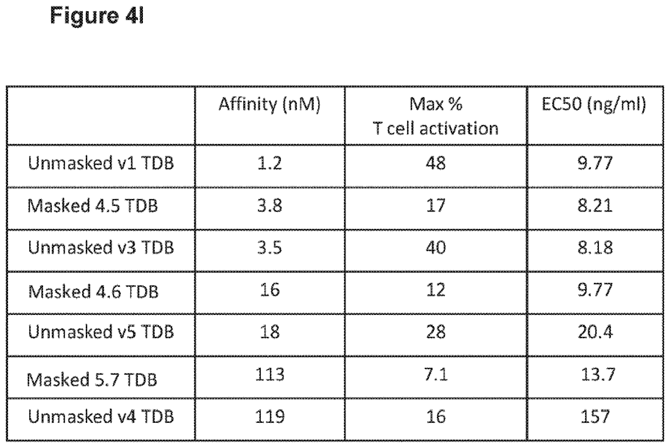

FIGURE 4I is a table summarizing the binding affinity, T cell activation, and cytotoxic activity for affinity variants of unmasked CD3/CD20 TDBs (unmasked v1, unmasked v3, unmasked v4, and unmasked v5) and masked CD3/CD20 TDBs (masked 4.5, masked 4.6, and masked 5.7).

### DETAILED DESCRIPTION OF EMBODIMENTS OF THE INVENTION

### I. DEFINITIONS

The term "about" as used herein refers to the usual error range for the respective value readily known to the skilled person in this technical field. Reference to "about" a value or parameter herein includes (and describes) embodiments that are directed to that value or parameter per se.

The terms "anti-CD3 antibody" and "an antibody that binds to CD3" refer to an antibody that is capable of binding CD3 with sufficient affinity such that the antibody is useful as a diagnostic and/or therapeutic agent in targeting CD3. In one embodiment, the extent of binding of an anti-CD3 antibody to an unrelated, non-CD3 protein is less than about 10% of the binding of the antibody to CD3 as measured, e.g., by a radioimmunoassay (RIA). In certain embodiments, an antibody that binds to CD3 has a dissociation constant (Kd) of ≤ 1µM, ≤ 100 nM, ≤ 10 nM, ≤ 1 nM, ≤ 0.1 nM, ≤ 0.01 nM, or ≤ 0.001 nM (e.g. 10⁻⁸ M or less, e.g. from 10⁻⁸ M to 10⁻¹³ M, e.g., from 10⁻⁹ M to 10⁻¹³ M). In certain embodiments, an anti-CD3 antibody binds to an epitope of CD3 that is conserved among CD3 from different species. In certain embodiments, the anti-CD3 antibody is masked (i.e., it contains a polypeptide mask).

The term "antibody" herein is used in the broadest sense and encompasses various antibody structures, including but not limited to monoclonal antibodies, polyclonal antibodies, multispecific antibodies (e.g., bispecific antibodies), and antibody fragments so long as they exhibit the desired antigen-binding activity.

An "antibody fragment" refers to a molecule other than an intact antibody that comprises a portion of an intact antibody that binds the antigen to which the intact antibody binds. Examples of antibody fragments include but are not limited to Fv, Fab, Fab', Fab'-SH, F(ab')₂; diabodies; bispecific diabodies (e.g., bsDb); single chain diabodies (e.g., scDb); linear antibodies; single-chain antibody molecules (e.g. scFv); tandem single-chain antibody molecules (e.g., TaFv); dual affinity retargeting molecules (e.g., DART); bispecific T-cell engagers (e.g., BiTE); variable domain of heavy chain-only antibody molecules (e.g., V_{H}H); and multispecific antibodies formed from antibody fragments.

By "binding domain" is meant a part of a compound or a molecule that specifically binds to a target epitope, antigen, ligand, or receptor. Binding domains include but are not limited to antibodies (e.g., monoclonal, polyclonal, recombinant, humanized, and chimeric antibodies), antibody fragments or portions thereof (e.g., Fab fragments, Fab', (Fab')₂, Fab'-SH, Fv antibodies, scFv antibodies, TaFv antibodies, SMIP, domain antibodies, diabodies, bsDb, scDb, DART, BiTE, minibodies, scFv-Fc, affibodies, nanobodies, V_{H}H domain antibodies, and VH and/or VL domains of antibodies), receptors, ligands, aptamers, and other molecules having an identified binding partner.

A "chemotherapeutic agent" is a chemical compound useful in the treatment of cancer. Examples of chemotherapeutic agents include alkylating agents such as thiotepa and cyclosphosphamide (CYTOXAN®); alkyl sulfonates such as busulfan, improsulfan and piposulfan; aziridines such as benzodopa, carboquone, meturedopa, and uredopa; ethylenimines and methylamelamines including altretamine, triethylenemeiamine, triethylenephosphoramide, triethylenethiophosphoramide and trimethylomelamine; acetogenins (especially bullatacin and bullatacinone); delta-9-tetrahydrocannabinol (dronabinol, MARiNOL®); beta-lapachone; lapachol; colchicines; betulinic acid; a camptothecin (including the synthetic analogue topotecan (HYCAMTIN®), CPT-11 (irinotecan, CAMPTOSAR®), acetylcamptothecin, scopolectin, and 9-aminocamptothecin); bryostatin; callystatin; CC-1065 (including its adozelesin, carzelesin and bizelesin synthetic analogues); podophyllotoxin; podophyllinic acid; teniposide; cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards such as chlorambucil, chlomaphazine, chlorophosphamide, estramustine, ifosfamide, mechlorethamine, mechlorethamine oxide hydrochloride, melphalan, novembichin, phenesterine, prednimustine, trofosfamide, uracil mustard; nitrosoureas such as carmustine, chlorozotocin, fotemustine, lomustine, nimustine, and ranimnustine; antibiotics such as the enediyne antibiotics (e.g., calicheamicin, especially calicheamicin gamma1I and calicheamicin omegaII (see, e.g., Nicolaou et al., Angew. Chem Intl. Ed. Engl., 33: 183-186 (1994)); CDP323, an oral alpha-4 integrin inhibitor; dynemicin, including dynemicin A; an esperamicin; as well as neocarzinostatin chromophore and related chromoprotein enediyne antibiotic chromophores), aclacinomysins, actinomycin, authramycin, azaserine, bleomycins, cactinomycin, carabicin, caminomycin, carzinophtlin, chromomycins, dactinomycin, daunorubicin, detorubicin, 6-diazo-5-oxo-L-norleucine, doxorubicin (including ADRIAMYCIN®, morpholino-doxorubicin, cyanornorpholino-doxorubicin, 2-pyrrolino-doxorubicin, doxorubicin HCl liposome injection (DOXIL®), liposomal doxorubicin TLC D-99 (MYOCET®), peglylated liposomal doxorubicin (CAELYX®), and deoxydoxorubicin), epirubicin, esorubicin, idarubicin, marcellomycin, mitomycins such as mitomycin C, mycophenolic acid, nogalamycin, olivomycins, pepiomycin, porfiromycin, puromycin, quelamycin, rodorubicin, streptonigrin, streptozocin, tubercidin, ubenimex, zinostatin, zorubicin; antimetabolites such as methotrexate, gemcitabine (GEMZAR®), tegafur (UFTORAL®), capecitabine (XELODA®), an epothilone, and 5-fluorouracil (5-FU); combretastatin; folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, triiostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elformithine; elliptinium acetate; an epothilone; etoglucid; gallium nitrate; hydroxyurea; lentinan; Ionidainine; maytansinoids such as maytansine and ansamitocins; mitoguazone; mitoxantrone; mopidanmol; nitraerine; pentostatin; phenamet; pirarubicin; losoxantrone; 2-ethylhydrazide; procarbazine; PSK® polysaccharide complex (JHS Natural Products, Eugene, Oreg.); razoxane; rhizoxin; sizofuran; spirogermanium; tenuazonic acid; triaziquone; 2,2',2'-trichlorotriethylamine; trichothecenes (especially T-2 toxin, verracurin A, roridin A and anguidine); urethan; vindesine (ELDISINE®, FILDESIN®); dacarbazine; mannomustine; mitobronitol; mitolactol; pipobroman; gacytosine; arabinoside ("Ara-C"); thiotepa; taxoid, e.g., paclitaxel (TAXOL®, Bristol-Myers Squibb Oncology, Princeton, N.J.), albumin-engineered nanoparticle formulation of paclitaxel (ABRAXANE™), and docetaxel (TAXOTERE®, Rhome-Poulene Rorer, Antony, France); chloranbucil; 6-thioguanine; mercaptopurine; methotrexate; platinum agents such as cisplatin, oxaliplatin (e.g., ELOXATIN®), and carboplatin; vincas, which prevent tubulin polymerization from forming microtubules, including vinblastine (VELBAN®), vincristine (ONCOVIN®), vindesine (ELDISINE®, FILDESIN®), and vinorelbine (NAVELBINE®); etoposide (VP-16): ifosfamide; mitoxantrone; leucovorin; novantrone; edatrexate; daunomycin; aminopterin; ibandronate; topoisomerase inhibitor RFS 2000; difluoromethylornithine (DMFO); retinoids such as retinoic acid, including bexarotene (TARGRETIN®); bisphosphonates such as clodronate (for example, BONEFOS® or OSTAC®), etidronate (DIDROCAL®), NE-58095, zoledronic acid/zoledronate (ZOMETA®), alendronate (FOSAMAX®), pamidronate (AREDIA®), tiludronate (SKELID®), or risedronate (ACTONEL®); troxacitabine (a 1,3-dioxolane nucleoside cytosine analog); antisense oligonucleotides, particularly those that inhibit expression of genes in signaling pathways implicated in aberrant cell proliferation, such as, for example, PKC-alpha, Raf, H-Ras, and epidermal growth factor receptor (EGF-R) (e.g., erlotinib (Tarceva™)); and VEGF-A that reduce cell proliferation; vaccines such as THERATOPE® vaccine and gene therapy vaccines, for example, ALLOVECTIN® vaccine, LEUVECTIN® vaccine, and VAXID® vaccine; topoisomerase 1 inhibitor (e.g., LURTOTECAN®); rmRH (e.g., ABARELIX®); BAY439006 (sorafenib; Bayer); SU-11248 (sunitinib, SUTENT®, Pfizer); perifosine, COX-2 inhibitor (e.g. celecoxib or etoricoxib), proteosome inhibitor (e.g. PS341); bortezomib (VELCADE®); CCI-779; tipifarnib (R11577); orafenib, ABT510; Bcl-2 inhibitor such as oblimersen sodium (GENASENSE®); pixantrone; EGFR inhibitors; tyrosine kinase inhibitors; serine-threonine kinase inhibitors such as rapamycin (sirolimus, RAPAMUNE®); farnesyltransferase inhibitors such as lonafarnib (SCH 6636, SARASAR™); and pharmaceutically acceptable salts, acids or derivatives of any of the above; as well as combinations of two or more of the above such as CHOP, an abbreviation for a combined therapy of cyclophosphamide, doxorubicin, vincristine, and prednisolone; and FOLFOX, an abbreviation for a treatment regimen with oxaliplatin (ELOXATIN™) combined with 5-FU and leucovorin, and pharmaceutically acceptable salts, acids or derivatives of any of the above; as well as combinations of two or more of the above.

Chemotherapeutic agents as defined herein include "anti-hormonal agents" or "endocrine therapeutics" which act to regulate, reduce, block, or inhibit the effects of hormones that can promote the growth of cancer. They may be hormones themselves, including, but not limited to: anti-estrogens and selective estrogen receptor modulators (SERMs), including, for example, tamoxifen (including NOLVADEX® tamoxifen), raloxifene, droloxifene, 4-hydroxytamoxifen, trioxifene, keoxifene, LY117018, onapristone, and FARESTON.cndot.toremifene; aromatase inhibitors that inhibit the enzyme aromatase, which regulates estrogen production in the adrenal glands, such as, for example, 4(5)-imidazoles, aminoglutethimide, MEGASE® megestrol acetate, AROMASIN® exemestane, formestanie, fadrozole, RIVISOR® vorozole, FEMARA® letrozole, and ARIMIDEX® anastrozole; and anti-androgens such as flutamide, nilutamide, bicalutamide, leuprolide, and goserelin; as well as troxacitabine (a 1,3-dioxolane nucleoside cytosine analog); antisense oligonucleotides, particularly those which inhibit expression of genes in signaling pathways implicated in abherant cell proliferation, such as, for example, PKC-alpha, Raf and H-Ras; ribozymes such as a VEGF expression inhibitor (e.g., ANGIOZYME® ribozyme) and a HER2 expression inhibitor; vaccines such as gene therapy vaccines, for example, ALLOVECTIN® vaccine, LEUVECTIN® vaccine, and VAXID® vaccine; PROLEUKIN® rIL-2; LURTOTECAN® topoisomerase 1 inhibitor; ABARELIX® rmRH; Vinorelbine and Esperamicins (see U.S. Pat. No. 4,675,187), and pharmaceutically acceptable salts, acids or derivatives of any of the above; as well as combinations of two or more of the above.

The term "chimeric" antibody refers to an antibody in which a portion of the heavy and/or light chain is derived from a particular source or species, while the remainder of the heavy and/or light chain is derived from a different source or species.

The term "cleavable moiety" or "CM" refers to an optional component of a polypeptide mask that, when present, joins the polypeptide mask to an antibody, or antigen-binding fragment thereof (e.g., an anti-CD3 antibody of the invention, or antigen-binding fragment thereof, e.g., a CD20/CD3 TDB of the invention, or antigen-binding fragment thereof) and, when cleaved, results in the physical separation of the polypeptide mask from the antibody, or antigen-binding fragment thereof, to which it was joined. In some embodiments, the CM may include an amino acid sequence that can serve as a substrate for an enzyme (e.g., a protease, such as a protease that is co-expressed or up-regulated by the target cell other than the targeted immune effector cell). In other embodiments, the CM comprises a cysteine-cysteine pair capable of forming a disulfide bond, which can be cleaved by action of a reducing agent. In other embodiments, the CM comprises a substrate capable of being cleaved upon photolysis. In some embodiments, the CM comprises an acid-labile linker that is capable of being cleaved in an acidic pH environment (e.g., the lysosome of a cell or a tumor microenvironment).

The term "cluster of differentiation 3" or "CD3," as used herein, refers to any native CD3 from any vertebrate source, including mammals such as primates (e.g. humans) and rodents (e.g., mice and rats), unless otherwise indicated, including, for example, CD3ε, CD3γ, CD3α, and CD3β chains, and encompasses full-length, "unprocessed" CD3 (e.g., unprocessed or unmodified CD3ε or CD3γ), as well as any form of CD3 that results from processing in the cell, such as a "processed" CD3ε polypeptide without all or a portion of its signal peptide, including, in particular, a CD3ε polypeptide without the first 21 or 22 amino acids of the sequence of NCBI RefSeq No. NP_000724 (human CD3ε protein). The term also encompasses naturally other occurring variants of CD3, including, for example, splice variants or allelic variants. CD3 includes, for example, both human CD3ε protein (NCBI RefSeq No. NP_000724), which is 207 amino acids in length, and human CD3γ protein (NCBI RefSeq No. NP_000064), which is 182 amino acids in length, in unprocessed or processed form.

The "class" of an antibody refers to the type of constant domain or constant region possessed by its heavy chain. There are five major classes of antibodies: IgA, IgD, IgE, IgG, and IgM, and several of these may be further divided into subclasses (isotypes), e.g., IgG1, IgG₂, IgG₃, IgG₄, IgA₁, and IgA2, The heavy chain constant domains that correspond to the different classes of immunoglobulins are called α, δ, ε, γ, and µ, respectively.

It is understood that aspects and embodiments of the invention described herein include "comprising," "consisting," and "consisting essentially of" aspects and embodiments.

The term "cytotoxic agent" as used herein refers to a substance that inhibits or prevents a cellular function and/or causes cell death or destruction. Cytotoxic agents include, but are not limited to, radioactive isotopes (e.g., At²¹¹, I¹³¹, I¹²⁵, Y⁹⁰, Re¹⁸⁶, Re¹⁸⁶, Sm¹⁵³, Bi²¹², P³², Pb²¹² and radioactive isotopes of Lu); chemotherapeutic agents or drugs (e.g., methotrexate, adriamicin, vinca alkaloids (vincristine, vinblastine, etoposide), doxorubicin, melphalan, mitomycin C, chlorambucil, daunorubicin or other intercalating agents); growth inhibitory agents; enzymes and fragments thereof such as nucleolytic enzymes; antibiotics; toxins such as small molecule toxins or enzymatically active toxins of bacterial, fungal, plant or animal origin, including fragments and/or variants thereof; and the various antitumor or anticancer agents disclosed below.

A "disorder" is any condition that would benefit from treatment including, but not limited to, chronic and acute disorders or diseases including those pathological conditions which predispose the mammal to the disorder in question.

The terms "cell proliferative disorder" and "proliferative disorder" refer to disorders that are associated with some degree of abnormal cell proliferation. In one embodiment, the cell proliferative disorder is cancer. In one embodiment, the cell proliferative disorder is a tumor.

The terms "cancer" and "cancerous" refer to or describe the physiological condition in mammals that is typically characterized by unregulated cell growth. Examples of cancer include but are not limited to, carcinoma, lymphoma, blastoma, sarcoma, and leukemia or lymphoid malignancies. More particular examples of such cancers include, but not limited to, squamous cell cancer (*e.g*., epithelial squamous cell cancer), lung cancer including small-cell lung cancer, non-small cell lung cancer, adenocarcinoma of the lung and squamous carcinoma of the lung, cancer of the peritoneum, hepatocellular cancer, gastric or stomach cancer including gastrointestinal cancer and gastrointestinal stromal cancer, pancreatic cancer, glioblastoma, cervical cancer, ovarian cancer, liver cancer, bladder cancer, cancer of the urinary tract, hepatoma, breast cancer, colon cancer, rectal cancer, colorectal cancer, endometrial or uterine carcinoma, salivary gland carcinoma, kidney or renal cancer, prostate cancer, vulval cancer, thyroid cancer, hepatic carcinoma, anal carcinoma, penile carcinoma, melanoma, superficial spreading melanoma, lentigo maligna melanoma, acral lentiginous melanomas, nodular melanomas, multiple myeloma and B-cell lymphoma (including low grade/follicular non-Hodgkin's lymphoma (NHL); small lymphocytic (SL) NHL; intermediate grade/follicular NHL; intermediate grade diffuse NHL; high grade immunoblastic NHL; high grade lymphoblastic NHL; high grade small non-cleaved cell NHL; bulky disease NHL; mantle cell lymphoma; AIDS-related lymphoma; and Waldenstrom's Macroglobulinemia); chronic lymphocytic leukemia (CLL); acute lymphoblastic leukemia (ALL); hairy cell leukemia; chronic myeloblastic leukemia; and post-transplant lymphoproliferative disorder (PTLD), as well as abnormal vascular proliferation associated with phakomatoses, edema (such as that associated with brain tumors), Meigs' syndrome, brain, as well as head and neck cancer, and associated metastases. In certain embodiments, cancers that are amenable to treatment by the antibodies of the invention include breast cancer, colorectal cancer, rectal cancer, non-small cell lung cancer, glioblastoma, non-Hodgkins lymphoma (NHL), renal cell cancer, prostate cancer, liver cancer, pancreatic cancer, soft-tissue sarcoma, kaposi's sarcoma, carcinoid carcinoma, head and neck cancer, ovarian cancer, mesothelioma, and multiple myeloma. In some embodiments, the cancer is selected from: small cell lung cancer, gliblastoma, neuroblastomas, melanoma, breast carcinoma, gastric cancer, colorectal cancer (CRC), and hepatocellular carcinoma. Yet, in some embodiments, the cancer is selected from: non-small cell lung cancer, colorectal cancer, glioblastoma and breast carcinoma, including metastatic forms of those cancers.

"Tumor," as used herein, refers to all neoplastic cell growth and proliferation, whether malignant or benign, and all pre-cancerous and cancerous cells and tissues. The terms "cancer", "cancerous", "cell proliferative disorder", "proliferative disorder" and "tumor" are not mutually exclusive as referred to herein.

The term "tumor antigen," as used herein, may be understood as those antigens that are presented on tumor cells. These antigens can be presented on the cell surface with art extracellular part, which is often combined with a transmembrane and cytoplasmic part of the molecule. These antigens can sometimes be presented only by tumor cells and never by the normal ones. Tumor antigens can be exclusively expressed on tumor cells or might represent a tumor specific mutation compared to normal cells. In this case, they are called tumor-specific antigens. More common are tumor antigens that are presented by tumor cells and normal cells, and they are called tumor-associated antigens. These tumor-associated antigens can be overexpressed compared to normal cells or are accessible for antibody binding in tumor cells due to the less compact structure of the tumor tissue compared to normal tissue. In one aspect the tumor antigen is selected from those set forth in Table 1 below.

"Effector functions" refer to those biological activities attributable to the Fc region of an antibody, which vary with the antibody isotype. Examples of antibody effector functions include: C1q binding and complement dependent cytotoxicity (CDC); Fc receptor binding; antibody-dependent cell-mediated cytotoxicity (ADCC); phagocytosis; down regulation of cell surface receptors (e.g. B cell receptor); and B cell activation.

An "effective amount" of a compound, for example, an anti-CD3 antibody of the invention or a composition (e.g., pharmaceutical composition) thereof, is at least the minimum amount required to achieve the desired therapeutic or prophylactic result, such as a measurable improvement or prevention of a particular disorder (e.g., a cell proliferative disorder, e.g., cancer). An effective amount herein may vary according to factors such as the disease state, age, sex, and weight of the patient, and the ability of the antibody to elicit a desired response in the individual. An effective amount is also one in which any toxic or detrimental effects of the treatment are outweighed by the therapeutically beneficial effects. For prophylactic use, beneficial or desired results include results such as eliminating or reducing the risk, lessening the severity, or delaying the onset of the disease, including biochemical, histological and/or behavioral symptoms of the disease, its complications and intermediate pathological phenotypes presenting during development of the disease. For therapeutic use, beneficial or desired results include clinical results such as decreasing one or more symptoms resulting from the disease, increasing the quality of life of those suffering from the disease, decreasing the dose of other medications required to treat the disease, enhancing effect of another medication such as via targeting, delaying the progression of the disease, and/or prolonging survival. In the case of cancer or tumor, an effective amount of the drug may have the effect in reducing the number of cancer cells; reducing the tumor size; inhibiting (*i.e.,* slow to some extent or desirably stop) cancer cell infiltration into peripheral organs; inhibit (*i.e*., slow to some extent and desirably stop) tumor metastasis; inhibiting to some extent tumor growth; and/or relieving to some extent one or more of the symptoms associated with the disorder. An effective amount can be administered in one or more administrations. For purposes of this invention, an effective amount of drug, compound, or pharmaceutical composition is an amount sufficient to accomplish prophylactic or therapeutic treatment either directly or indirectly. As is understood in the clinical context, an effective amount of a drug, compound, or pharmaceutical composition may or may not be achieved in conjunction with another drug, compound, or pharmaceutical composition. Thus, an "effective amount" may be considered in the context of administering one or more therapeutic agents, and a single agent may be considered to be given in an effective amount if, in conjunction with one or more other agents, a desirable result may be or is achieved.

The term "Fc region" herein is used to define a C-terminal region of an immunoglobulin heavy chain that contains at least a portion of the constant region. The term includes native sequence Fc regions and variant Fc regions. In one embodiment, a human IgG heavy chain Fc region extends from Cys226, or from Pro230, to the carboxyl-terminus of the heavy chain. However, the C-terminal lysine (Lys447) of the Fc region may or may not be present. Unless otherwise specified herein, numbering of amino acid residues in the Fc region or constant region is according to the EU numbering system, also called the EU index, as described in Kabat et al., Sequences of Proteins of Immunological interest, 5th Ed. Public Health Service, National Institutes of Health, Bethesda, MD, 1991.

"Framework" or "FR" refers to variable domain residues other than hypervariable region (HVR) residues. The FR of a variable domain generally consists of four FR domains: FR1, FR2, FR3, and FR4. Accordingly, the HVR and FR sequences generally appear in the following sequence in VH (or VL): FR1-H1 (L.1)-FR2-H2(L2)-FR3-H3(L3)-FR4.

The terms "full-length antibody," "intact antibody," and "whole antibody" are used herein interchangeably to refer to an antibody having a structure substantially similar to a native antibody structure or having heavy chains that contain an Fc region as defined herein.

A "growth inhibitory agent" when used herein refers to a compound or composition which inhibits growth of a cell either *in vitro* or *in vivo.* In one embodiment, growth inhibitory agent is growth inhibitory antibody that prevents or reduces proliferation of a cell expressing an antigen to which the antibody binds. In another embodiment, the growth inhibitory agent may be one which significantly reduces the percentage of cells in S phase. Examples of growth inhibitory agents include agents that block cell cycle progression (at a place other than S phase), such as agents that induce G1 arrest and M-phase arrest. Classical M-phase blockers include the vincas (vincristine and vinblastine), taxanes, and topoisomerase II inhibitors such as doxorubicin, epirubicin, daunorubicin, etoposide, and bleomycin. Those agents that arrest G1 also spill over into S-phase arrest, for example, DNA alkylating agents such as tamoxifen, prednisone, dacarbazine, mechlorethamine, cisplatin, methotrexate, 5-fluorouracil, and ara-C. Further information can be found in Mendelsohn and Israel, eds., The Molecular Basis of Cancer, Chapter 1, entitled "Cell cycle regulation, oncogenes, and antineoplastic drugs" by Murakami et al (W.B. Saunders, Philadelphia, 1995), e.g., p. 13. The taxanes (paclitaxel and docetaxel) are anticancer drugs both derived from the yew tree. Docetaxel (TAXOTERE®, Rhone-Poulenc Rorer), derived from the European yew, is a semisynthetic analogue of paclitaxel (TAXOL®, Bristol-Myers Squibb). Paclitaxel and docetaxel promote the assembly of microtubules from tubulin dimers and stabilize microtubules by preventing depolymerization, which results in the inhibition of mitosis in cells.

The terms "host cell," "host cell line," and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein.

A "human antibody" is one which possesses an amino acid sequence which corresponds to that of an antibody produced by a human or a human cell or derived from a non-human source that utilizes human antibody repertoires or other human antibody-encoding sequences. This definition of a human antibody specifically excludes a humanized antibody comprising non-human antigen-binding residues. Human antibodies can be produced using various techniques known in the art, including phage-display libraries. Hoogenboom and Winter, J. Mol. Biol., 227:381(1991); Marks et al., J. Mol. Biol., 222:581 (1991). Also available for the preparation of human monoclonal antibodies are methods described in Cole et a/., Monoclonal Antibodies and Cancer Therapy, Alan R. Liss, p. 77 (1985); Boerner et al., J. Immunol.. 147(1):86-95 (1991). See also van Dijk and van de Winkel, Curr. Opin. Pharmacol., 5: 368-74 (2001). Human antibodies can be prepared by administering the antigen to a transgenic animal that has been modified to produce such antibodies in response to antigenic challenge, but whose endogenous loci have been disabled, e.g., immunized xenomice (see, e.g., U.S. Pat. Nos. 6,075,181 and 6,150,584 regarding XENOMOUSE™ technology). See also, for example, Li et al., Proc. Natl. Acad. Sci. USA, 103:3557-3562 (2006) regarding human antibodies generated via a human B-cell hybridoma technology.

The term "hypervariable region" or "HVR" as used herein refers to each of the regions of an antibody variable domain which are hypervariable in sequence ("complementarity determining regions" or "CDRs") and/or form structurally defined loops ("hypervariable loops") and/or contain the antigen-contacting residues ("antigen contacts"). Generally, antibodies comprise six HVRs: three in the VH (H1, H2, H3), and three in the VL (L1, L2, L3). Exemplary HVRs herein include:
(a) hypervariable loops occurring at amino acid residues 26-32 (L1), 50-52 (L2), 91-96 (L3), 26-32 (H1), 53-55 (H2), and 96-101 (H3) (Chothia and Lesk, J. Mol. Biol. 196:901-917 (1987));
(b) CDRs occurring at amino acid residues 24-34 (L1), 50-56 (L2), 89-97 (L3), 31-35b (H1), 50-65 (H2), and 95-102 (H3) (Kabat et al., Sequences of Proteins of Immunological Interest, 5th Ed. Public Health Service, National Institutes of Health, Bethesda, MD (1991));
(c) antigen contacts occurring at amino acid residues 27c-36 (L1), 46-55 (L2), 89-96 (L3), 30-35b (H1), 47-58 (H2), and 93-101 (H3) (MacCallum et al. J. Mol. Biol. 262: 732-745 (1996)); and
(d) combinations of (a), (b), and/or (c), including HVR amino acid residues 46-56 (L2), 47-56 (L2), 48-56 (L2), 49-56 (L2), 26-35 (H1), 26-35b (H1), 49-65 (H2), 93-102 (H3), and 94-102 (H3).

Unless otherwise indicated, HVR residues and other residues in the variable domain (e.g., FR residues) are numbered herein according to Kabat et al., *supra.*

An "immunoconjugate" is an antibody conjugated to one or more heterologous molecule(s), including but not limited to a cytotoxic agent.

A "subject" or an "individual" is a mammal. Mammals include, but are not limited to, domesticated animals (e.g., cows, sheep, cats, dogs, and horses), primates (e.g., humans and non-human primates such as monkeys), rabbits, and rodents (e.g., mice and rats). In certain embodiments, the subject or individual is a human.

An "isolated" antibody is one which has been separated from a component of its natural environment. In some embodiments, an antibody is purified to greater than 95% or 99% purity as determined by, for example, electrophoretic (e.g., SDS-PAGE, isoelectric focusing (IEF), capillary electrophoresis) or chromatographic (e.g., ion exchange or reverse phase HPLC). For review of methods for assessment of antibody purity, see, e.g., Flatman et al., J. Chromatogr. B 848:79-87 (2007).

An "isolated" nucleic acid refers to a nucleic acid molecule that has been separated from a component of its natural environment. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

"Isolated nucleic acid encoding an anti-CD3 antibody" refers to one or more nucleic acid molecules encoding antibody heavy and light chains (or fragments thereof), including such nucleic acid molecule(s) in a single vector or separate vectors, and such nucleic acid molecule(s) present at one or more locations in a host cell.

By a "linker moiety" or "LM" as used herein is meant an optional component of a polypeptide mask that, when present, joins the MM component of the polypeptide mask directly or indirectly to an antibody, or antigen-binding fragment thereof (e.g., an anti-CD3 antibody of the invention, or antigen-binding fragment thereof, e.g., a CD20/CD3 TDB of the invention, or antigen-binding fragment thereof). In general, LMs are of between 5-24 amino acids in length (e.g., 5, 6, 7, 8, 9, 10, 11, 12, 13,14, 15, 16, 17, 18, 19, 20, 21, 22, 23, or 24 amino acids in length). In some embodiments, the LM is between 5-15 amino acids in length (e.g., 5,6, 7, 8, 9, 10, 11, 12, 13, 14, or 15 amino acids in length). In some embodiments, the LM is highly flexible and may be rich in glycine (G) and/or serine (S) residues, which may be present in the form of GS repeats.

The term "masking moiety" or "MM" refers to a component of a polypeptide mask that reduces the ability of an antibody, or antigen-binding fragment thereof (e.g., an anti-CD3 antibody of the invention, or antigen-binding fragment thereof, e.g., a CD20/CD3 TDB of the invention, or antigen-binding fragment thereof), to specifically bind its target (e.g., CD3). In some embodiments, the MM includes an amino acid sequence comprising a fragment of a CD3 polypeptide (e.g., a human CD3ε polypeptide, e.g., an N-terminal fragment of a human CD3ε polypeptide, e.g., at least the first three amino acids of a processed human CD3ε polypeptide). In some embodiments, the MM includes an amino acid sequence comprising an N-terminal cyclicized glutamine (also referred to as pyroglutamic acid, 5-oxopyrrolidine-2-carboxylic acid (PCA), 5-oxoproline, pidolic acid, or pyroglutamate). The MM may be joined to the antibody, or antigen-binding fragment thereof, via a LM, a CM, or both a LM and a CM. Alternatively, the MM may be joined directly to the antibody, or antigen-binding fragment thereof.

The term "monoclonal antibody" as used herein refers to an antibody obtained from a population of substantially homogeneous antibodies, i.e., the individual antibodies comprising the population are identical and/or bind the same epitope, except for possible variant antibodies, e.g., containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen. Thus, the modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including but not limited to the hybridoma method, recombinant DNA methods, phage-display methods, and methods utilizing transgenic animals containing all or part of the human immunoglobulin loci, such methods and other exemplary methods for making monoclonal antibodies being described herein.

A "naked antibody" refers to an antibody that is not conjugated to a heterologous moiety (e.g., a cytotoxic moiety) or radiolabel. The naked antibody may be present in a pharmaceutical formulation,

"Native antibodies" refer to naturally occurring immunoglobulin molecules with varying structures. For example, native IgG antibodies are heterotetrameric glycoproteins of about 150,000 daltons, composed of two identical light chains and two identical heavy chains that are disulfide-bonded. From N-to C-terminus, each heavy chain has a variable region (VH), also called a variable heavy domain or a heavy chain variable domain, followed by three constant domains (CH1, CH2, and CH3). Similarly, from N- to C-terminus, each light chain has a variable region (VL), also called a variable light domain or a light chain variable domain, followed by a constant light (CL) domain. The light chain of an antibody may be assigned to one of two types, called kappa (κ) and lambda (λ), based on the amino acid sequence of its constant domain.

The term "package insert" is used to refer to instructions customarily included in commercial packages of therapeutic products, that contain information about the indications, usage, dosage, administration, combination therapy, contraindications and/or warnings concerning the use of such therapeutic products.

The term "PD-1 axis binding antagonist" refers to a molecule that inhibits the interaction of a PD-1 axis binding partner with either one or more of its binding partner, so as to remove T-cell dysfunction resulting from signaling on the PD-1 signaling axis - with a result being to restore or enhance T-cell function (e.g., proliferation, cytokine production, target cell killing). As used herein, a PD-1 axis binding antagonist includes a PD-1 binding antagonist, a PD-L1 binding antagonist and a PD-L2 binding antagonist.

The term "PD-1 binding antagonist" refers to a molecule that decreases, blocks, inhibits, abrogates or interferes with signal transduction resulting from the interaction of PD-1 with one or more of its binding partners, such as PD-L1, PD-L2. In some embodiments, the PD-1 binding antagonist is a molecule that inhibits the binding of PD-1 to one or more of its binding partners. In a specific aspect, the PD-1 binding antagonist inhibits the binding of PD-1 to PD-L1 and/or PD-L2. For example, PD-1 binding antagonists include anti-PD-1 antibodies, antigen binding fragments. thereof, immunoadhesins, fusion proteins, oligopeptides and other molecules that decrease, block, inhibit, abrogate or interfere with signal transduction resulting from the interaction of PD-1 with PD-L1 and/or PD-L2. In one embodiment, a PD-1 binding antagonist reduces the negative co-stimulatory signal mediated by or through cell surface proteins expressed on T lymphocytes mediated signaling through PD-1 so as render a dysfunctional T-cell less dysfunctional (e.g., enhancing effector responses to antigen recognition). In some embodiments, the PD-1 binding antagonist is an anti-PD-1 antibody. In a specific aspect, a PD-1 binding antagonist is MDX-1106 (nivolumab) described herein. In another specific aspect, a PD-1 binding antagonist is MK-3475 (lambrolizumab) described herein. In another specific aspect, a PD-1 binding antagonist is CT-011 (pidilizumab) described herein. In another specific aspect, a PD-1 binding antagonist is AMP-224 described herein.

The term "PD-L1 binding antagonist" refers to a molecule that decreases, blocks, inhibits, abrogates or interferes with signal transduction resulting from the interaction of PD-L1 with either one or more of its binding partners, such as PD-1, B7-1. In some embodiments, a PD-L1 binding antagonist is a molecule that inhibits the binding of PD-L1 to its binding partners. In a specific aspect, the PD-L1 binding antagonist inhibits binding of PD-L1 to PD-1 and/or B7-1. In some embodiments, the PD-L1 binding antagonists include anti-PD-L1 antibodies, antigen binding fragments thereof, immunoadhesins, fusion proteins, oligopeptides and other molecules that decrease, block, inhibit, abrogate or interfere with signal transduction resulting from the interaction of PD-L1 with one or more of its binding partners, such as PD-1, B7-1. In one embodiment, a PD-L1 binding antagonist reduces the negative co-stimulatory signal mediated by or through cell surface proteins expressed on T lymphocytes mediated signaling through PD-L1 so as to render a dysfunctional T-cell less dysfunctional (e.g., enhancing effector responses to antigen recognition). In some embodiments, a PD-L1 binding antagonist is an anti-PD-L1 antibody. In a specific aspect, an anti-PD-L1 antibody is YW243.55.S70 described herein. In another specific aspect, an anti-PD-L1 antibody is MDX-1 105 described herein, in still another specific aspect, an anti-PD-L1 antibody is MPDL3280A described herein. In still another specific aspect, an anti-PD-L1 antibody is MEDI4736 described herein.

The term "PD-L2 binding antagonist" refers to a molecule that decreases, blocks, inhibits, abrogates or interferes with signal transduction resulting from the interaction of PD-L2 with either one or more of its binding partners, such as PD-1. In some embodiments, a PD-L2 binding antagonist is a molecule that inhibits the binding of PD-L2 to one or more of its binding partners. In a specific aspect, the PD-L2 binding antagonist inhibits binding of PD-L2 to PD-1. In some embodiments, the PD-L2 antagonists include anti-PD-L2 antibodies, antigen binding fragments, thereof, immunoadhesins, fusion proteins, oligopeptides and other molecules that decrease, block, inhibit, abrogate or interfere with signal transduction resulting from the interaction of PD-L2 with either one or more of its binding partners, such as PD-1. In one embodiment, a PD-L2 binding antagonist reduces the negative co-stimulatory signal mediated by or through cell surface proteins expressed on T lymphocytes mediated signaling through PD-L2 so as render a dysfunctional T-cell less dysfunctional (e.g., enhancing effector responses to antigen recognition). In some embodiments, a PD-L2 binding antagonist is an immunoadhesin.

"Percent (%) amino acid sequence identity" with respect to a reference polypeptide sequence is defined as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for aligning sequences, including any algorithms needed to achieve maximal alignment over the full length of the sequences being compared. For purposes herein, however, % amino acid sequence identity values are generated using the sequence comparison computer program ALIGN-2. The ALIGN-2 sequence comparison computer program was authored by Genentech, Inc., and the source code has been filed with user documentation in the U.S. Copyright Office, Washington D.C., 20559, where it is registered under U.S. Copyright Registration No. TXU510087. The ALIGN-2 program is publicly available from Genentech, Inc., South San Francisco, California, or may be compiled from the source code. The ALIGN-2 program should be compiled for use on a UNIX operating system, including digital UNIX V4.0D. All sequence comparison parameters are set by the ALIGN-2 program and do not vary.

In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:
100 times the fraction X/Y
where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. Unless specifically stated otherwise, all % amino acid sequence identity values used herein are obtained as described in the immediately preceding paragraph using the ALIGN-2 computer program.

The term "pharmaceutical formulation" refers to a preparation which is in such form as to permit the biological activity of an active ingredient contained therein to be effective, and which contains no additional components which are unacceptably toxic to a subject to which the formulation would be administered.

A "pharmaceutically acceptable carrier" refers to an ingredient in a pharmaceutical formulation, other than an active ingredient, which is nontoxic to a subject. A pharmaceutically acceptable carrier includes, but is not limited to, a buffer, excipient, stabilizer, or preservative.

The term "polypeptide mask" refers to an amino acid sequence joined to an antibody or antigen-binding fragment thereof (e.g., an anti-CD3 antibody of the invention, or antigen-binding fragment thereof, e.g., a CD20/CD3 TDB of the invention, or antigen-binding fragment thereof) and positioned such that it reduces the ability of the antibody, or antigen-binding fragment thereof, to specifically bind its target (e.g., CD3). In some embodiments, the polypeptide mask includes a MM, which may be joined to the antibody or antigen-binding fragment thereof via a LM, a CM, or both a LM and a CM.

As used herein, "treatment" (and grammatical variations thereof such as "treat" or "treating") refers to clinical intervention in an attempt to alter the natural course of the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, antibodies of the invention are used to delay development of a disease or to slow the progression of a disease.

As used herein, "delaying progression" of a disorder or disease means to defer, hinder, slow, retard, stabilize, and/or postpone development of the disease or disorder (e.g., a cell proliferative disorder, e.g., cancer). This delay can be of varying lengths of time, depending on the history of the disease and/or individual being treated. As is evident to one skilled in the art, a sufficient or significant delay can, in effect, encompass prevention, in that the individual does not develop the disease. For example, a late stage cancer, such as development of metastasis, may be delayed.

By "reduce" or "inhibit" is meant the ability to cause an overall decrease, for example, of 20% or greater, of 50% or greater, or of 75%, 85%, 90%, 95%, or greater. In certain embodiments, reduce or inhibit can refer to the overall decrease in the ability of an anti-CD3 antibody to bind to a human CD3 polypeptide when masked (i.e., when the anti-CD3 antibody comprises a polypeptide mask). In other embodiments, reduce or inhibit can refer to the effector function of an antibody that is mediated by the antibody Fc region, such effector functions specifically including complement-dependent cytotoxicity (CDC), antibody-dependent cellular cytotoxicity (ADCC), and antibody-dependent cellular phagocytosis (ADCP).

The term "variable region" or "variable domain" refers to the domain of an antibody heavy or light chain that is involved in binding the antibody to antigen. The variable domains of the heavy chain and light chain (VH and VL, respectively) of a native antibody generally have similar structures, with each domain comprising four conserved framework regions (FRs) and three hypervariable regions (HVRs). (See, e.g., Kindt et al. Kuby Immunology, 6th ed., W.H. Freeman and Co., page 91 (2007).) A single VH or VL domain may be sufficient to confer antigen-binding specificity. Furthermore, antibodies that bind a particular antigen may be isolated using a VH or VL domain from an antibody that binds the antigen to screen a library of complementary VL or VH domains, respectively. See, e.g., Portolano et al., J. Immunol. 150:880-887 (1993); Clarkson et al., Nature 352:624-628 (1991).

The term "vector," as used herein, refers to a nucleic acid molecule capable of propagating another nucleic acid to which it is linked. The term includes the vector as a self-replicating nucleic acid structure as well as the vector incorporated into the genome of a host cell into which it has been introduced, Certain vectors are capable of directing the expression of nucleic acids to which they are operatively linked. Such vectors are referred to herein as "expression vectors."

As used herein, "administering" is meant a method of giving a dosage of a compound (e.g., an anti-CD3 antibody of the invention or a nucleic acid encoding an anti-CD3 antibody of the invention) or a composition (e.g., a pharmaceutical composition, e.g., a pharmaceutical composition including an anti-CD3 antibody of the invention) to a subject. The compositions utilized in the methods described herein can be administered, for example, intramuscularly, intravenously, intradermally, percutaneously, intraarterially, intraperitoneally, intralesionally, intracranially, intraarticularly, intraprostatically, intrapleurally, intratracheally, intranasally, intravitreally, intravaginally, intrarectally, topically, intratumorally, peritoneally, subcutaneously, subconjunctivally, intravesicularily, mucosally, intrapericardially, intraumbilically, intraocularly, orally, topically, locally, by inhalation, by injection, by infusion, by continuous infusion, by localized perfusion bathing target cells directly, by catheter, by lavage, in cremes, or in lipid compositions. The method of administration can vary depending on various factors (e.g., the compound or composition being administered and the severity of the condition, disease, or disorder being treated).

### II. COMPOSITIONS AND METHODS

In one aspect, the invention is based, in part, on masked anti-CD3 antibodies that include a polypeptide mask. In certain embodiments, the masked anti-CD3 antibodies are multispecific (e.g., bsspecific) and capable of binding, in addition to CD3 or a fragment thereof, a second biological molecule (e.g., a cell surface antigen, e.g., a tumor antigen). The masked antibodies of the invention may be useful, for example, for treating or delaying the progression of a cell proliferative disorder (e.g., cancer) or an autoimmune disorder, or for enhancing immune function in a subject having such a disorder, in a manner that specifically accounts for and controls the kinetics of T cell engagement and activation.

### A. Masked Anti-CD3 Antibodies

In one aspect, the invention provides masked anti-CD3 antibodies that include (a) a binding domain that is capable of binding to CD3 (e.g., CD3ε and/or CD3γ, e.g., human CD3ε and/or CD3y) and (b) a polypeptide mask that is positioned such that it reduces or inhibits the ability of the antibody, or antigen-binding fragment thereof, to specifically bind CD3. In the specific context of a T cell-dependent bispecific (TDB) antibody that includes an anti-CD3 arm and an anti-tumor target arm, a polypeptide mask joined to the TDB can uniquely shift the cellular biodistribution of engaged tumor target cells and T cells and thereby alter the efficacy of the TDBs.

A polypeptide mask may be joined to the binding domain of an anti-CD3 antibody by its heavy chain variable (VH) domain or light chain variable (VL) domain. In some embodiments, the polypeptide mask is joined to the N-terminus of the VH domain or VL domain of the anti-CD3 antibody. An anti-CD3 antibody joined to or modified with a polypeptide mask can be represented by the following formulae (in order from an amino-terminal region to carboxy-terminal region): (polypeptide mask)-(anti-CD3 antibody).

The polypeptide mask may inhibit the ability of the anti-CD3 antibody to bind to a CD3 polypeptide (e.g., a human CD3 polypeptide, e.g., a human CD3ε polypeptide) by at least 10% (e.g., by 10%, 11%, 12%, 13%, 14%, 15%, 16%, 17%, 18%, 19%, 20%, 21%, 22%, 23%, 24%, 25%, 26%, 27%, 28%, or 29% or more), by at least 30% (e.g., by 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, or 59% or more), by at least 60% (e.g., by 60%, 61%, 62%, 63%, 64%, 65%, 66%, 67%, 68%, 69%, 70%, 71%, 72%, 73%, 74%, 75%, 76%, 77%, 78%, or 79%), by at least 80% (e.g., by 80%, 81%, 82%, 83%, 84%, 85%, 86%, 87%, 88%, or 89%), by at least 90% (e.g., by 90%, 91%, 92%, 93%, 94%, 95%, 96%, 97%, 98%, or 99%), or by about 100%.

Similarly, the presence of a polypeptide mask may result in a dissociation constant (K_{d}) of the masked anti-CD3 antibody for CD3 that is at least 5, 10, 25, 50, 100, 250, 500, 1,000, 2,500, 5,000, 10,000, 50,000, 100,000, 500,000, 1,000,000, 5,000,000, 10,000,000, 50,000,000 or greater, or between 5-10, 10-100, 10-1,000, 10-10,000, 10-100,000, 10-1,000,000, 10-10,000,000, 100-1,000, 100-10,000, 100-100,000, 100-1,000,000, 100-10,000,000, 1,000-10,000, 1,000-100,000, 1,000-1,000,000, 1000-10,000,000, 10,000-100,000, 10,000-1,000,000, 10,000-10,000,000, 100,000-1,000,000, or 100,000-10,000,000 times or greater than the K_{d} of the same anti-CD3 antibody in unmasked form for CD3.

The masked anti-CD3 antibody may have a lower affinity for its polypeptide mask than it has towards its CD3 target. For example, the K_{d} of the anti-CD3 antibody towards its mask can be at least 5, 10, 25, 50, 100, 250, 500, 1,000, 2,500, 5,000, 10,000, or 100,000 times greater than the K_{d} of the anti-CD3 antibody towards CD3. Such a mask may, for example, be useful in instances when the mask does not have a cleavable moiety to facilitate its removal from the anti-CD3 antibody to which it is joined. Alternatively, the masked anti-CD3 antibody may have a higher affinity for its polypeptide mask than it has towards its CD3 target. For example, the K_{d} of the anti-CD3 antibody towards its polypeptide mask can be at least 5, 10, 25, 50, 100, 250, 500, 1,000, 2,500, 5,000, 10,000, or 100,000 times lower than the K_{d} of the anti-CD3 antibody towards CD3. Such a mask may, for example, be useful in instances when the mask has a cleavable moiety to facilitate its removal from the anti-CD3 antibody to which it is joined.

In some instances, the binding domain of the masked anti-CD3 antibody comprises at least one. two, three, four, five, or six hypervariable regions (HVRs) selected from (a) HVR-H1 comprising the amino acid sequence of SEQ ID NO: 2; (b) HVR-H2 comprising the amino acid sequence of SEQ ID NO: 3; (c) HVR-H3 comprising the amino acid sequence of SEQ ID NO: 4; (d) HVR-L1 comprising the amino acid sequence of SEQ ID NO: 5; (e) HVR-L2 comprising the amino acid sequence of SEQ ID NO: 6; and (f) HVR-L3 comprising the amino acid sequence of SEQ ID NO: 7. In some instances, the masked anti-CD3 antibody comprises at least one (e.g., 1, 2, 3, or 4) of heavy chain framework regions FR-H1, FR-H2, FR-H3, and FR-H4 comprising the sequences of SEQ ID NOs: 28-31, respectively, and/or at least one (e.g., 1, 2, 3, or 4) of the light chain framework regions FR-L1, FR-L2, FR-L3, and FR-L4 comprising the sequences of SEQ ID NOs: 32-35, respectively. In some instances, the masked anti-CD3 antibody may have a heavy chain variable (VH) domain including an amino acid sequence having at least 90% sequence identity (e.g., at least 91%, 92%, 93%, 94%, 95%, 96%, 97%, 98%, or 99% sequence identity) to, or the sequence of, SEQ ID NO: 8 and/or a light chain variable (VL) domain comprising an amino acid sequence having at least 90% sequence identity (e.g., at least 91%, 92%, 93%, 94%, 95%, 96%, 97%, 98%, or 99% sequence identity) to, or the sequence of, SEQ ID NO: 9, or a derivative or a clonal relative thereof.

In some instances, the masked antibody of the invention comprises (a) a VH domain comprising at least one, at least two, or all three VH HVR sequences selected from (i) HVR-H1 comprising the amino acid sequence of SEQ ID NO: 2, (ii) HVR-H2 comprising the amino acid sequence of SEQ ID NO: 3, and (iii) HVR-H3 comprising an amino acid sequence selected from SEQ ID NO: 4; and (b) a VL domain comprising at least one, at least two, or all three VL HVR sequences selected from (i) HVR-L1 comprising the amino acid sequence of SEQ ID NO: 5, (ii) HVR-L2 comprising the amino acid sequence of SEQ ID NO: 6, and (iii) HVR-L3 comprising the amino acid sequence of SEQ ID NO: 7. In some instances, the masked anti-CD3 antibody may have a VH domain comprising the amino acid sequence of SEQ ID NO: 8 and a VL domain comprising the amino acid sequence of SEQ ID NO: 9. In some instances, the binding domain of the masked anti-CD3 antibody comprises at least one, two, three, four, five, or six HVRs selected from (a) HVR-H1 comprising the amino acid sequence of SEQ ID NO: 10; (b) HVR-H2 comprising the amino acid sequence of SEQ ID NO: 11; (c) HVR-H3 comprising the amino acid sequence of SEQ ID NO: 12; (d) HVR-L1 comprising the amino acid sequence of SEQ ID NO: 13; (e) HVR-L2 comprising the amino acid sequence of SEQ ID NO: 14; and (f) HVR-L3 comprising the amino acid sequence of SEQ ID NO: 15.

In certain embodiments, any one or more amino acids of an anti-CD3 antibody as provided above are substituted at the following HVR positions:
- in HVR-H3 (SEQ ID NO: 12): positions 1, 2, 5, 6, and 7; and
- in HVR-L3 (SEQ ID NO: 15): positions 1, 2, 4, and 5

In certain embodiments, the substitutions are conservative substitutions, as provided herein. In certain embodiments, any one or more of the following substitutions may be made in any combination:
- in HVR-H3 (SEQ ID NO: 12): D1T or S; S1D or T; T1D or S; G2A or S; A2G or S; S2A or G; R5N; N5R; Y6A; A6Y; A7Y; and Y7A; and
- in HVR-L3 (SEQ ID NO: 15): K1T; T1K; Q2A; A2Q; F4A; A4F; I5A and A5I.

For example, in some instances, the invention provides a masked anti-CD3 antibody having a binding domain comprising at least one, two, three, four, five, or six HVRs selected from (a) HVR-H1 comprising the amino acid sequence of SEQ ID NO: 10; (b) HVR-H2 comprising the amino acid sequence of SEQ ID NO: 11; (c) HVR-H3 comprising the amino acid sequence of SEQ ID NO: 16; (d) HVR-L1 comprising the amino acid sequence of SEQ ID NO: 13; (e) HVR-L2 comprising the amino acid sequence of SEQ ID NO: 14: and (f) HVR-L3 comprising the amino acid sequence of SEQ ID NO: 17. In some instances, the masked anti-CD3 antibody comprises at least one (e.g., 1, 2, 3, or 4) of heavy chain framework regions FR-H1, FR-H2, FR-H3, and FR-H4 comprising the sequences of SEQ ID NOs: 36-39, respectively, and/or at least one (e.g., 1, 2, 3, or 4) of the light chain framework regions FR-L1, FR-L2, FR-L3, and FR-L4 comprising the sequences of SEQ ID NOs: 40-43, respectively. In some instances, the masked anti-CD3 antibody may have a VH domain comprising an amino acid sequence having at least 90% sequence identity (e.g., at least 91%, 92%, 93%, 94%, 95%, 96%, 97%, 98%, or 99% sequence identity) to, or the sequence of, SEQ ID NO: 18 and/or a VL domain comprising an amino acid sequence having at least 90% sequence identity (e.g., at least 91%, 92%, 93%, 94%, 95%, 96%, 97%, 98%, or 99% sequence identity) to, or the sequence of, SEQ ID NO: 19, or a derivative or clonal relative thereof.

In some instances, the invention provides a masked anti-CD3 antibody having a binding domain comprising at least one, two, three, four, five, or six HVRs selected from (a) HVR-H1 comprising the amino acid sequence of SEQ ID NO: 10; (b) HVR-H2 comprising the amino acid sequence of SEQ ID NO: 11; (c) HVR-H3 comprising the amino acid sequence of SEQ ID NO: 16; (d) HVR-L1 comprising the amino acid sequence of SEQ ID NO: 13; (e) HVR-L2 comprising the amino acid sequence of SEQ ID NO: 14; and (f) HVR-L3 comprising the amino acid sequence of SEQ ID NO: 104. In some instances, the masked anti-CD3 antibody comprises at least one (e.g., 1, 2, 3, or 4) of heavy chain framework regions FR-H1, FR-H2, FR-H3, and FR-H4 comprising the sequences of SEQ ID NOs: 36-39, respectively, and/or at least one (e.g., 1, 2, 3, or 4) of the light chain framework regions FR-L1, FR-L2, FR-L3, and FR-L4 comprising the sequences of SEQ ID NOs: 40-43, respectively. In some instances, the masked anti-CD3 antibody may have a VH domain comprising an amino acid sequence having at least 90% sequence identity (e.g., at least 91%, 92%, 93%, 94%, 95%, 96%, 97%, 98%, or 99% sequence identity) to, or the sequence of, SEQ ID NO: 107 and/or a VL domain comprising an amino acid sequence having at least 90% sequence identity (e.g., at least 91%, 92%, 93%, 94%, 95%, 96%, 97%, 98%, or 99% sequence identity) to, or the sequence of, SEQ ID NO: 108, or a derivative or clonal relative thereof.

In some instances, the invention provides a masked anti-CD3 antibody having a binding domain comprising at least one, two, three, four, five, or six HVRs selected from (a) HVR-H1 comprising the amino acid sequence of SEQ ID NO: 10; (b) HVR-H2 comprising the amino acid sequence of SEQ ID NO: 11; (c) HVR-H3 comprising the amino acid sequence of SEQ ID NO: 16; (d) HVR-L1 comprising the amino acid sequence of SEQ ID NO: 13; (e) HVR-L2 comprising the amino acid sequence of SEQ ID NO: 14; and (f) HVR-L3 comprising the amino acid sequence of SEQ ID NO: 105. In some instances, the masked anti-CD3 antibody comprises at least one (e.g., 1, 2, 3, or 4) of heavy chain framework regions FR-H1, FR-H2, FR-H3, and FR-H4 comprising the sequences of SEQ ID NOs: 36-39, respectively, and/or at least one (e.g., 1, 2, 3, or 4) of the light chain framework regions FR-L1, FR-L2, FR-L3, and FR-L4 comprising the sequences of SEQ ID NOs: 40-43, respectively. In some instances, the masked anti-CD3 antibody may have a VH domain comprising an amino acid sequence having at least 90% sequence identity (e.g., at least 91%, 92%, 93%, 94%, 95%, 96%, 97%, 98%, or 99% sequence identity) to, or the sequence of, SEQ ID NO: 109 and/or a VL domain comprising an amino acid sequence having at least 90% sequence identity (e.g., at least 91%, 92%, 93%, 94%, 95%, 96%, 97%, 98%, or 99% sequence identity) to, or the sequence of, SEQ ID NO: 110, or a derivative or clonal relative thereof.

In some instances, the invention provides a masked anti-CD3 antibody having a binding domain comprising at least one, two, three, four, five, or six HVRs selected from (a) HVR-H1 comprising the amino acid sequence of SEQ ID NO: 10; (b) HVR-H2 comprising the amino acid sequence of SEQ ID NO: 11; (c) HVR-H3 comprising the amino acid sequence of SEQ ID NO: 16; (d) HVR-L1 comprising the amino acid sequence of SEQ ID NO: 13; (e) HVR-L2 comprising the amino acid sequence of SEQ ID NO: 14; and (f) HVR-L3 comprising the amino acid sequence of SEQ ID NO: 106. in some instances, the masked anti-CD3 antibody comprises at least one (e.g., 1, 2, 3, or 4) of heavy chain framework regions FR-H1, FR-H2, FR-H3, and FR-H4 comprising the sequences of SEQ ID NOs: 36-39, respectively, and/or at least one (e.g., 1, 2, 3, or 4) of the light chain framework regions FR-L1, FR-L2, FR-L3, and FR-L4 comprising the sequences of SEQ ID NOs: 40-43, respectively. In some instances, the masked anti-CD3 antibody may have a VH domain comprising an amino acid sequence having at least 90% sequence identity (e.g., at least 91%, 92%, 93%, 94%, 95%, 96%, 97%, 98%, or 99% sequence identity) to, or the sequence of, SEQ ID NO: 111 and/or a VL domain comprising an amino acid sequence having at least 90% sequence identity (e.g., at least 91%, 92%, 93%, 94%, 95%, 96%, 97%, 98%, or 99% sequence identity) to, or the sequence of, SEQ ID NO: 112, or a derivative or clonal relative thereof.

Other anti-CD3 antibodies contemplated for advantageous use in a masked form in accordance with the disclosures of the invention include anti-CD3 antibody SP34 (Pessano et al. The EMBO Journal. 4: 337-344, 1985) and the other anti-CD3 antibodies disclosed in U.S.S.N. 14/574,132 (U.S. Pub. No. 2015-0166661), which is incorporated herein by reference in its entirety.

In any of the above embodiments, the masked anti-CD3 antibody may be humanized. For example, a masked anti-CD3 antibody may comprise HVRs as in any of the above embodiments, and further comprise an acceptor human framework, e.g., a human immunoglobulin framework or a human consensus framework.

The masked anti-CD3 antibodies may comprise an aglycosylation site mutation, which can be a substitution mutation at one or more specific residues of the antibody. For example, the aglycosylation site mutation may be a substitution mutation at amino acid residue N297, L234, L235, and/or D265 (EU numbering) (e.g., N297G, N297A, L234A, L235A, and/or D265A). The aglycosylation site mutation can reduce effector function of the masked anti-CD3 antibody.

The masked anti-CD3 antibody according to any one of the above embodiments can be a monoclonal antibody, a chimeric, a humanized, or a human antibody. The masked anti-CD3 antibody can be an antibody fragment, for example, a Fv, Fab, Fab', Fab'-SH, (Fab')₂, scFv, TaFv, diabody, bsDb, scDb, DART, BiTE, or V_{H}H fragment. Alternatively, the masked anti-CD3 antibody can be a full length antibody, e.g., an intact IgG antibody (e.g., an intact IgG1 antibody) or other antibody class or isotype as defined herein.

### 1. Masking Moiety (MM)

In some embodiments, the masked anti-CD3 antibody has a polypeptide mask comprising a masking moiety (MM) comprising the amino acid sequence of at least amino acid residues 1-3 of SEQ ID NO: 1, which corresponds to the first 27 amino acid residues of processed human CD3ε (i.e., human CD3ε without its 21-amino acid signal sequence). Accordingly, the MM can comprise amino acid residues 1 to 3, 1 to 4, 1 to 5, 1 to 6, 1 to 7, 1 to 8, 1 to 9,1 to 10, 1 to 11, 1 to 12, 1 to 13, 1 to 14, 1 to 15, 1 to 16, 1 to 17, 1 to 18, 1 to 19, 1 to 20, 1 to 21, 1 to 22, 1 to 23, 1 to 24, 1 to 25, 1 to 26, or 1 to 27 of SEQ ID NO: 1, or an N-terminal cyclicized glutamine derivative thereof (e.g., a polypeptide mask including a MM having a 5-oxopyrrolidine-2-carboxylic acid (PCA) (also referred to as pyroglutamate, pyroglutamic acid, 5-oxoproline, or pidolic acid) residue at position 1 of SEQ ID NO: 1). In any of the foregoinog embodiments, the MM may include a PCA residue at position 1 of any one of SEQ ID NOs: 1 and 52-103. For example, the MM can include amino acid residues 1-3 of SEQ ID NO: 1, where the glutamine residue at position 1 is replaced with an N-terminal cyclicized glutamine and the amino acid sequence is PCA-D-G. The MM may be positioned relative to the anti-CD3 antibody in an N-terminal to C-terminal direction as (MM)-(anti-CD3 antibody). In some instances, the MM is extended, either directly or indirectly, at one end (i.e., the C-terminal end) by a non-native CD3 polypeptide sequence, such as a cleavable moiety (CM) and/or linker moiety (LM).

### 2. Cleavable Moiety (CM)

As noted above, the masked anti-CD3 antibody may comprise a polypeptide mask having both a MM and a cleavable moiety (CM). In some embodiments, the CM contains an amino acid sequence that is capable of being cleaved by an enzyme, such as a protease. In other embodiments, the CM provides a cysteine-cysteine disulfide bond that is cleavable by reduction. In additional embodiments, the CM provides an acid-labile linker that is cleaved in the presence of an acidic pH environment. In yet other embodiments, the CM provides a photolytic substrate that is activatable by photolysis.

Accordingly, a masked anti-CD3 antibody comprising a polypeptide mask with a CM can exist in either a cleaved state or an uncleaved state. As used herein, the term cleaved state refers to the condition of the anti-CD3 antibody following modification of the CM, for example, by a protease, reduction of a cysteine-cysteine disulfide bond of the CM, and/or photoactivation. The term uncleaved state, as used herein, refers to the condition of the anti-CD3 antibody in the absence of cleavage of the CM, for example, by a protease, in the absence reduction of a cysteine-cysteine disulfide bond of the CM, in the absence of an acidic pH environment (e.g., in a neutral or basic pH environment), and/or in the absence of light. It will be apparent to the ordinarily skilled artisan that in some embodiments a cleaved anti-CD3 antibody may lack an MM due to cleavage of the CM by, for example, a protease, resulting in release of at least the MM. Thus, when a masked anti-CD3 antibody is in the uncleaved state, the masked anti-CD3 antibody would show reduced binding to CD3 because the binding domain of the antibody is effectively masked from the CD3 target molecule. In the cleaved state, the anti-CD3 antibody would show higher affinity for CD3 than an antibody it would in its uncleaved state because the binding domain of the antibody would no longer be inhibited by the MM of the polypeptide mask.

When the CM is capable of being cleaved by an enzyme (e.g., a protease) and the masked anti-CD3 antibody is a TDB, the enzyme may be selected based on a protease that is co-localized in tissue with the desired target of the TDB. A variety of different conditions are known in which a target of interest is co-localized with a protease, where the substrate of the protease is known in the art. In the example of cancer, the target tissue can be a cancerous tissue, particularly cancerous tissue of a solid tumor. Increased levels of proteases having known substrates in a number of cancers, such as solid tumors, are known in the art (see, e.g., La Rocca et al. British J. of Cancer. 90(7): 1414-1421, 2004 and Lopez-Otin et al. Nat Rev Cancer. 7: 800-808, 2007). Exemplary CMs can include, but are not limited to, substrates that are cleavable by one or more of the enzymes (e.g., proteases) specified in WO 2010/081173, WO 2009/025846, WO 2010/096838, and/or one or more of the following enzymes listed below in Table 1.

**Table 1. Exemplary Enzymes**

| Plasmin | Legumain asparaginyl endopeptidase | Transmembrane Protease | Serine (TMPRSS-3/4) |
|---|---|---|---|
| Matrix Metalloprotease (MMP)-1 | MMP-2 | MMP-3 | MMP-7 |
| MMP-8 | MMP-9 | MMP-12 | MMP-13 |
| MMP-14 | Membrane type 1 matrix metalloprotease (MT1-MMP) | Cathepsin A | Cathepsin B |
| Cathepsin D | Cathepsin E | Cathepsin F | Cathepsin H |
| Cathepsin K | Cathepsin K | Cathepsin L | Cathepsin L2 |
| Cathepsin O | Cathepsin S | Caspase 1 | Caspase 2 |
| Caspase 3 | Caspase 4 | Caspase 5 | Caspase 6 |
| Caspase 7 | Caspase 8 | Caspase 9 | Caspase 10 |
| Caspase 11 | Caspase 12 | Caspase 13 | Caspase 14 |
| Human Neutrophil Elastase | Urokinase/urokinase-Type Plasminogen Activator (uPA) | A Disintegrin and Metalloprotease (ADAM)10 | ADAM12 |
| ADAM 17 | ADAM with Thrombospondin Motifs (ADAMTS) | ADAMTS5 | Beta Secretase (BACE) |
| Fibroblast Activation Protease (FAP) | Granzyme A | Granzyme B | Guanidinobenzoatase |
| Gepsin | Matriptase | Matriptase 2 | Meprin |
| Neprilysin | Prostate-Specific Membrane Antigen | Tumor Necrosis Factor-Converting Enzyme | Kallikrein-Related Peptidase (KLK)3 |

| | (PSMA) | (TACE) | |
|---|---|---|---|
| KLK5 | KLK7 | KLK11 | NS3/4 Protease of Hepatitis C Virus (HCV-NS3/4) |
| Tissue Plasminogen Activator (tPA) | Calpain | Calpain 2 | Glutamate Carboxypeptidase II |
| Plasma Kallikrein | AMSH-Like Protease | AMSH | γ-Secretase Component |
| Antiplasmin Cleaving Enzyme (APCE) | Decysin 1 | Apoptosis-Related Cysteine Peptidase | N-Acetylated Alpha-Linked Acidic Dipeptidase-Like 1 |
| Thrombin | | | |

Alternatively or in addition, the masked anti-CD3 antibody can comprise a CM that includes a disulfide bond of a cysteine pair, which is thus cleavable by a reducing agent. These include, but are not limited to, a cellular reducing agent such as glutathione (GSH), thioredoxins, NADPH, flavins, ascorbate, and the like, which can be present in large amounts in tissue of or surrounding a solid tumor.

In other embodiments, the masked anti-CD3 antibody can comprise a CM that includes an acid-labile linker (e.g., a hydrazone, an imino, an ester, or an amido group) which is thus cleavable in the presence of an acidic pH environment, as described in PCT publication number WO 2006/108052, which is herein incorporated by reference in its entirety. This includes, but is not limited to, an acidic pH environment that can be present in lysosomes of a cell or in a tumor microenvironment.

The CM may be positioned relative to the anti-CD3 antibody and MM in an N-terminal to C-terminal direction as (MM)-(CM)-(anti-CD3 antibody).

In other embodiments, the masked anti-CD3 antibody can include one or more (e.g., 2 or 3 or more) distinct CMs within its polypeptide mask.

### 3. Linker Moiety (LM)

As noted above, the masked anti-CD3 antibody may comprise a polypeptide mask having both a MM and a linker moiety (LM), or, alternatively, all three moieties (i.e., a MM, LM, and CM). LMs suitable for use in a polypeptide mask described herein are generally ones that provide flexibility and/or length to the mask to facilitate or modulate the degree of inhibition of the binding of the anti-CD3 antibody to CD3. Such LMs can also be referred to as flexible linkers. Suitable LMs can be readily selected and can be of different suitable lengths, such as from 1 amino acid (e.g., one glycine (G) or one serine (S) residue) to 30 amino acids (e.g., a LM containing a GS repeat sequence). A LM is preferably greater than one amino acid in length (e.g, 2, 3, 4, 5, 6, 7, 8, 9, 10, 11, 12, 13, 14, 15, 16, 17, 18, 19, 20, 21, 22, 23, 24, 25, 26, 27, 28, 29, or 30 or more amino acids in length). In some instances, the LM can be between 5 to 24 amino acids in length, such as between 5 to 15 amino acids in length. The LM may high in G and/or S content (i.e. a G/S-rich LM) and may include GS repeats. For example, the LM may include glycine polymers (G)ₙ, glycine-serine polymers (including, for example, (GS)ₙ, (GSGGS)ₙ and (GGGS)ₙ, where n is an integer of at least one), glycine-alanine polymers, alanine-serine polymers, and other flexible linker combinations known in the art. Glycine and glycine-serine polymers are relatively unstructured, and therefore may be able to serve as a neutral LM that indirectly or directly joins the MM component of the polypeptide mask to the anti-CD3 antibody. Glycine accesses significantly more phi-psi space than even alanine, and is much less restricted than residues with longer side chains (see, e.g., Scheraga. Rev. Computational Chem, 11173-142, 1992). Exemplary flexible linkers include, but are not limited to Gly-Gly-Ser-Gly, Gly-Gly-Ser-Gly-Gly, Gly-Ser-Gly-Ser-Gly, Gly-Ser-Gly-Gly-Gly, Gly-Gly-Gly-Ser-Gly, Gly-Ser-Ser-Ser-Gly, and the like. The ordinarily skilled artisan will recognize that design of a polypeptide mask can include a LM that is completely or partially flexible. For example, a LM may include a flexible portion as well as one or more portions that confer less flexible structure to yield a masked anti-CD3 antibody exhibiting a desired degree of inhibition of CD3 binding, which can be assessed using, for example, an assay such as the phage binding ELISA described in detail below.

When the polypeptide mask does not include a CM, the LM may be positioned relative to the anti-CD3 antibody and MM in an N-terminal to C-terminal direction as (MM)-(LM)-(anti-CD3 antibody). When the polypeptide mask does include a CM, the LM may be positioned relative to the anti-CD3 antibody, MM, and CM in an N-terminal to C-terminal direction as (MM)-(LM)-(CM)-(anti-CD3 antibody) or (MM)-(CM)-(LM)-(anti-CD3 antibody).

In other embodiments, the masked anti-CD3 antibody can include one or more (e.g., 2 or 3 or more) distinct CMs within its polypeptide mask.

The masked anti-CD3 antibody according to any one of the above embodiments may incorporate any of the features, singly or in combination, as described in Sections 4-10 below.

### 4. Antibody Affinity

In certain embodiments, a masked anti-CD3 antibody provided herein has a dissociation constant (K_{d}) of ≤ 1µM, ≤ 100 nM, ≤ 10 nM, ≤ 1 nM, ≤ 0.1 nM, ≤ 0.01 nM, or ≤ 0.001 nM (e.g., 10⁻⁸ M or less, e.g., from 10⁻⁸ M to 10⁻¹³ M, e.g., from 10⁻⁹ M to 10⁻¹³ M). In some instances, the low K_{d} value is only observed upon removal of the polypeptide mask from the anti-CD3 antibody.

In one embodiment, K_{d} is measured by a radiolabeled antigen binding assay (RIA). In one embodiment, an RIA is performed with the Fab version of an antibody of interest and its antigen. For example, solution binding affinity of Fabs for antigen is measured by equilibrating Fab with a minimal concentration of (¹²⁵I)-labeled antigen in the presence of a titration series of unlabeled antigen, then capturing bound antigen with an anti-Fab antibody-coated plate (see, e.g., Chen et al., J. Mol. Biol. 293:865-881(1999)). To establish conditions for the assay, MICROTITER® multi-well plates (Thermo Scientific) are coated overnight with 5 µg/ml of a capturing anti-Fab antibody (Cappel Labs) in 50 mM sodium carbonate (pH 9.6), and subsequently blocked with 2% (w/v) bovine serum albumin in PBS for two to five hours at room temperature (approximately 23°C). In a non-adsorbent plate (Nunc #269620), 100 pM or 26 pM [¹²⁵I]-antigen are mixed with serial dilutions of a Fab of interest (e.g., consistent with assessment of the anti-VEGF antibody, Fab-12, in Presta et al., Cancer Res. 57:4593-4599 (1997)). The Fab of interest is then incubated overnight; however, the incubation may continue for a longer period (e.g., about 65 hours) to ensure that equilibrium is reached. Thereafter, the mixtures are transferred to the capture plate for incubation at room temperature (e.g., for one hour). The solution is then removed and the plate washed eight times with 0.1% polysorbate 20 (TWEEN-20®) in PBS. When the plates have dried, 150 µl/well of scintillant (MICROSCINT-20 ™; Packard) is added, and the plates are counted on a TOPCOUNT ™ gamma counter (Packard) for ten minutes. Concentrations of each Fab that give less than or equal to 20% of maximal binding are chosen for use in competitive binding assays.

According to another embodiment, K_{d} is measured using a BIACORE® surface plasmon resonance assay. For example, an assay using a BIACORE®-2000 or a BIACORE®-3000 (BIAcore, Inc., Piscataway, NJ) is performed at 25°C with immobilized antigen CM5 chips at -10 response units (RU). In one embodiment, carboxymethylated dextran biosensor chips (CM5, BIACORE, Inc.) are activated with *N*-ethyl-*N'*-(3-dimethylaminopropyl)-carbodiimide hydrochloride (EDC) and *N*-hydroxysuccinimide (NHS) according to the supplier's instructions. Antigen is diluted with 10 mM sodium acetate, pH 4.8, to 5 µg/ml (∼0.2 µM) before injection at a flow rate of 5 µl/minute to achieve approximately 10 response units (RU) of coupled protein. Following the injection of antigen, 1 M ethanolamine is injected to block unreacted groups. For kinetics measurements, two-fold serial dilutions of Fab (0.78 nM to 500 nM) are injected in PBS with 0.05% polysorbate 20 (TWEEN-20™) surfactant (PBST) at 25°C at a flow rate of approximately 25 µl/min. Association rates (kₒₙ) and dissociation rates (k_{off}) are calculated using a simple one-to-one Langmuir binding model (BIACORE ® Evaluation Software version 3.2) by simultaneously fitting the association and dissociation sensorgrams. The equilibrium dissociation constant (K_{d}) is calculated as the ratio kₒₙ/k_{off}. See, for example, Chen et al., J. Mol. Biol. 293:865-881 (1999). If the on-rate exceeds 10⁶M⁻¹s⁻¹ by the surface plasmon resonance assay above, then the on-rate can be determined by using a fluorescent quenching technique that measures the increase or decrease in fluorescence emission intensity (excitation = 295 nm; emission = 340 nm, 16 nm band-pass) at 25°C of a 20 nM anti-antigen antibody (Fab form) in PBS, pH 7.2, in the presence of increasing concentrations of antigen as measured in a spectrometer, such as a stop-flow equipped spectrophometer (Aviv Instruments) or a 8000-series SLM-AMINCO™ spectrophotometer (ThermoSpectronic) with a stirred cuvette.

### 5. Antibody Fragments

In certain embodiments, a masked anti-CD3 antibody provided herein can be an antibody fragment. Antibody fragments include, but are not limited to, Fab, Fab', Fab'-SH, F(ab')₂, Fv, TaFv, scFv, diabody, bsDb, scDb, DART, BiTE, and V_{H}H fragments, and other fragments described below. For a review of certain antibody fragments, see Hudson et al. Nat. Med. 9:129-134 (2003). For a review of scFv fragments, see, e.g., Pluckthün, in The Pharmacology of Monoclonal Antibodies, vol. 113, Rosenburg and Moore eds., (Springer-Verlag, New York), pp. 269-315 (1994); see also WO 93/16185; and U.S. Patent Nos. 5,571,894 and 5,587,458. For discussion of Fab and F(ab')₂ fragments comprising salvage receptor binding epitope residues and having increased *in vivo* half-life, see U.S. Patent No. 5,869,046.

Diabodies are antibody fragments with two antigen-binding sites that may be bivalent or bispecific. See, for example, EP 404,097; WO 1993/01161; Hudson et al. Nat. Med. 9:129-134 (2003); and Hollinger et al. Proc. Natl. Acad. Sci. USA 90: 6444-6448 (1993). Triabodies and tetrabodies are also described in Hudson et al. Nat. Med. 9:129-134 (2003).

Single-domain antibodies are antibody fragments comprising all or a portion of the heavy chain variable domain or all or a portion of the light chain variable domain of an antibody. In certain embodiments, a single-domain antibody is a human single-domain antibody (Domantis, Inc., Waltham, MA; see, e.g., U.S. Patent No. 6,248,516 B1).

Antibody fragments can be made by various techniques, including but not limited to proteolytic digestion of an intact antibody as well as production by recombinant host cells (e.g. *E*. *coli* or phage), as described herein.

### 6. Chimeric and Humanized Antibodies

In certain embodiments, a masked anti-CD3 antibody provided herein is a chimeric antibody. Certain chimeric antibodies are described, e.g., in U.S. Patent No. 4,816,567; and Morrison et al. Proc. Natl. Acad. Sci. USA, 81:6851-6855 (1984)). In one example, a chimeric antibody comprises a non-human variable region (e.g., a variable region derived from a mouse, rat, hamster, rabbit, or non-human primate, such as a monkey) and a human constant region. In a further example, a chimeric antibody is a "class switched" antibody in which the class or subclass has been changed from that of the parent antibody. Chimeric antibodies include antigen-binding fragments thereof.

In certain embodiments, a chimeric antibody is a humanized antibody. Typically, a non-human antibody is humanized to reduce immunogenicity to humans, while retaining the specificity and affinity of the parental non-human antibody. Generally, a humanized antibody comprises one or more variable domains in which HVRs, e.g., CDRs, (or portions thereof) are derived from a non-human antibody, and FRs (or portions thereof) are derived from human antibody sequences. A humanized antibody optionally will also comprise at least a portion of a human constant region. In some embodiments, some FR residues in a humanized antibody are substituted with corresponding residues from a non-human antibody (e.g., the antibody from which the HVR residues are derived), e.g., to restore or improve antibody specificity or affinity.

Humanized antibodies and methods of making them are reviewed, e.g.. in Almagro and Fransson, Front. Biosci. 13:1619-1633 (2008), and are further described, e.g., in Riechmann et al., Nature 332:323-329 (1988); Queen et al., Proc. Nat'l Acad. Sci. USA 86:10029-10033 (1989); US Patent Nos. 5,821,337, 7,527,791, 6,982,321, and 7,087,409; Kashmiri et al., Methods 36:25-34 (2005) (describing specificity determining region (SDR) grafting); Padlan, Mol. Immunol. 28:489-498 (1991) (describing "resurfacing"); Dali'Acqua et al., Methods 36:43-60 (2005) (describing "FR shuffling"); and Osbourn et al., Methods 36:61-68 (2005) and Klimka et al., Br. J. Cancer, 83:252-260 (2000) (describing the "guided selection" approach to FR shuffling).

Human framework regions that may be used for humanization include but are not limited to: framework regions selected using the "best-fit" method (see, e.g., Sims et al. J. Immunol. 151:2296 (1993)); framework regions derived from the consensus sequence of human antibodies of a particular subgroup of light or heavy chain variable regions (see, e.g., Carter et al. Proc. Natl. Acad. Sci. USA. 89:4285 (1992); and Presta et al. J. Immunol., 151:2623 (1993)); human mature (somatically mutated) framework regions or human germline framework regions (see, e.g., Almagro and Fransson, Front. Biosci. 13:1619-1633 (2008)); and framework regions derived from screening FR libraries (see, e.g., Baca et al., J. Biol. Chem. 272:10678-10684 (1997) and Rosok et al., J. Biol. Chem. 271:22611-22618 (1996)).

### 7. Human Antibodies

In certain embodiments, a masked anti-CD3 antibody provided herein is a human antibody. Human antibodies can be produced using various techniques known in the art. Human antibodies are described generally in van Dijk and van de Winkel, Curr. Opin. Pharmacol. 5: 368-74 (2001) and Lonberg, Curr. Opin. Immunol. 20:450-459 (2008).

Human antibodies may be prepared by administering an immunogen to a transgenic animal that has been modified to produce intact human antibodies or intact antibodies with human variable regions in response to antigenic challenge. Such animals typically contain all or a portion of the human immunoglobulin loci, which replace the endogenous immunoglobulin loci, or which are present extrachromosomally or integrated randomly into the animal's chromosomes. In such transgenic mice, the endogenous immunoglobulin loci have generally been inactivated. For review of methods for obtaining human antibodies from transgenic animals, see Lonberg, Nat. Biotech. 23:1117-1125 (2005). See also, e.g., U.S. Patent Nos. 6,075,181 and 6,150,584 describing XENOMOUSE™ technology; U.S. Patent No. 5,770,429 describing HUMAB® technology; U.S. Patent No. 7,041,670 describing K-M MOUSE® technology, and U.S. Patent Application Publication No. US 2007/0061900, describing VELOCIMOUSE® technology). Human variable regions from intact antibodies generated by such animals may be further modified, e.g., by combining with a different human constant region.

Human antibodies can also be made by hybridoma-based methods. Human myeloma and mouse-human heteromyeloma cell lines for the production of human monoclonal antibodies have been described. (See, e.g., Kozbor J. Immunol., 133: 3001 (1984); Brodeur et al., Monoclonal Antibody Production Techniques and Applications, pp. 51-63 (Marcel Dekker, Inc., New York, 1987); and Boerner et al., J. Immunol., 147: 86 (1991).) *Human* antibodies generated via human B-cell hybridoma technology are also described in Li et al., Proc. Natl. Acad. Sci. USA. 103:3557-3562 (2006). Additional methods include those described, for example, in U.S. Patent No. 7,189,826 (describing production of monoclonal human IgM antibodies from hybridoma cell lines) and Ni. Xiandai Mianyixue, 26(4):265-268 (2006) (describing human-human hybridomas). Human hybridoma technology (Trioma technology) is also described in Vollmers and Brandlein, Histology and Histopathology, 20(3):927-937 (2005) and Vollmers and Brandlein, Methods and Findings in Experimental and Clinical Pharmacology, 27(3):185-91 (2005).

Human antibodies may also be generated by isolating Fv clone variable domain sequences selected from human-derived phage display libraries. Such variable domain sequences may then be combined with a desired human constant domain. Techniques for selecting human antibodies from antibody libraries are described below,

### 8. Library-Derived Antibodies

Masked anti-CD3 antibodies of the invention may be generated by screening combinatorial libraries for anti-CD3 antibodies with the desired activity or activities and subsequently joining the VH or VL domain of the identified anti-CD3 antibody with a polypeptide mask, as described above. For example, a variety of methods are known in the art for generating phage display libraries and screening such libraries for antibodies possessing the desired binding characteristics. Such methods are reviewed, e.g., in Hoogenboom et al. in Methods in Molecular Biology 178:1-37 (O'Brien et al., ed., Human Press, Totowa, NJ, 2001) and further described, e.g., in the McCafferty et al., Nature 348:552-554; Clackson et al., Nature 352: 624-628 (1991); Marks et al., J. Mol. Biol. 222: 581-597 (1992); Marks and Bradbury, in Methods in Molecular Biology 248:161-175 (Lo, ed., Human Press, Totowa, NJ, 2003); Sidhu et al., J. Mol. Biol. 338(2): 299-310 (2004); Lee et al., J. Mol. Biol 340(5): 1073-1093 (2004); Fellouse, Proc. Natl. Acad. Sci. USA 101(34): 12467-12472 (2004); and Lee et al., J. Immunol. Methods 284(1-2): 119-132(2004).

In certain phage display methods, repertoires of VH and VL genes are separately cloned by polymerase chain reaction (PCR) and recombined randomly in phage libraries, which can then be screened for antigen-binding phage as described in Winter et al., Ann. Rev. Immunol., 12: 433-455 (1994). Phage typically display antibody fragments, either as single-chain Fv (scFv) fragments or as Fab fragments. Libraries from immunized sources provide high-affinity antibodies to the immunogen without the requirement of constructing hybridomas. Alternatively, the naive repertoire can be cloned (e.g., from human) to provide a single source of antibodies to a wide range of non-self and also self antigens without any immunization as described by Griffiths et al., EMBO J, 12: 725-734 (1993). Finally, naive libraries can also be made synthetically by cloning unrearranged V-gene segments from stem cells, and using PCR primers containing random sequence to encode the highly variable CDR3 regions and to accomplish rearrangement *in vitro,* as described by Hoogenboom and Winter, J. Mol. Biol., 227: 381-388 (1992). Patent publications describing human antibody phage libraries include, for example: US Patent No. 5,750,373, and US Patent Publication Nos. 2005/0079574, 2005/0119455, 2005/0266000, 2007/0117126, 2007/0160598, 2007/0237764, 2007/0292936, and 2009/0002360.

Antibodies or antibody fragments isolated from human antibody libraries are considered human antibodies or human antibody fragments herein, and these antibodies may be joined with a polypeptide mask as described above to generate a masked anti-CD3 antibody of the invention.

### 9. Multispecific Antibodies

In any one of the above aspects, the masked anti-CD3 antibody provided herein is a multispecific antibody, for example, a bispecific antibody, such as a T cell-dependent bispecific (TDB) antibody. Multispecific antibodies are monoclonal antibodies that have binding specificities for at least two different sites. In certain embodiments, the masked anti-CD3 bispecific antibodies may be capable of binding to two different epitopes of CD3 (e.g., CD3ε or CD3γ) and have one or both anti-CD3 arms joined to a polypeptide mask. In other embodiments, one of the binding specificities is for CD3 (e.g., CD3ε or CD3γ) and the other is for any other antigen (e.g., a second biological molecule, e.g., a cell surface antigen, e.g., a tumor antigen). Accordingly, a masked anti-CD3 antibody may have binding specificities for CD3 and a second biological molecule, such as a second biological molecule (e.g., a tumor antigen) listed in Table 2 and described in U.S. Pub. No. 2010/0111856, and the anti-CD3 arm of the antibody can be joined to a polypeptide mask.

**Table 2. Tumor antigen targets of the bispecific anti-CD3 antibodies of the invention**

| | | |
|---|---|---|
| CD20 | Sema 5b | LY64 |
| FcRH5 | PSCA hlg | FcRH1 |
| HER2 | ETBR | IRTA2 |
| LYPD1 | MSG783 | TMEFF1 |
| Ly6G6D | STEAP2 | GDNF-Ra1 |
| PMEL1 7 | TrpM4 | TMEM46 |
| Ly6E | CRIPTO | LGR5 |
| CD19 | CD21 | LY6K |
| CD33 | FcRH2 | GPR19 |
| CD22 | NCA | GPR54 |
| CD79a | MDP | ASPHD1 |
| CD79b | IL20Ra | Tyrosinase |
| EDAR | Brevican | TMEM118 |
| GFRA1 | EphB2R | GPR172A |
| MRP4 | ASLG659 | GPC3 |
| RET | PSCA | CLL1 |
| STEAP1 | GEDA | B7-H4 |
| TENB2 | BAFF-R | RNF43 |
| E16 | CXCR5 | CD70 |
| 0772P | HLA-DOB | CXORF61 |
| MPF | P2X5 | SLC53D3 |
| Napi3b | CD72 | |

More particularly, a CD3 TDB antibody may have binding specificities for CD3 and a second biological molecule selected from the group consisting of CD20, FcRH5, HER2, LYPD1, LY6G6D, PMEL17, LY6E, CD19, CD33, CD22, CD79A, CD79B, EDAR, GFRA1, MRP4, RET, Steap1, and TenB2.

For example, in some instances, the antibody is a masked CD3/CD20 TDB (masked CD20 TDB) comprising a first binding domain comprising at least one, two, three, four, five, or six hypervariable regions (HVRs) selected from (a) HVR-H1 comprising the amino acid sequence of SEQ ID NO: 2; (b) HVR-H2 comprising the amino acid sequence of SEQ ID NO: 3; (c) HVR-H3 comprising the amino acid sequence of SEQ ID NO: 4; (d) HVR-L1 comprising the amino acid sequence of SEQ ID NO: 5; (e) HVR-L2 comprising the amino acid sequence of SEQ ID NO: 6; and (f) HVR-L3 comprising the amino acid sequence of SEQ ID NO: 7, and a second binding domain that binds to CD20, wherein either the VH or VL domain of the first binding domain (i.e., the anti-CD3 binding domain) is joined to a polypeptide mask, such as a polypeptide mask described above. The second binding domain that binds to CD20 may, for example, comprise at least one, two, three, four, five, or six hypervariable regions (HVRs) selected from (a) HVR-H1 comprising the amino acid sequence of SEQ ID NO: 20; (b) HVR-H2 comprising the amino acid sequence of SEQ ID NO: 21; (c) HVR-H3 comprising the amino acid sequence of SEQ ID NO: 22; (d) HVR-L1 comprising the amino acid sequence of SEQ ID NO: 23; (e) HVR-L2 comprising the amino acid sequence of SEQ ID NO: 24; and (f) HVR-L3 comprising the amino acid sequence of SEQ ID NO: 25. In some instances, the second binding domain that binds CD20 comprises at least one (e.g., 1, 2, 3, or 4) of heavy chain framework regions FR-H1, FR-H2, FR-H3, and FR-H4 comprising the sequences of SEQ ID NOs: 44-47, respectively, and/or at least one (e.g., 1, 2, 3, or 4) of the light chain framework regions FR-L1, FR-L2, FR-L3, and FR-L4 comprising the sequences of SEQ ID NOs: 48-51, respectively. In some instances, the second binding domain that binds to CD20 may, for example, comprise (a) a VH domain comprising an amino acid sequence having at least 90% sequence identity (e.g., at least 91%, 92%, 93%, 94%, 95%, 96%, 97%, 98%, or 99% sequence identity) to, or the sequence of, SEQ ID NO: 26; (b) a VL domain comprising an amino acid sequence having at least 90% sequence identity (e.g., at least 91%, 92%, 93%, 94%, 95%, 96%, 97%, 98%, or 99% sequence identity) to, or the sequence of, SEQ ID NO: 27; or (c) a VH domain as in (a) and a VL domain as in (b).

### 10. Antibody Variants

In certain embodiments, amino acid sequence variants of the masked anti-CD3 antibodies of the invention (e.g., masked anti-CD3 antibodies of the invention that bind to CD3 and a second biological molecule, e.g., a cell surface antigen, e.g., a tumor antigen, such as masked TDB antibodies of the invention or variants thereof) are contemplated. For example, it may be desirable to improve the binding affinity and/or other biological properties of the antibody. Amino acid sequence variants of an antibody may be prepared by introducing appropriate modifications into the nucleotide sequence encoding the antibody, or by peptide synthesis. Such modifications include, for example, deletions from, and/or insertions into and/or substitutions of residues within the amino acid sequences of the antibody. Any combination of deletion, insertion, and substitution can be made to arrive at the final construct, provided that the final construct possesses the desired characteristics, for example, antigen-binding.

### a. Substitution, Insertion, and Deletion Variants

In certain embodiments, masked anti-CD3 antibody variants having one or more amino acid substitutions are provided. Sites of interest for substitutional mutagenesis include the HVRs and FRs. Conservative substitutions are shown in Table 3 under the heading of "preferred substitutions" More substantial changes are provided in Table 3 under the heading of "exemplary substitutions," and as further described below in reference to amino acid side chain classes. Amino acid substitutions may be introduced into an antibody of interest and the products screened for a desired activity, for example, retained/improved antigen binding, decreased immunogenicity, or improved ADCC or CDC.

**Table 3. Exemplary and Preferred Amino Acid Substitutions**

| **Original Residue** | **Exemplary Substitutions** | **Preferred Substitutions** |
|---|---|---|
| Ala (A) | Val; Leu; Ile | Val |
| Arg (R) | Lys; Gln; Asn | L ys |
| Asn (N) | Gln; His; Asp, Lys; Arg | Gin |
| Asp (D) | Glu; Asn | Glu |
| Cys (C) | Ser; Ala | Ser |
| Gln (Q) | Asn; Glu; PCA | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; Norleucine | Leu |
| Leu (L) | Norleucine; Ile; Val; Met; Ala; Phe | lie |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val: Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Amino acids may be grouped according to common side-chain properties:
(1) hydrophobic: Norleucine, Met, Ala, Val, Leu, Ile;
(2) neutral hydrophilic: Cys, Ser, Thr, Asn, Gln;
(3) acidic: Asp, Glu;
(4) basic: His, Lys, Arg;
(5) residues that influence chain orientation: Gly, Pro;
(6) aromatic: Trp, Tyr, Phe.

Non-conservative substitutions will entail exchanging a member of one of these classes for another class.

One type of substitutional variant involves substituting one or more hypervariable region residues of a parent antibody (*e.g*. a humanized or human antibody). Generally, the resulting variant(s) selected for further study will have modifications (e.g., improvements) in certain biological properties (e.g., increased affinity, reduced immunogenicity) relative to the parent antibody and/or will have substantially retained certain biological properties of the parent antibody. An exemplary substitutional variant is an affinity matured antibody, which may be conveniently generated, e.g., using phage display-based affinity maturation techniques such as those described herein. Briefly, one or more HVR residues are mutated and the variant antibodies displayed on phage and screened for a particular biological activity (e.g. binding affinity).

Alterations (e.g., substitutions) may be made in HVRs, e.g., to improve antibody affinity. Such alterations may be made in HVR "hotspots," i.e., residues encoded by codons that undergo mutation at high frequency during the somatic maturation process (see, e.g., Chowdhury, Methods Mol. Biol. 207:179-196 (2008)), and/or residues that contact antigen, with the resulting variant VH or VL being tested for binding affinity. Affinity maturation by constructing and reselecting from secondary libraries has been described, e.g., in Hoogenboom et al. in Methods in Molecular Biology 178:1-37 (O'Brien et al., ed., Human Press, Totowa, NJ, (2001).) In some embodiments of affinity maturation, diversity is introduced into the variable genes chosen for maturation by any of a variety of methods (e.g., error-prone PCR, chain shuffling, or oligonucleotide-directed mutagenesis). A secondary library is then created. The library is then screened to identify any antibody variants with the desired affinity. Another method to introduce diversity involves HVR-directed approaches, in which several HVR residues (e.g., 4-6 residues at a time) are randomized. HVR residues involved in antigen binding may be specifically identified, e.g., using alanine scanning mutagenesis or modeling. CDR-H3 and CDR-L3 in particular are often targeted,

In certain embodiments, substitutions, insertions, or deletions may occur within one or more HVRs so long as such alterations do not substantially reduce the ability of the antibody to bind antigen. For example, conservative alterations (e.g., conservative substitutions as provided herein) that do not substantially reduce binding affinity may be made in HVRs. Such alterations may, for example, be outside of antigen contacting residues in the HVRs. In certain embodiments of the variant VH and VL sequences provided above, each HVR either is unaltered, or contains no more than one, two or three amino acid substitutions.

A useful method for identification of residues or regions of an antibody that may be targeted for mutagenesis is called "alanine scanning mutagenesis" as described by Cunningham and Wells (1989) Science, 244:1081-1085. In this method, a residue or group of target residues (e.g., charged residues such as arg, asp, his, lys, and glu) are identified and replaced by a neutral or negatively charged amino acid (e.g., alanine or polyalanine) to determine whether the interaction of the antibody with antigen is affected. Further substitutions may be introduced at the amino acid locations demonstrating functional sensitivity to the initial substitutions. Alternatively, or additionally, a crystal structure of an antigen-antibody complex to identify contact points between the antibody and antigen. Such contact residues and neighboring residues may be targeted or eliminated as candidates for substitution. Variants may be screened to determine whether they contain the desired properties. Similar strategies may also be used to identify residue(s) of the polypeptide mask of a masked anti-CD3 antibody that can tolerate or benefit from mutagenesis, such as one or more (e.g., 1, 2, 3, 4, 5, 6, 7, 8, 9, or 10 or more) directed substitution mutations.

Amino acid sequence insertions include amino- and/or carboxyl-terminal fusions ranging in length from one residue to polypeptides containing a hundred or more residues, as well as intra-sequence insertions of single or multiple amino acid residues. Examples of terminal insertions include an antibody with an N-terminal methionyl residue. Other insertional variants of the antibody molecule include the fusion to the N- or C-terminus of the antibody to an enzyme (e.g. for ADEPT) or a polypeptide which increases the serum half-life of the antibody.

### b. Glycosylation variants

In certain embodiments, masked anti-CD3 antibodies of the invention (e.g., masked anti-CD3 antibodies of the invention that bind to CD3 and a second biological molecule, e.g., a cell surface antigen, e.g., a tumor antigen, such as masked TDB antibodies of the invention or variants thereof) can be altered to increase or decrease the extent to which the antibody is glycosylated. Addition or deletion of glycosylation sites to masked anti-CD3 antibody of the invention may be conveniently accomplished by altering the amino acid sequence such that one or more glycosylation sites is created or removed.

Where the antibody comprises an Fc region, the carbohydrate attached thereto may be altered. Native antibodies produced by mammalian cells typically comprise a branched, biantennary oligosaccharide that is generally attached by an N-linkage to Asn297 of the CH2 domain of the Fc region. See, e.g., Wright et al. TIBTECH 15:26-32 (1997). The oligosaccharide may include various carbohydrates, e.g., mannose, N-acetyl glucosamine (GlcNAc), galactose, and sialic acid, as well as a fucose attached to a GlcNAc in the "stem" of the biantennary oligosaccharide structure. In some embodiments, modifications of the oligosaccharide in an antibody of the invention may be made in order to create antibody variants with certain improved properties.

In one embodiment, masked anti-CD3 antibody variants are provided having a carbohydrate structure that lacks fucose attached (directly or indirectly) to an Fc region. For example, the amount of fucose in such antibody may be from 1 % to 80%, from 1 % to 65%, from 5% to 65% or from 20% to 40%. The amount of fucose is determined by calculating the average amount of fucose within the sugar chain at Asn297, relative to the sum of all glycostructures attached to Asn 297 (e. g. complex, hybrid and high mannose structures) as measured by MALDI-TOF mass spectrometry, as described in WO 2008/077546, for example. Asn297 refers to the asparagine residue located at about position 297 in the Fc region (EU numbering of Fc region residues); however, Asn297 may also be located about ± 3 amino acids upstream or downstream of position 297, i.e., between positions 294 and 300, due to minor sequence variations in antibodies. Such fucosylation variants may have improved ADCC function. See, e.g., US Patent Publication Nos. US 2003/0157108 (Presta, L.); US 2004/0093621 (Kyowa Hakko Kogyo Co., Ltd). Examples of publications related to "defucosylated" or "fucose-deficient" antibody variants include: US 2003/0157108; WO 2000/61739; WO 2001/29246; US 2003/0115614; US 2002/0164328; US 2004/0093621; US 2004/0132140; US 2004/0110704; US 2004/0110282; US 2004/0109865; WO 2003/085119; WO 2003/084570; WO 2005/035586; WO 2005/035778; WO2005/053742; WO2002/031140; Okazaki et al. J. Mol. Biol. 336:1239-1249 (2004); Yamane-Ohnuki et al. Biotech. Bioeng. 87: 614 (2004). Examples of cell lines capable of producing defucosylated antibodies include Lec13 CHO cells deficient in protein fucosylation (Ripka et al. Arch. Biochem. Biophys. 249:533-545 (1986); US Pat Appl No US 2003/0157108 A1, Presta, L; and WO 2004/056312 A1, Adams et al.*,* especially at Example 11), and knockout cell lines, such as alpha-1,6-fucosyltransferase gene, *FUT8,* knockout CHO cells (see, e.g., Yamane-Ohnuki et al. Biotech. Bioeng. 87:614 (2004); Kanda, Y. et al., Biotechnol. Bioeng., 94(4):680-688 (2006); and WO2003/085107).

Masked anti-CD3 antibody variants are further provided with bisected oligosaccharides, for example, in which a biantennary oligosaccharide attached to the Fc region of the antibody is bisected by GlcNAc. Such antibody variants may have reduced fucosylation and/or improved ADCC function. Examples of such antibody variants are described, e.g., in WO 2003/011878 (Jean-Mairet et al.); US Patent No. 6,602,684 (Umana et al.); and US 2005/0123546 (Umana et al.)*.* Antibody variants with at least one galactose residue in the oligosaccharide attached to the Fc region are also provided. Such antibody variants may have improved CDC function. Such antibody variants are described, e.g., in WO 1997/30087 (Patel et al.); WO 1998/58964 (Raju, S.); and WO 1999/22764 (Raju, S.).

### c. Fc region variants

In certain embodiments, one or more amino acid modifications may be introduced into the Fc region of a masked anti-CD3 antibody of the invention (e.g., a masked anti-CD3 antibody of the invention that binds to CD3 and a second biological molecule, e.g.. a cell surface antigen, e.g., a tumor antigen, such as a masked TDB antibody of the invention or variant thereof), thereby generating an Fc region variant. The Fc region variant may comprise a human Fc region sequence (*e.g*., a human IgG1, IgG2, IgG3 or IgG4 Fc region) comprising an amino acid modification (*e.g*., a substitution) at one or more amino acid positions.

In certain embodiments, the invention contemplates a masked anti-CD3 antibody variant that possesses some, but not all, effector functions, which make it a desirable candidate for applications in which the half life of the antibody *in vivo* is important yet certain effector functions (such as complement and ADCC) are unnecessary or deleterious. *In vitro* and/or *in vivo* cytotoxicity assays can be conducted to confirm the reduction/depletion of CDC and/or ADCC activities. For example, Fc receptor (FcR) binding assays can be conducted to ensure that the antibody lacks FcyR binding (hence likely lacking ADCC activity), but retains FcRn binding ability. The primary cells for mediating ADCC, NK cells, express FcγRIII only, whereas monocytes express FcγRI, FcγRII and FcγRIII. FcR expression on hematopoietic cells is summarized in Table 3 on page 464 of Ravetch and Kinet, Annu. Rev. Immunol. 9:457-492 (1991). Non-limiting examples of *in vitro* assays to assess ADCC activity of a molecule of interest is described in U.S. Patent No. 5,500,362 (see, e.g. Hellstrom, I. et al. Proc. Nat'l Acad. Sci. USA 83:7059-7063 (1986)) and Hellstrom, I et al., Proc. Nat'l Acad. Sci. USA 82:1499-1502 (1985): 5,821,337 (see Bruggemann, M. et al., J. Exp. Med. 166:1351-1361 (1987)). Alternatively, non-radioactive assays methods may be employed (see, for example, ACTI™ non-radioactive cytotoxicity assay for flow cytometry (CellTechnology, Inc. Mountain View, CA; and CytoTox 96® non-radioactive cytotoxicity assay (Promega, Madison, WI). Useful effector cells for such assays include peripheral blood mononuclear cells (PBMC) and Natural Killer (NK) cells. Alternatively, or additionally. ADCC activity of the molecule of interest may be assessed *in vivo, e.g,* in a animal model such as that disclosed in Clynes et al. Proc. Nat'l Acad. Sci. USA 95:652-656 (1998). C1q binding assays may also be carried out to confirm that the antibody is unable to bind C1q and hence lacks CDC activity. See, e.g., C1q and C3c binding ELISA in WO 2006/029879 and WO 2005/100402. To assess complement activation, a CDC assay may be performed (see, for example, Gazzano-Santoro et al. J. Immunol. Methods 202:163 (1996); Cragg, M.S. et al. Blood. 101:1045-1052 (2003); and Cragg, M.S. and M.J. Glennie Blood. 103:2738-2743 (2004)). FcRn binding and *in vivo* clearance/half life determinations can also be performed using methods known in the art (see, e.g., Petkova, S.B. et al. Int'l. Immunol. 18(12):1759-1769 (2006)).

Antibodies with reduced effector function include those with substitution of one or more of Fc region residues 238, 265,269, 270, 297, 327 and 329 (U.S. Patent Nos. 6,737,056 and 8,219,149). Such Fc mutants include Fc mutants with substitutions at two or more of amino acid positions 265, 269, 270, 297 and 327, including the so-called "DANA" Fc mutant with substitution of residues 265 and 297 to alanine (US Patent No. 7,332,581 and 8,219,149).

Certain antibody variants with improved or diminished binding to FcRs are described. (See, e.g., U.S. Patent No. 6,737,056; WO 2004/056312, and Shields et al., J. Biol. Chem. 9(2): 6591-6604 (2001).)

In certain embodiments, an antibody variant comprises an Fc region with one or more amino acid substitutions which improve ADCC, e.g., substitutions at positions 298, 333, and/or 334 of the Fc region (EU numbering of residues).

In some embodiments, alterations are made in the Fc region that result in altered (*i.e*., either improved or diminished) C1q binding and/or Complement Dependent Cytotoxicity (CDC), e.g., as described in US Patent No. 6,194,551, WO 99/51642, and Idusogie et al. J. Immunol. 164: 4178-4184 (2000).

Antibodies with increased half lives and improved binding to the neonatal Fc receptor (FcRn), which is responsible for the transfer of maternal IgGs to the fetus (Guyer et al., J. Immunol. 117:587 (1976) and Kim et al., J. Immunol. 24:249 (1994)), are described in US2005/0014934A1 (Hinton et al.). Those antibodies comprise an Fc region with one or more substitutions therein which improve binding of the Fc region to FcRn. Such Fc variants include those with substitutions at one or more of Fc region residues: 238, 256, 265, 272, 286, 303, 305, 307, 311, 312, 317, 340, 356, 360, 362, 376, 378, 380, 382, 413, 424 or 434, e.g., substitution of Fc region residue 434 (US Patent No. 7,371,826).

See also Duncan & Winter, Nature 322:738-40 (1988); U.S. Patent No. 5,648,260; U.S. Patent No. 5,624,821; and WO 94/29351 concerning other examples of Fc region variants.

In some aspects the masked anti-CD3 antibody (e.g., masked TDB) comprises an Fc region comprising an N297G mutation. In some embodiments, the masked anti-CD3 antibody comprising the N297G mutation comprises an anti-CD3 arm comprising a first binding domain comprising the following six HVRs: (a) an HVR-H1 comprising the amino acid sequence of SEQ ID NO: 2; (b) an HVR-H2 comprising the amino acid sequence of SEQ ID NO: 3; (c) an HVR-H3 comprising the amino acid sequence of SEQ ID NO: 4; (d) an HVR-L1 comprising the amino acid sequence of SEQ ID NO: 5; (e) an HVR-L2 comprising the amino acid sequence of SEQ ID NO: 6; and (f) an HVR-L3 comprising the amino acid sequence of SEQ ID NO: 7: an anti-CD20 arm comprising a second binding domain comprising the following six HVRs: (a) an HVR-H1 comprising the amino acid sequence of SEQ ID NO: 20; (b) an HVR-H2 comprising the amino acid sequence of SEQ ID NO: 21: (c) an HVR-H3 comprising the amino acid sequence of SEQ ID NO: 22; (d) an HVR-L1 comprising the amino acid sequence of SEQ ID NO: 23; (e) an HVR-L2 comprising the amino acid sequence of SEQ ID NO: 24; and (f) an HVR-L3 comprising the amino acid sequence of SEQ ID NO: 25; and a polypeptide mask joined to the VH or VL domain of the binding domain of the anti-CD3 arm.

In some embodiments, the masked anti-CD3 antibody comprising the N297G mutation comprises an anti-CD3 arm comprising a first binding domain comprising (a) a VH domain comprising an amino acid sequence of SEQ ID NO: 8 and (b) a VL domain comprising an amino acid sequence of SEQ ID NO: 9; an anti-CD20 arm comprising a second binding domain comprising (a) a VH domain comprising an amino acid sequence of SEQ ID NO: 26 and (b) a VL domain comprising an amino acid sequence of SEQ ID NO: 27; and a polypeptide mask joined to the VH or VL domain of the binding domain of the anti-CD3 arm.

In some embodiments, the masked anti-CD3 antibody comprising the N297G mutation comprises one or more heavy chain constant domains, wherein the one or more heavy chain constant domains are selected from a first CH1 (CH1*₁*) domain, a first CH2 (CH2*₁*) domain, a first CH3 (CH3*₁*) domain, a second CH1 (CH1*₂*) domain, second CH2 (CH2*₂*) domain, and a second CH3 (CH3*₂*) domain. In some instances, at least one of the one or more heavy chain constant domains is paired with another heavy chain constant domain. In some instances, the CH3*₁* and CH3*₂* domains each comprise a protuberance or cavity, and wherein the protuberance or cavity in the CH3*₁* domain is positionable in the cavity or protuberance, respectively, in the CH3*₂* domain. In some instances, the CH3*₁* and CH3*₂* domains meet at an interface between said protuberance and cavity. In some instances, the CH2*₁*, and CH2*₂* domains each comprise a protuberance or cavity, and wherein the protuberance or cavity in the CH2*₁* domain is positionable in the cavity or protuberance, respectively, in the CH2*₂* domain. In other instances, the CH2*₁* and CH2*₂* domains meet at an interface between said protuberance and cavity. In some instances, the anti-CD3 antibody is an IgG1 antibody.

In other embodiments, the masked anti-CD3 antibody comprising the N297G mutation comprises an anti-CD3 arm comprising a first binding domain comprising (a) a VH domain comprising an amino acid sequence of SEQ ID NO: 8 and (b) a VL domain comprising an amino acid sequence of SEQ ID NO: 9; an anti-CD20 arm comprising a second binding domain comprising (a) a VH domain comprising an amino acid sequence of SEQ ID NO: 26 and (b) a VL domain comprising an amino acid sequence of SEQ ID NO: 27; and a polypeptide mask joined to the VH or VL domain of the binding domain of the anti-CD3 arm, wherein (a) the anti-CD3 arm comprises T366S, L368A, Y407V, and N297G substitution mutations and (b) the anti-CD20 arm comprises T366W and N297G substitution mutations.

### d. Cysteine engineered antibody variants

In certain embodiments, it may be desirable to create cysteine engineered antibodies, e.g., "thioMAbs." in which one or more residues of an antibody are substituted with cysteine residues. In particular embodiments, the substituted residues occur at accessible sites of the antibody. By substituting those residues with cysteine, reactive thiol groups are thereby positioned at accessible sites of the antibody and may be used to conjugate the antibody to other moieties, such as drug moieties or linker-drug moleties, to create an immunoconjugate, as described further herein. In certain embodiments, any one or more of the following residues may be substituted with cysteine: V205 (Kabat numbering) of the light chain; A118 (EU numbering) of the heavy chain; and S400 (EU numbering) of the heavy chain Fc region. Cysteine engineered antibodies may be generated as described, for example, in U.S. Patent No. 7,521,541.

### e. Antibody derivatives

In certain embodiments, a masked anti-CD3 antibody of the invention (e.g., a masked anti-CD3 antibody of the invention that binds to CD3 and a second biological molecule, e.g., a cell surface antigen, e.g., a tumor antigen, such as a masked TDB antibody of the invention or variant thereof) provided herein may be further modified to contain additional non-proteinaceous moieties that are known in the art and readily available. The moieties suitable for derivatization of the antibody include but are not limited to water soluble polymers. Non-limiting examples of water soluble polymers include, but are not limited to polyethylene glycol (PEG), copolymers of ethylene glycol/propylene glycol, carboxymethylcellulose, dextran, polyvinyl alcohol, polyvinyl pyrrolidone, poly-1, 3-dioxolane, poly-1,3,6-trioxane, ethylene/maleic anhydride copolymer, polyaminoacids (either homopolymers or random copolymers), and dextran or poly(n-vinyl pyrrolidone)polyethylene glycol, propropylene glycol homopolymers, prolypropylene oxide/ethylene oxide co-polymers, polyoxyethylated polyols (e.g., glycerol), polyvinyl alcohol, and mixtures thereof. Polyethylene glycol propionaldehyde may have advantages in manufacturing due to its stability in water. The polymer may be of any molecular weight, and may be branched or unbranched. The number of polymers attached to the antibody may vary, and if more than one polymer are attached, they can be the same or different molecules. In general, the number and/or type of polymers used for derivatization can be determined based on considerations including, but not limited to, the particular properties or functions of the antibody to be improved, whether the antibody derivative will be used in a therapy under defined conditions, etc.

In another embodiment, conjugates of a masked anti-CD3 antibody and non-proteinaceous moiety that may be selectively heated by exposure to radiation are provided. In one embodiment, the non-proteinaceous moiety is a carbon nanotube (Kam et al., Proc. Natl. Acad. Sci. USA 102: 11600-11605 (2005)). The radiation may be of any wavelength, and includes, but is not limited to, wavelengths that do not harm ordinary cells, but which heat the nonproteinaceous moiety to a temperature at which cells proximal to the antibody-nonproteinaceous moiety are killed.

### B. Recombinant Methods and Compositions

Masked anti-CD3 antibodies of the invention (e.g., masked anti-CD3 antibodies of the invention that bind to CD3 and a second biological molecule, e.g., a cell surface antigen, e.g., a tumor antigen, such as masked TDB antibodies of the invention or variants thereof) may be produced using recombinant methods and compositions, for example, as described in U.S. Patent No. 4,816,567. In one embodiment, isolated nucleic acid encoding a masked anti-CD3 antibody described herein is provided. Such nucleic acid may encode an amino acid sequence comprising the VL and/or an amino acid sequence comprising the VH of the antibody (e.g., the light and/or heavy chains of the antibody), wherein the polypeptide mask is encoded within the same open reading frame (ORF) as either the VL or VH domain. In a further embodiment, one or more vectors (e.g., expression vectors) comprising such nucleic acid are provided. In a further embodiment, a host cell comprising such nucleic acid is provided. In one such embodiment, a host cell comprises (e.g., has been transformed with): (1) a vector comprising a nucleic acid that encodes an amino acid sequence comprising the VL of the antibody and an amino acid sequence comprising the VH of the antibody, wherein the polypeptide mask is encoded within the same ORF as either the VL or VH domain, or (2) a first vector comprising a nucleic acid that encodes an amino acid sequence comprising the VL of the antibody and a second vector comprising a nucleic acid that encodes an amino acid sequence comprising the VH of the antibody, wherein the polypeptide mask is encoded within the same ORF as either the VL or VH domain, In one embodiment, the host cell is eukaryotic, e.g. a Chinese Hamster Ovary (CHO) cell or lymphoid cell (e.g., Y0, NS0, Sp20 cell). In one embodiment, a method of making a masked anti-CD3 antibody is provided, wherein the method comprises culturing a host cell comprising a nucleic acid encoding the antibody, as provided above, under conditions suitable for expression of the antibody, and optionally recovering the antibody from the host cell (or host cell culture medium).

For recombinant production of a masked anti-CD3 antibody, nucleic acid encoding the antibody, e.g., as described above, is isolated and inserted into one or more vectors for further cloning and/or expression in a host cell. Such nucleic acid may be readily isolated and sequenced using conventional procedures (e.g., by using oligonucleotide probes that are capable of binding specifically to genes encoding the heavy and light chains of the antibody).

Suitable host cells for cloning or expression of antibody-encoding vectors include prokaryotic or eukaryotic cells described herein. For example, masked anti-CD3 antibodies may be produced in bacteria, in particular when glycosylation and Fc effector function are not needed. For expression of antibody fragments and polypeptides in bacteria, see, e.g., U.S. Patent Nos. 5,648,237, 5,789,199, and 5,840,523. (See also Charlton, Methods in Molecular Biology, Vol. 248 (B.K.C. Lo, ed., Humana Press, Totowa, NJ, 2003), pp. 245-254, describing expression of antibody fragments in *E. coli*.) After expression, the masked anti-CD3 antibody may be isolated from the bacterial cell paste in a soluble fraction and can be further purified.

In addition to prokaryotes, eukaryotic microbes such as filamentous fungi or yeast are suitable cloning or expression hosts for antibody-encoding vectors, including fungi and yeast strains whose glycosylation pathways have been "humanized," resulting in the production of a masked anti-CD3 antibody with a partially or fully human glycosylation pattern. See Gemgross, Nat. Biotech. 22:1409-1414 (2004), and Li et al., Nat. Biotech. 24:210-215 (2006).

Suitable host cells for the expression of glycosylated masked anti-CD3 antibody are also derived from multicellular organisms (invertebrates and vertebrates). Examples of invertebrate cells include plant and insect cells. Numerous baculoviral strains have been identified which may be used in conjunction with insect cells, particularly for transfection of *Spodoptera frugiperda* cells.

Plant cell cultures can also be utilized as hosts. See, e.g., US Patent Nos. 5,959,177, 6,040,498, 6,420,548, 7,125,978, and 6,417,429 (describing PLANTIBODIES™ technology for producing antibodies in transgenic plants).

Vertebrate cells may also be used as hosts. For example, mammalian cell lines that are adapted to grow in suspension may be useful. Other examples of useful mammalian host cell lines are monkey kidney CV1 line transformed by SV40 (COS-7); human embryonic kidney line (293 or 293 cells as described, e.g., in Graham et al., J. Gen Virol. 36:59 (1977)); baby hamster kidney cells (BHK); mouse Sertoli cells (TM4 cells as described, e.g., in Mather, Biol. Reprod. 23:243-251 (1980)); monkey kidney cells (CV1); African green monkey kidney cells (VERO-76); human cervical carcinoma cells (HELA); canine kidney cells (MDCK; buffalo rat liver cells (BRL 3A); human lung cells (W138); human liver cells (Hep G2); mouse mammary tumor (MMT 060562); TRI cells, as described, e.g., in Mather et at, Annals N.Y. Acad. Sci. 383:44-68 (1982); MRC 5 cells; and FS4 cells. Other useful mammalian host cell lines include Chinese hamster ovary (CHO) cells, including DHFR- CHO cells (Urlaub et al., Proc. Natl. Acad. Sci. USA 77:4216 (1980)); and myeloma cell lines such as Y0, NS0 and Sp2/0. For a review of certain mammalian host cell lines suitable for antibody production, see, e.g., Yazaki and Wu, Methods in Molecular Biology, Vol. 248 (B.K.C. Lo, ed., Humana Press, Totowa, NJ). pp. 255-268 (2003).

### C. Assays

Masked anti-CD3 antibodies of the invention (e.g., masked anti-CD3 antibodies of the invention that bind to CD3 and a second biological molecule, e.g., a cell surface antigen, e.g., a tumor antigen, such as masked TDB antibodies of the invention or variants thereof) provided herein may be characterized for their physical/chemical properties and/or biological activities by various assays known in the art.

### 1. Binding assays and competition assays

In one aspect, a masked anti-CD3 antibody of the invention is tested for its binding activity, for example, by known methods such as ELISA, Western blot, etc.

In another aspect, competition assays may be used to identify an antibody that competes with an anti-CD3 antibody of the invention for binding to CD3. In certain embodiments, such a competing antibody binds to the same epitope (e.g., a linear or a conformational epitope) that is bound by an anti-CD3 antibody of the invention. Detailed exemplary methods for mapping an epitope to which an antibody binds are provided in Morris (1996) "Epitope Mapping Protocols," in Methods in Molecular Biology vol. 66 (Humana Press, Totowa, NJ).

In an exemplary competition assay, immobilized CD3 is incubated in a solution comprising a first labeled antibody that binds to CD3 and a second unlabeled antibody that is being tested for its ability to compete with the first antibody for binding to CD3. The second antibody may be present in a hybridoma supernatant. As a control, immobilized CD3 is incubated in a solution comprising the first labeled antibody but not the second unlabeled antibody. After incubation under conditions permissive for binding of the first antibody to CD3, excess unbound antibody is removed, and the amount of label associated with immobilized CD3 is measured. If the amount of label associated with immobilized CD3 is substantially reduced in the test sample relative to the control sample, then that indicates that the second antibody is competing with the first antibody for binding to CD3. See, e.g., Harlow and Lane (1988) Antibodies: A Laboratory Manual. Ch.14 (Cold Spring Harbor Laboratory, Cold Spring Harbor, NY).

### 2. Activity assays

In one aspect, assays are provided for identifying masked anti-CD3 antibodies having desired biological activity. Biological activity may include, for example, binding to CD3 (e.g., CD3 on the surface of a T cell), or a peptide fragment thereof, at a desired degree (i.e., ranging from no CD3 binding to binding CD3 with a low K_{d}, or a preferred intermediate affinity of the masked anti-CD3 antibody for CD3), either *in vivo, in vitro,* or ex *vivo.*

In the case of a masked TDB of the invention, desirable biological activity may also include, for example, effector cell activation (e.g., T cell (e.g., CD8+ and/or CD4+ T cell) activation) and/or effector cell population expansion (i.e., an increase in T cell count) in a cleaved state but not an uncleaved state, if the polypeptide mask is cleavable. If the polypeptide mask is not cleavable, desirable biological activity may include, for example, a reduction or inhibition of effector cell activation (e.g., T cell (e.g., CD8+ and/or CD4+ T cell) activation) and/or effector cell population expansion (i.e., an increase in T cell count) compared to such activity of the anti-CD3 antibody in the absence of the polypeptide mask. Other desirable activity may include, for example, a decrease or inhibition of target cell population reduction (i.e., a decrease in the population of cells expressing the second biological molecule on their cell surfaces) and/or target cell killing in the uncleaved state compared to such activity of the anti-CD3 antibody in the cleaved state, if the polypeptide mask is cleavable. If the polypeptide mask is not cleavable, desirable activity may include, for example, a decrease or inhibition of target cell population reduction (i.e., a decrease in the population of cells expressing the second biological molecule on their cell surfaces) and/or target cell killing compared to such activity of the anti-CD3 antibody in the absence of the polypeptide mask. In other instances, target cell population reduction and/or target cell killing by the masked anti-CD3 antibody occurs in the absence of effector cell activation (e.g., T cell (e.g., CD8+ and/or CD4+ T cell) activation) and/or effector cell population expansion (i.e., an increase in T cell count).

In certain embodiments, a masked anti-CD3 antibody of the invention is tested for such biological activity, as described in detail in the Examples herein below.

### D. Immunoconjugates and Labeled Antibodies

The invention also provides immunoconjugates comprising a masked anti-CD3 antibody herein conjugated to one or more cytotoxic agents, such as chemotherapeutic agents or drugs, growth inhibitory agents, toxins (e.g., protein toxins, enzymatically active toxins of bacterial, fungal, plant, or animal origin, or fragments thereof), or radioactive isotopes.

In one embodiment, an immunoconjugate is an antibody-drug conjugate (ADC) in which a masked anti-CD3 antibody is conjugated to one or more drugs, including but not limited to a maytansinoid (see U.S. Patent Nos. 5,208,020, 5,416,064 and European Patent EP 0 425 235 B1); an auristatin such as monomethylauristatin drug moieties DE and DF (MMAE and MMAF) (see U.S. Patent Nos. 5,635,483 and 5,780,588, and 7,498,298); a dolastatin; a calicheamicin or derivative thereof (see U.S. Patent Nos. 5,712,374, 5,714,586, 5,739,116, 5,767,285, 5,770,701, 5,770,710, 5,773,001, and 5,877.296; Hinman et al., Cancer Res. 53:3336-3342 (1993); and Lode et al., Cancer Res. 58:2925-2928 (1998)); an anthracycline such as daunomycin or doxorubicin (see Kratz et al., Current Med. Chem. 13:477-523 (2006); Jeffrey et al., Bioorganic & Med. Chem. Letters 16:358-362 (2006); Torgov et al., Bioconj. Chem. 16:717-721 (2005); Nagy et al., Proc. Natl. Acad. Sci. USA 97:829-834 (2000); Dubowchik et al., Bioorg. & Med. Chem. Letters 12:1529-1532 (2002); King et al., J. Med. Chem. 45:4336-4343 (2002); and U.S. Patent No, 6,630,579); methotrexate; vindesine; a taxane such as docetaxel, paclitaxel, larotaxel, tesetaxel, and ortataxel; a trichothecene; and CC1 065.

In another embodiment, an immunoconjugate comprises a masked anti-CD3 antibody as described herein conjugated to an enzymatically active toxin or fragment thereof, including but not limited to diphtheria A chain, nonbinding active fragments of diphtheria toxin, exotoxin A chain (from Pseudomonas aeruginosa), ricin A chain, abrin A chain, modeccin A chain, alpha-sarcin. Aleurites fordii proteins, dianthin proteins, Phytolaca americana proteins (PAPI, PAPII, and PAP-S), momordica charantia inhibitor, curcin, crotin, sapaonaria officinalis inhibitor, gelonin, mitogellin, restrictocin, phenomycin, enomycin, and the tricothecenes.

In another embodiment, an immunoconjugate comprises a masked anti-CD3 antibody as described herein conjugated to a radioactive atom to form a radioconjugate. A variety of radioactive isotopes are available for the production of radioconjugates. Examples include At²¹¹, I¹³¹, I¹²⁵, Y⁹⁰, Re¹⁸⁶, Re¹⁸⁸, Sm¹⁵³, Bi²¹², P³², Pb²¹² and radioactive isotopes of Lu. When the radioconjugate is used for detection, it may comprise a radioactive atom for scintigraphic studies, for example tc99m or i123, or a spin label for nuclear magnetic resonance (NMR) imaging (also known as magnetic resonance imaging, mri), such as iodine-123 again, iodine-131, indium-111, fluorine-19, carbon-13, nitrogen-15, oxygen-17, gadolinium, manganese or iron.

Conjugates of a masked anti-CD3 antibody of the invention and a cytotoxic agent may be made using a variety of bifunctional protein coupling agents such as N-succinimidyl-3-(2-pyridyldithio) propionate (SPDP), succinimidyl-4-(N-maleimidomethyl) cyclohexane-1-carboxylate (SMCC), iminothiolane (IT), bifunctional derivatives of imidoesters (such as dimethyl adipimidate HCl), active esters (such as disuccinimidyl suberate), aldehydes (such as glutaraldehyde), bis-azido compounds (such as bis (p-azidobenzoyl) hexanediamine), bis-diazonium derivatives (such as bis-(p-diazoniumbenzoyl)-ethylenediamine), diisocyanates (such as toluene 2,6-diisocyanate), and bis-active fluorine compounds (such as 1,5-difluoro-2,4-dinitrobenzene), For example, a ricin immunotoxin can be prepared as described in Vitetta et al., Science 238:1098 (1987). Carbon-14-labeled 1-isothiocyanatobenzyl-3-methyldiethylene triaminepentaacetic acid (MX-DTPA) is an exemplary chelating agent for conjugation of radionucleotide to the antibody. See, for example, WO94/11026. The coupling agent may be reversible to facilitate release of a cytotoxic drug in the cell. See, for example, Chari et al. Cancer Res. 52:127-131, 1992 and U.S. Patent No. 5,208,020.

The immunuoconjugates or ADCs herein expressly contemplate, but are not limited to such conjugates prepared with cross-linker reagents including, but not limited to, BMPS, EMCS, GMBS, HBVS, LC-SMCC, MBS, MPBH, SBAP, SIA, SIAB, SMCC, SMPB. SMPH, sulfo-EMCS, sulfo-GMBS, sulfo-KMUS, sulfo-MBS, sulfo-SIAS, sulfo-SMCC, and sulfo-SMPB, and SVSB (succinimidyl-(4-vinylsulfone)benzoate) which are commercially available (e.g., from Pierce Biotechnology, Inc., Rockford, IL., U.S.A).

In certain embodiments, labeled masked anti-CD3 antibodies are provided. Labels include, but are not limited to, labels or moieties that are detected directly (such as fluorescent, chromophoric, electron-dense, chemiluminescent, and radioactive labels), as well as moieties, such as enzymes or ligands, that are detected indirectly, e.g., through an enzymatic reaction or molecular interaction. Exemplary labels include, but are not limited to, the radioisotopes ³²P, ¹⁴C, ¹²⁵I, ³H, and ¹³¹I, fluorophores such as rare earth chelates or fluorescein and its derivatives, rhodamine and its derivatives, dansyl, umbelliferone, luceriferases, e.g., firefly luciferase and bacterial luciferase (U.S. Patent No. 4,737,456), luciferin, 2,3-dihydrophthalazinediones, horseradish peroxidase (HRP), alkaline phosphatase, β-galactosidase, glucoamylase, lysozyme, saccharide oxidases, e.g., glucose oxidase, galactose oxidase, and glucose-β-phosphate dehydrogenase, heterocyclic oxidases such as uricase and xanthine oxidase, coupled with an enzyme that employs hydrogen peroxide to oxidize a dye precursor such as HRP, lactoperoxidase, or microperoxidase, biotin/avidin, spin labels, bacteriophage labels, stable free radicals, and the like.

### E. Pharmaceutical Formulations

Pharmaceutical formulations of a masked anti-CD3 antibody of the invention (e.g., masked anti-CD3 antibody of the invention that binds to CD3 and a second biological molecule, e.g., a cell surface antigen, e.g., a tumor antigen, such as a masked TDB antibody of the invention or variant thereof) are prepared by mixing such antibody having the desired degree of purity with one or more optional pharmaceutically acceptable carriers (Remington's Pharmaceutical Sciences 16th edition, Osol, A. Ed. (1980)), in the form of lyophilized formulations or aqueous solutions. Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as polyethylene glycol (PEG). Exemplary pharmaceutically acceptable carriers herein further include insterstitial drug dispersion agents such as soluble neutral-active hyaluronidase glycoproteins (sHASEGP), for example, human soluble PH-20 hyaluronidase glycoproteins, such as rHuPH20 (HYLENEX®, Baxter International, Inc.). Certain exemplary sHASEGPs and methods of use, including rHuPH20, are described in US Patent Publication Nos. 2005/0260186 and 2006/0104968. In one aspect, a sHASEGP is combined with one or more additional glycosaminoglycanases such as chondroitinases.

Exemplary lyophilized antibody formulations are described in US Patent No. 6,267,958. Aqueous antibody formulations include those described in US Patent No. 6,171,586 and WO2006/044908, the latter formulations including a histidine-acetate buffer.

The formulation herein may also contain more than one active ingredients as necessary for the particular indication being treated, preferably those with complementary activities that do not adversely affect each other. For example, it may be desirable to further provide an additional therapeutic agent (e.g., a chemotherapeutic agent, a cytotoxic agent, a growth inhibitory agent, and/or an anti-hormonal agent, such as those recited herein above). Such active ingredients are suitably present in combination in amounts that are effective for the purpose intended.

Active ingredients may be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980),

Sustained-release preparations may be prepared. Suitable examples of sustained-release preparations include semipermeable matrices of solid hydrophobic polymers containing the antibody, which matrices are in the form of shaped articles, for example, films, or microcapsules.

The formulations to be used for *in vivo* administration are generally sterile. Sterility may be readily accomplished, e.g., by filtration through sterile filtration membranes.

### F. Therapeutic Methods and Compositions

Any of the masked anti-CD3 antibodies of the invention (e.g., masked anti-CD3 antibodies of the invention that bind to CD3 and a second biological molecule, e.g., a cell surface antigen, e.g., a tumor antigen, such as masked TDB antibodies of the invention or variants thereof) may be used in therapeutic methods.

In one aspect, a masked anti-CD3 antibody for use as a medicament is provided. In further aspects, a masked anti-CD3 antibody for use in treating or delaying progression of a cell proliferative disorder (e.g., cancer) or an autoimmune disorder (e.g., arthritis) is provided. In certain embodiments, a masked anti-CD3 antibody for use in a method of treatment is provided. In certain embodiments, the invention provides a masked anti-CD3 antibody for use in a method of treating an individual having a cell proliferative disorder or an autoimmune disorder comprising administering to the individual an effective amount of the masked anti-CD3 antibody. In one such embodiment, the method further comprises administering to the individual an effective amount of at least one additional therapeutic agent, for example, as described below. In further embodiments, the invention provides a masked anti-CD3 antibody for use in enhancing immune function in an individual having a cell proliferative disorder or an autoimmune disorder. In certain embodiments, the invention provides a masked anti-CD3 antibody for use in a method of enhancing immune function in an individual having a cell proliferative disorder or an autoimmune disorder comprising administering to the individual an effective of the masked anti-CD3 antibody to activate effector cells (e.g., T cells, e.g., CD8+ and/or CD4+ T cells), expand (increase) an effector cell population, reduce a target cell (e.g., a cell expressing a second biological molecule recognized by a masked TDB of the invention) population, and/or kill a target cell (e.g., target tumor cell). An "individual" according to any of the above embodiments may be a human.

In a further aspect, the invention provides for the use of a masked anti-CD3 antibody in the manufacture or preparation of a medicament. In one embodiment, the medicament is for treatment of a cell proliferative disorder (e.g., cancer) or an autoimmune disorder (e.g., arthritis). In a further embodiment, the medicament is for use in a method of treating a cell proliferative disorder or an autoimmune disorder comprising administering to an individual having a cell proliferative disorder or an autoimmune disorder an effective amount of the medicament. In one such embodiment, the method further comprises administering to the individual an effective amount of at least one additional therapeutic agent, for example, as described below. In a further embodiment, the medicament is for activating effector cells (e.g., T cells, e.g., CD8+ and/or CD4+ T cells), expanding (increasing) an effector cell population, reducing a target cell (e.g., a cell expressing a second biological molecule recognized by a masked TDB of the invention) population, and/or killing target cells (e.g., target tumor cells) in the individual. In a further embodiment, the medicament is for use in a method of enhancing immune function in an individual having a cell proliferative disorder or an autoimmune disorder comprising administering to the individual an amount effective of the medicament to activate effector cells (e.g., T cells, e.g., CD8+ and/or CD4+ T cells), expand (increase) an effector cell population, reduce a target cell (e.g., a cell expressing a second biological molecule recognized by a masked TDB of the invention) population, and/or kill a target cell (e.g., target tumor cell). An "individual" according to any of the above embodiments may be a human.

In a further aspect, the invention provides a method for treating a cell proliferative disorder (e.g., cancer) or an autoimmune disorder (e.g., arthritis). In one embodiment, the method comprises administering to an individual having such a cell proliferative disorder or an autoimmune disorder an effective amount of a masked anti-CD3 antibody, In one such embodiment, the method further comprises administering to the individual an effective amount of at least one additional therapeutic agent, for example, as described below. An "individual" according to any of the above embodiments may be a human.

In a further aspect, the invention provides a method for enhancing immune function in an individual having a cell proliferative disorder or an autoimmune disorder in an individual having a cell proliferative disorder or an autoimmune disorder. In one embodiment, the method comprises administering to the individual an effective amount of a masked anti-CD3 antibody to activate effector cells (e.g., T cells, e.g., CD8+ and/or CD4+ T cells), expand (increase) an effector cell population, reduce a target cell (e.g., a cell expressing a second biological molecule recognized by a masked TDB of the invention) population, and/or kill a target cell (e.g., target tumor cell). In one embodiment, an "individual" is a human.

In a further aspect, the invention provides pharmaceutical formulations comprising any of the masked anti-CD3 antibodies provided herein, for example, for use in any of the above therapeutic methods. In one embodiment, a pharmaceutical formulation comprises any of the masked anti-CD3 antibodies provided herein and a pharmaceutically acceptable carrier. In another embodiment, a pharmaceutical formulation comprises any of the masked anti-CD3 antibodies provided herein and at least one additional therapeutic agent, for example, as described herein.

The masked anti-CD3 antibodies of the invention can be used either alone or in combination with other agents in a therapy. For instance, an antibody of the invention may be co-administered with at least one additional therapeutic agent. In certain embodiments, an additional therapeutic agent is a chemotherapeutic agent, growth inhibitory agent, cytotoxic agent, agent used in radiation therapy, anti-angiogenesis agent, apoptotic agent, anti-tubulin agent, or other agent, such as a epidermal growth factor receptor (EGFR) antagonist (*e.g.*, a tyrosine kinase inhibitor), HER1/EGFR inhibitor *(e.g*., erlotinib (Tarceva™), platelet derived growth factor inhibitor (*e.g.*, Gleevec™ (Imatinib Mesylate)), a COX-2 inhibitor (*e.g*,, celecoxib), interferon, cytokine, antibody other than the anti-CD3 antibody of the invention, such as an antibody that bind to one or more of the following targets ErbB2, ErbB3, ErbB4, PDGFR-beta, BlyS, APRIL, BCMA VEGF, or VEGF receptor(s), TRAIL/Apo2, or another bioactive or organic chemical agent.

The masked anti-CD3 antibodies of the invention can also be used in combination with a PD-1 axis binding antagonist, alone or in conjunction with an additional therapeutic agent. In some instances, the PD-1 axis binding antagonist can be a PD-1 binding antagonist, a PD-L1 binding antagonist, or a PD-L2 binding antagonist. The PD-1 binding antagonist can be, for example, MDX-1106 (nivolumab), MK-3475 (lambrolizumab), CT-011 (pidilizumab), or AMP-224. The PD-L1 binding antagonist can be, for example, YW243.55.S70, MPDL3280A, MDX-1105, or MED14736. The PD-L2 binding antagonist can be, for example, an antibody or an immunoadhesin.

In other instances, the masked anti-CD3 antibodies of the invention may be used in combination with a glucocorticoid, such as dexamethasone, In any of the above combination therapies, the masked anti-CD3 antibodies may also be used in combination with rituximab.

Such combination therapies noted above encompass combined administration (where two or more therapeutic agents are included in the same or separate formulations), and separate administration, in which case, administration of the antibody of the invention can occur prior to, simultaneously, and/or following, administration of the additional therapeutic agent or agents. In one embodiment, administration of the masked anti-CD3 antibody and administration of an additional therapeutic agent occur within about one month, or within about one, two or three weeks, or within about one, two, three, four, five, or six days, of each other. Masked anti-CD3 antibodies of the invention (e.g., masked anti-CD3 antibodies of the invention that bind to CD3 and a second biological molecule, e.g., a cell surface antigen, e.g., a tumor antigen, such as a masked TDB antibody of the invention or variant thereof) can also be used in combination with radiation therapy.

An antibody of the invention (and/or any additional therapeutic agent) can be administered by any suitable means, including parenteral, intrapulmonary, and intranasal, and, if desired for local treatment, intralesional administration. Parenteral infusions include intramuscular, intravenous, intraarterial, intraperitoneal, or subcutaneous administration. Dosing can be by any suitable route, for example, by injections, such as intravenous or subcutaneous injections, depending in part on whether the administration is brief or chronic. Various dosing schedules including but not limited to single or multiple administrations over various time-points, bolus administration, and pulse infusion are contemplated herein.

Masked antibodies of the invention would be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The antibody need not be, but is optionally formulated with one or more agents currently used to prevent or treat the disorder in question. The effective amount of such other agents depends on the amount of antibody present in the formulation, the type of disorder or treatment, and other factors discussed above. These are generally used in the same dosages and with administration routes as described herein, or about from 1 to 99% of the dosages described herein, or in any dosage and by any route that is empirically/clinically determined to be appropriate.

For the prevention or treatment of disease, the appropriate dosage of a masked antibody of the invention (when used alone or in combination with one or more other additional therapeutic agents) will depend on the type of disease to be treated, the type of antibody, the severity and course of the disease, whether the antibody is administered for preventive or therapeutic purposes, previous therapy, the patient's clinical history and response to the antibody, and the discretion of the attending physician. The antibody is suitably administered to the patient at one time or over a series of treatments.

As a general proposition, the therapeutically effective amount of the masked anti-CD3 antibody administered to human will be in the range of about 0.01 to about 100 mg/kg of patient body weight whether by one or more administrations. In some embodiments, the masked antibody used is about 0.01 to about 45 mg/kg, about 0.01 to about 40 mg/kg, about 0.01 to about 35 mg/kg, about 0.01 to about 30 mg/kg, about 0.01 to about 29 mg/kg, about 0.01 to about 28 mg/kg, about 0.01 to about 27 mg/kg, about 0.01 to about 26 mg/kg, about 0.01 to about 25 mg/kg, about 0.01 to about 24 mg/kg, about 0.01 to about 23 mg/kg, about 0.01 to about 22 mg/kg, about 0.01 to about 21 mg/kg, about 0.01 to about 20 mg/kg, about 0.01 to about 19 mg/kg, about 0.01 to about 18 mg/kg, about 0.01 to about 17 mg/kg, about 0.01 to about 16 mg/kg, about 0.01 to about 15 mg/kg, about 0.01 to about 14 mg/kg, about 0.01 to about 13 mg/kg, about 0.01 to about 12 mg/kg, about 0.01 to about 11 mg/kg, about 0.01 to about 10 mg/kg, about 0.01 to about 9 mg/kg, about 0.01 to about 8 mg/kg, about 0.01 to about 7 mg/kg, about 0.01 to about 6 mg/kg, about 0.01 to about 5 mg/kg, about 0.01 to about 4 mg/kg, about 0.01 to about 3 mg/kg, about 0.01 to about 2 mg/kg, or about 0.01 to about 1 mg/kg administered daily, for example. In one embodiment, a masked anti-CD3 antibody described herein is administered to a human at a dose of about 100 mg, about 200 mg, about 300 mg, about 400 mg, about 500 mg, about 600 mg, about 700 mg, about 800 mg, about 900 mg, about 1000 mg, about 1100 mg, about 1200 mg, about 1300 mg or about 1400 mg on day 1 of 21-day cycles. The dose may be administered as a single dose or as multiple doses (e.g., 2 or 3 doses), such as infusions. For repeated administrations over several days or longer, depending on the condition, the treatment would generally be sustained until a desired suppression of disease symptoms occurs. One exemplary dosage of the masked anti-CD3 antibody would be in the range from about 0.05 mg/kg to about 10 mg/kg. Thus, one or more doses of about 0.5 mg/kg, 2.0 mg/kg, 4.0 mg/kg, or 10 mg/kg (or any combination thereof) may be administered to the patient. Such doses may be administered intermittently, for example, every week or every three weeks (e.g., such that the patient receives from about two to about twenty, or, for example, about six doses of the masked anti-CD3 antibody). An initial higher loading dose, followed by one or more lower doses may be administered. The progress of this therapy is easily monitored by conventional techniques and assays.

In some embodiments, the methods may further comprise an additional therapy. The additional therapy may be radiation therapy, surgery, chemotherapy, gene therapy, DNA therapy, viral therapy, RNA therapy, immunotherapy, bone marrow transplantation, nanotherapy, monoclonal antibody therapy, or a combination of the foregoing. The additional therapy may be in the form of adjuvant or neoadjuvant therapy. In some embodiments, the additional therapy is the administration of small molecule enzymatic inhibitor or anti-metastatic agent. In some embodiments, the additional therapy is the administration of side-effect limiting agents (e.g., agents intended to lessen the occurrence and/or severity of side effects of treatment, such as anti-nausea agents, etc.). In some embodiments, the additional therapy is radiation therapy. In some embodiments, the additional therapy is surgery. In some embodiments, the additional therapy is a combination of radiation therapy and surgery. In some embodiments, the additional therapy is gamma irradiation. In some embodiments, the additional therapy may be a separate administration of one or more of the therapeutic agents described above.

### G. Articles of Manufacture

In another aspect of the invention, an article of manufacture containing materials useful for the treatment, prevention and/or diagnosis of the disorders described above is provided. The article of manufacture comprises a container and a label or package insert on or associated with the container. Suitable containers include, for example, bottles, vials, syringes. IV solution bags, etc, The containers may be formed from a variety of materials such as glass or plastic. The container holds a composition which is by itself or combined with another composition effective for treating, preventing and/or diagnosing the condition and may have a sterile access port (for example the container may be an intravenous solution bag or a vial having a stopper pierceable by a hypodermic injection needle). At least one active agent in the composition is a masked anti-CD3 antibody of the invention. The label or package insert indicates that the composition is used for treating the condition of choice. Moreover, the article of manufacture may comprise (a) a first container with a composition contained therein, wherein the composition comprises a masked anti-CD3 antibody of the invention; and (b) a second container with a composition contained therein, wherein the composition comprises a further cytotoxic or otherwise therapeutic agent. The article of manufacture in this embodiment of the invention may further comprise a package insert indicating that the compositions can be used to treat a particular condition. Alternatively, or additionally, the article of manufacture may further comprise a second (or third) container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

### III. EXAMPLES

The following are examples of methods and compositions of the invention. It is understood that various other embodiments may be practiced, given the general description provided above.

### Example 1. Materials and Methods

### Phage binding ELISA

All PEG-precipitated phage preparations were treated with Q-cyclase prior to the assay in order to cyclize the N-terminal glutamine residue to generate a pyroglutamate (PCA) residue. Phage preps were diluted to a concentration that was previously tested to give an ELISA binding signal of about 1.0 OD at 450nm for unmasked anti-CD3 antibody phage.

For thrombin treated samples, a Thrombin ClenCleave Kit (Sigma-Aldrich, #RECOMT) was used according to the kit instructions. Thrombin CleanCleave is a 50% (v/v) suspension of thrombin-agarose. Resin slurry was washed three times with cleavage buffer (50mM Tris-HCL, pH8.0, 10mM CaCl₂), removing supernatant by 500 x g centrifugation after each wash. The pelleted resin was re-suspended in 1x cleavage buffer, diluting the resin 1:5.

Masked anti-CD3 phage variants were purified from 10.0 ml overnight cultures. After 2 rounds of PEG precipitation, pelleted phage was re-suspended in 1x cleavage buffer to give an OD reading of 4.0 at 268nm. For the cleavage reaction, 200 µl of thrombin agarose slurry was added to 200 µl of purified phage in a microcentrifuge tube. The mixture was incubated at 37°C overnight with gentle agitation. Beads were removed by centrifugation and remaining supernatant containing phage was added to the assay plate.

CD3ε¹⁻²⁷Fc (CD3ε-Fc; see, e.g., U.S.S.N. 61/949,950) was coated overnight in PBS on Nunc Maxisorp plates at 4°C. After blocking 1 h with 2% milk in PBS Tween (PBS, containing 0.05% Tween 20), the PEG purified masked anti-CD3 antibody variants displayed on phage were allowed to bind either before or after pre-treatment with thrombin. After 1 hour, microtiter plates were washed three times and incubated with HRP conjugated anti-M13 antibody. Binding signals were normalized for display as assessed by binding to a gD tag displayed on the C-terminus of the light chain.

### Human T cell activation assay

Human blood was collected in heparinized syringes and peripheral blood mononuclear cells (PBMCs) were isolated using Leucosep (Greiner Bio-One, Cat. No. 227290P) and Ficoll Paque Plus (GE Healthcare Biosciences, Cat. No. 95038-168), as recommended by the manufacture. Cells were washed in RPMI medium containing 10% FBS, supplemented with GlutaMax (Gibco, Cat No. 35050-061), penicillin, and streptomycin (Gibco, Cat. No. 15140-122). and approximately 200,000 suspended cells were added to a 96-well U-bottom plate. CD3/CD20 TDBs (CD20 TDBs) having one anti-CD20 arm and one anti-CD3 arm were produced as full-length antibodies in the knob-into-hole format as human IgG1 as previously described (see., e.g., Atwell et al. J. Mol. Biol. 270: 26-35, 1997 and U.S.S.N. 61/949,950). The CD20 TDBs were added at between 10 and 0.01 µg/ml to the wells. After culturing for approximately 20 hours, cells were washed with FACS buffer (0.5% BSA, 0.05% sodium azide in PBS). Cells were then stained with anti-CD69-FITC (BD Biosciences, Cat. No. 555530), anti-CD25..PE (BD Biosciences, Cat. No. 555432), and anti-CDS-APC (BD Biosciences, Cat. No. 555369) in FACS buffer, washed with FACS buffer, and suspend in 100 µl of FACS buffer containing 1 µg/ml propidium iodide (PI). Data were collected on a FACSCalibur Flow Cytometer (BD Biosciences). The extent of T cell activation was determined comparing the percentage of CD69⁺ and CD25⁺ population in CD8+ T cells.

### Endogenous B-cell killing

Human PBMCs were isolated from whole blood of healthy donors by Ficoll separation. CD4* T cells and CD8+ T cells were separated with Miltenyi kits according to manufacturer's instructions. Cells were cultured in RPMI1640 supplemented with 10%FBS (Sigma-Aldrich) at 37°C in a humidified standard cell culture incubator. 200,000 PBMCs were incubated for 48 hours with various concentrations of CD20 TDB antibodies (described above). At the end of each assay, live B cells were gated out as PI-CD19⁺ or PI-CD20⁺ B cells by FACS, and absolute cell count was obtained with FITC beads added to reaction mixture as an internal counting control. The percentage of cell killing was calculated based on non-TDB treated controls. Activated T cells were detected by CD69 and CD25 surface expression.

### Example 2. Effects of Polypeptide Mask Length on Inhibition

The anti-CD3 antibodies provided herein and in U.S. Pub. No. 2015-0166661, which is incorporated by reference herein in its entirety, bind to the N-terminus of CD3ε. As described herein, tethering portions of the natural CD3ε N-terminal sequence to an anti-CD3 antibody creates a "polypeptide mask" that is capable of inhibiting the anti-CD3 antibody from binding to CD3ε on T cells.

As shown in Figures 1 and 2, different levels of inhibition can be achieved by varying the overall length of the polypeptide mask by shortening or lengthening the length of the masking moiety (MM), which is the component of the polypeptide mask that includes the natural CD3ε N-terminal sequence. The effects of varying MM length in the context of two different anti-CD3 antibodies were tested. To this end, polypeptide masks having MMs ranging from the 1 to 27 amino acid residues (Figures 1C and 2C; SEQ ID NOs: 52-78) were joined either to the N-terminus of the heavy chain variable (VH) region of the anti-CD3 antibody SP34 (Figure 1A) or to the N-terminus of the VH region of a second, different anti-CD3 antibody variant (Figure 2A). In addition, polypeptide masks having MMs ranging from the 1 to 27 amino acid residues were similarly joined to either the N-terminus of the light chain variable (VL) region of SP34 (Figure 1B) or to the N-terminus of the VL region of a second, different anti-CD3 antibody variant (Figure 2B). Each of the constructs, generated in a phagemid vector and displayed monovalently on phage, contained a thrombin cleavage site (i.e., a cleavable moiety (CM)) between the MM component and N-terminus of either antibody variable domain. The binding to CD3ε was assessed using a phage ELISA, as described above.

For masked SP34 antibodies, inhibition of CD3ε binding was observed when the VH region was joined to a polypeptide mask having a masking moiety containing only the first 5 residues of processed human CD3ε via a thrombin cleavage site (Figure 1A). In general, longer polypeptide masks were necessary to block CD3ε binding when the mask was joined to the VL region of the SP34 antibody variant (Figure 1B). For masked variants of the second, different anti-CD3 antibody, the polypeptide mask having a masking moiety containing only the first 5 residues of processed human CD3ε joined to either the VL or VH region of the anti-CD3 antibody variant significantly blocked binding to CD3ε (Figures 2A and 2B).

For all masked variants, CD3ε binding was re-established following treatment with thrombin. In general, longer polypeptide masks were removed more effectively, suggesting that longer polypeptide masks support more efficient thrombin cleavage.

### Example 3. Effects of Polypeptide Mask Content on Inhibition

To determine the minimal content of N-terminal CD3ε sequence of the MM needed to inhibit phage-displayed anti-CD3 antibody binding to CD3ε, the total polypeptide mask length joined to the VH region of an anti-CD3 antibody was held constant at 27 amino acids by the inclusion of a GS linker moiety (LM), and the number of CD3ε residues was systematically reduced (Figure 3B; SEQ ID NOs: 79-88). As shown in Figure 3A, the first 6 residues of processed human CD3ε are sufficient to fully block CD3ε binding, Intermediate degrees of inhibition were observed using masks containing only the first 3 to 5 residues of processed human CD3ε (Figure 3A). The binding to CD3ε by all variants was restored upon treatment with thrombin (Figure 3A).

### Example 4. Effects of Masking on CD3/CD20 TDB (CD20 TDB) Biodistribution and Efficacy

The biodistribution of a bispecific antibody directed at 2 different target cell populations *in vivo* will be dependent upon the affinity and accessibility of the bispecific antibody for each target. For TDBs targeting solid tumors, the accessibility of the CD3ε on T cells is much higher than that of antigens presented on solid tumors due to the limited ability of IgG to penetrate tumors. Given that both arms of a bispecific are present at equal concentration, one approach that could shift biodistribution of the TDB towards the tumor would be to adjust the affinity of each arm of the bispecific so that the affinity for the tumor antigen is much higher than for CD3ε. However, the potential affinity ratio that can be obtained between the tumor antigen affinity and the CD3ε binding affinity has practical limits. Very high affinities to the target are sometimes difficult to attain, and very low affinity to CD3ε may reduce the potency of the TDB.

A polypeptide mask that attenuates CD3ε binding has distinct advantages and offers a novel approach to altering biodistribution. Rather than removing the polypeptide mask by proteolysis or other means to after CD3 binding inhibition and biodistribution, a controlled change in the inhibition of CD3 binding by an anti-CD3 antibody can also be established by use of a "fixed polypeptide mask." Fixed polypeptide masks, such as those shown in Figures 4B (SEQ ID NOs: 89-94) and Figures 4C (SEQ ID NOs: 95-99), do not contain a CM and cannot be readily removed from the anti-CD3 antibodies to which they are joined. As shown in Figure 4A, CD3ε binding by the anti-CD3 antibody can be specifically attenuated to any desired degree by using fixed polypeptide masks having different overall lengths. By selecting an appropriate length and content, CD3ε binding can be inhibited anywhere from 0 to 100%, which is akin to changing the affinity ratio from 1 to infinity. Further, the effect of this approach has a specific and direct impact on the association rate (Ka), as the active concentration of anti-CD3 antibody is effectively reduced by the mask, which is in equilibrium between a bound and unbound state that is controlled by the length and content of the mask. The polypeptide mask slows the rate of the anti-CD3 antibody binding to CD3ε, which results in a decreased ka.

To assess the effects of a polypeptide mask in the context of a TDB. CD3/CD20 TDBs (CD20 TDBs) were generated having one anti-CD20 arm and one anti-CD3 arm, which was joined at its VH region with a fixed 12- to 16-aa polypeptide mask (Figure 4B). The heavy chains of the generated masked CD20 TDBs were designed as follows.
12aa = anti-CD3 antibody 7-5: QDGNEEMGGSGG (SEQ ID NO: 100)-anti-CD3 antibody heavy chain
14aa = anti-CD3 antibody 7-7: QDGNEEMGGSGGSG (SEQ ID NO: 101)-anti-CD3 antibody heavy chain
15aa = anti-CD3 antibody 7-8: QDGNEEMGGSGGSGG (SEQ ID NO: 102)-anti-CD3 antibody heavy chain
16aa = anti-CD3 antibody 7-9: QDGNEEMGGSGGSGGS (SEQ ID NO: 103)-anti-CD3 antibody heavy chain
The masked CD20 TDBs were produced as full-length antibodies in knob-into-hole format as previously described (see, e.g., Atwell et al. J. Mol Biol. 270: 26-35, 1997 and U.S.S.N. 14/574,132 (U.S. Pub. No. 2015-0166661)) and have the general structure depicted in Figure 4D (except that the polypeptide masks of the CD20 TDBs joined at the VH region rather than the VL region).

These fixed masked CD20 TDBs were subsequently tested for efficacy in *in vitro* B cell killing and T cell activation assays compared to an unmasked CD20 TDB, as described above. Reduced CD3ε binding due to the presence of the fixed mask resulted in attenuation of T-cell dependent endogenous B-cell killing *in vitro,* with longer overall mask length resulting in a greater degree of attenuation of B cell killing *in vitro* (Figure 4E).

Additional CD20 TDBs joined at their VH regions with fixed 9- to 12-aa polypeptide masks of varied MM and LM lengths were also generated (Figure 4C). The heavy chains of these generated masked CD20 TDBs were designed as follows.
Masked 3.9 = QDGSGGGSGGGS (SEQ ID NO: 95)-anti-CD3 antibody heavy chain
Masked 4.5 = QDGNSGGGS (SEQ ID NO: 96)-anti-CD3 antibody heavy chain
Masked 4.6 = QDGNSGGGSG (SEQ ID NO: 97)-anti-CD3 antibody heavy chain
Masked 5.7 = QDGNESGGGSGG (SEQ ID NO: 98)-anti-CD3 antibody heavy chain
Masked 6.6 = QDGNEESGGGSG (SEQ ID NO: 99)-anti-CD3 antibody heavy chain
These masked CD20 TDBs were produced as full-length antibodies in knob-into-hole format as described above.

These fixed masked CD20 TDBs were also tested for efficacy in *in vitro* B cell killing and T cell activation assays compared to an unmasked CD20 TDB control, as described above. In general, longer MM length resulted in greater attenuation of CD8⁺ T cell activation (Figure 4F) and B cell killing *in vitro* (Figure 4G). Consistent with the results obtained from the other CD20 TDBs tested in Figures 4D and 4E, CD20 TDBs with longer overall mask length (e.g., by virtue of a longer LM) also resulted in a greater degree of attenuation of CD8⁺ T cell activation and B cell killing *in vitro* (compare, e.g., masked 4.5 and 4.6 CD20 TDBs).

Masked TDBs were further assayed for their binding affinity for recombinant antigen single chain CQ3εγ heterodimer (Kim, et al. J. Mol. Biol, 302: 899-916,2000), The binding affinities of masked 4.5, masked 4.6, and masked 5.7 CD3/CD20 TDBs were compared to those of four affinity variant unmasked CD3/CD20 TDBs, unmasked v1 (SEQ ID NO: 18 (VH) and SEQ ID NO: 19 (VL)), unmasked v3 (SEQ ID NO: 107 (VH) and SEQ ID NO:108 (VL)), unmasked v4 (SEQ ID NO: 109 (VH) and SEQ ID NO:110(VL)), and unmasked v5 (SEQ ID NO: 111 (VH) and SEQ ID NO: 112(VL)). In this assay, a masked or affinity variant CD3/CD20 TDB was immobilized on a Biacore CM5 Series S chip through amine coupling using an anti-human IgG (Fc) antibody capture kit (GE Healthcare). Recombinant CD3εγ antigen was passed over captured CD3/CD20 TDBs in a concentration series of two-fold dilutions from 0.39 nM to 500 nM, prepared in HBSP running buffer, pH 7.4. Each binding cycle was followed by a regeneration step using 10 mM Glycine, pH 1.7. The binding response was corrected by subtracting the binding signal from a blank flow cell. Affinity values were calculated by Biacore T200 BIAevaluation software using a 1:1 Languir model of simultaneous fitting of kₒₙ and k_{off}.

When a masked CD3/CD20 TDB variant is compared with an unmasked CD3/CD20 TDB affinity variant of the same overall affinity (K_{D}), masked versions of CD3/CD20 TDBs have a slower off-rate. In general, as the affinity of each masked CD3/CD20 TDB variant decreases, the off-rate remains constant. For unmasked CD3/CD20 TDB affinity variants, however, a decrease in affinity is reflected in the off-rate. The results summarized in the table in Figure 4H support the hypothesis that the mask lowers the active concentration of the anti-CD3 binding moiety, represented by a decrease in association rate (kₐ), which is a concentration dependent variable. The decrease in kₐ leads to the CD3/CD20 TDB preferentially binding to the target antigen (in this instance, CD20) before CD3 molecules on T cells. The kinetic features of the masked versions are favored for localized cell-killing activity. Slower on-rate lessens the probability of non-specific binding and activation of T cells, while a slower off-rate allows more time for cell-cell bridging contact and target specific cytotoxicity.

A comparison of T cell activation and T cell-mediated cytotoxicity for unmasked CD3/CD20 TDB affinity variants (unmasked v1, unmasked v3, unmasked v4, unmasked v5) and masked CD3/CD20 TDB variants (masked 4.5, masked 4,6, masked 5.7) reflects the desired kinetics for masked CD3/CD20 TDBs. Masked CD3/CD20 TDBs had lower levels of T cell activation when compared with unmasked CD3/CD20 affinity variants of comparable K_{D}. Additionally, unmasked CD3/CD20 TDB affinity variants exhibited a greater loss in cell killing activity associated with decreased affinity, while lower affinity masked CD3/CD20 TDBs maintained better cell killing activity with a reduced level of T cell activation (Figure 4I).

Collectively, the data demonstrate that masked anti-CD3 antibodies, such as masked TDBs, are able to uniquely alter the cellular biodistribution of engaged target cells and T cells to control the efficacy of the TDBs. In addition to varying the length and content of the polypeptide mask, activation of the masked anti-CD3 antibodies can also be regulated by the incorporation of a CM in the mask that allows for its removal in an environment-dependent manner (see, e.g., WO 2012/025525). Suitable proteolytic sites designed to take advantage of up-regulated protease activity in the tumor microenvironment, for example, have been described (Lopez-Otin et al. Nat Rev Cancer. 7:800-808. 2007).

### Other Embodiments

Although the foregoing invention has been described in some detail by way of illustration and example for purposes of clarity of understanding, the descriptions and examples should not be construed as limiting the scope of the invention. The disclosures of all patent and scientific literature cited herein are expressly incorporated in their entirety by reference.

The following numbered paragraphs (paras) contain further statements of various aspects of the present invention:
1. An anti-cluster of differentiation 3 (CD3) antibody, wherein the anti-CD3 antibody comprises (a) a binding domain and (b) a polypeptide mask, wherein the polypeptide mask comprises a masking moiety (MM) comprising the amino acid sequence of at least amino acid residues 1-3 of SEQ ID NO: 1.
2. The anti-CD3 antibody of para 1, wherein the binding domain comprises a heavy chain variable (VH) domain and a light chain variable (VL) domain and the polypeptide mask is joined to the VH domain or the VL domain.
3. The anti-CD3 antibody of para 1 or 2, wherein the MM is extended at the C-terminus by all or a portion of the remaining sequence of SEQ ID NO: 1.
4. The anti-CD3 antibody of any one of paras 1-3, wherein the MM comprises the amino acid sequence of at least amino acid residues 1-5 of SEQ ID NO: 1.
5. The anti-CD3 antibody of para 4, wherein the MM comprises the amino acid sequence of at least amino acid residues 1-6 of SEQ ID NO:1.
6. The anti-CD3 antibody of any one of paras 1-5, wherein the anti-CD3 antibody and MM are positioned relative to each other in an N-terminal to C-terminal direction as (MM)-(anti-CD3 antibody).
7. The anti-CD3 antibody of any one of paras 1-6, wherein the polypeptide mask further comprises a cleavable moiety (CM).
8. The anti-CD3 antibody of para 7, wherein the CM is capable of being cleaved by an enzyme.
9. The anti-CD3 antibody of para 8, wherein the enzyme is selected from the group consisting of matrix metalloprotease (MMP)-2, MMP-9, legumain asparaginyl endopeptidase, thrombin, fibroblast activation protease (FAP), MMP-1, MMP-3, MMP-7, MMP-8, MMP-12, MMP-13, MMP-14, membrane type 1 matrix metalloprotease (MT1-MMP), plasmin, transmembrane protease, serine (TMPRSS-3/4), cathepsin A, cathepsin B, cathepsin D, cathepsin E, cathepsin F, cathepsin H, cathepsin K, cathepsin L, cathepsin L2, cathepsin O, cathepsin S, caspase 1, caspase 2. caspase 3, caspase 4, caspase 5, caspase 6, caspase 7, caspase 8, caspase 9, caspase 10, caspase 11, caspase 12, caspase 13, caspase 14, human neutrophil elastase, urokinase/urokinase-type plasminogen activator (uPA), a disintegrin and metalloprotease (ADAM)10, ADAM12, ADAM17, ADAM with thrombospondin motifs (ADAMTS), ADAMTS5, beta secretase (BACE), granzyme A, granzyme B, guanidinobenzoatase, hepsin, matriptase, matriptase 2, meprin. neprilysin, prostate-specific membrane antigen (PSMA), tumor necrosis factor-converting enzyme (TACE), kallikrein-related peptidase (KLK)3, KLK5, KLK7, KLK11, NS3/4 protease of hepatitis C virus (HCV-NS3/4), tissue plasminogen activator (tPA), calpain, calpain 2, glutamate carboxypeptidase II, plasma kallikrein, AMSH-like protease, AMSH, γ-secretase component, antiplasmin cleaving enzyme (APCE), decysin 1, apoptosis-related cysteine peptidase, and N-acetylated alpha-linked acidic dipeptidase-like 1.
10. The anti-CD3 antibody of para 9, wherein the enzyme is MMP-2, MMP-9, legumain asparaginyl endopeptidase, thrombin, or FAP.
11. The anti-CD3 antibody of any one of paras 7-10, wherein the anti-CD3 antibody, MM, and CM are positioned relative to each other in an N-terminal to C-terminal direction as (MM)-(CM)-(anti-CD3 antibody).
12. The anti-CD3 antibody of any one of paras 1-6, wherein the polypeptide mask further comprises a linker moiety (LM).
13. The anti-CD3 antibody of para 12, wherein the LM is between 5 to 24 amino acids in length.
14. The anti-CD3 antibody of para 13, wherein the LM is between 5 to 15 amino acids in length.
15. The anti-CD3 antibody of any one of para 12-14, wherein the LM comprises glycine (G) and serine (S) residues.
16. The anti-CD3 antibody of para 15, wherein the LM comprises GS repeats.
17. The anti-CD3 antibody of any one of paras 12-16, wherein the anti-CD3 antibody, MM, and LM are positioned relative to each other in an N-terminal to C-terminal direction as (MM)-(LM)-(anti-CD3 antibody).
18. The anti-CD3 antibody of any one of paras 7-17, wherein the polypeptide mask comprises a cleavable moiety and a linker moiety, and wherein the anti-CD3 antibody, MM, CM, and LM are positioned relative to each other in an N-terminal to C-terminal direction as (MM)-(LM)-(CM)-(anti-CD3 antibody) or (MM)-(CM)-(LM)-(anti-CD3 antibody).
19. The anti-CD3 antibody of any one of paras 1-18, wherein the binding domain comprises the following six hypervariable regions (HVRs):
   (a) an HVR-H1 comprising the amino acid sequence of SEQ ID NO: 2;
   (b) an HVR-H2 comprising the amino acid sequence of SEQ ID NO: 3;
   (c) an HVR-H3 comprising the amino acid sequence of SEQ ID NO: 4;
   (d) an HVR-L1 comprising the amino acid sequence of SEQ ID NO: 5;
   (e) an HVR-L2 comprising the amino acid sequence of SEQ ID NO: 6; and
   (f) an HVR-L3 comprising the amino acid sequence of SEQ ID NO: 7.
20. The anti-CD3 antibody of para 19, wherein the binding domain comprises (a) a VH domain comprising an amino acid sequence having at least 95% sequence identity to the amino acid sequence of SEQ ID NO: 8; (b) a VL domain comprising an amino acid sequence having at least 95% sequence identity to the amino acid sequence of SEQ ID NO: 9; or (c) a VH domain as in (a) and a VL domain as in (b).
21. The anti-CD3 antibody of para 20, wherein the VH domain comprises the amino acid sequence of SEQ ID NO: 8.
22. The anti-CD3 antibody of para 20, wherein the VL domain comprises the amino acid sequence of SEQ ID NO: 9.
23. The anti-CD3 antibody of paras 21 or 22, wherein VH domain comprises the amino acid sequence of SEQ ID NO: 8 and the VL domain comprises the amino acid sequence of SEQ ID NO: 9.
24. The anti-CD3 antibody of any one of paras 1-18, wherein the binding domain comprises the following six HVRs:
   (a) an HVR-H1 comprising the amino acid sequence of SEQ ID NO: 10;
   (b) an HVR-H2 comprising the amino acid sequence of SEQ ID NO: 11;
   (c) an HVR-H3 comprising the amino acid sequence of X₁X₂YSX₃X₄X₅FDY, wherein X₁ is selected from the group consisting of D, T, and S; X₂ is selected from the group consisting of G, A, and S; X₃ is R or N; X₄ is Y or A; and X₅ is Y or A (SEQ ID NO: 12);
   (d) an HVR-L1 comprising the amino acid sequence of SEQ ID NO: 13;
   (e) an HVR-L2 comprising the amino acid sequence of SEQ ID NO: 14; and
   (f) an HVR-L3 comprising the amino acid sequence of X₁X₂SX₃X₄LRT, wherein X₁ is K or T; X₂ is Q or A; X₃ is F or A; and X₄ is I or A (SEQ ID NO: 15).
25. The anti-CD3 antibody of para 24, wherein the binding domain comprises the following six HVRs:
   (a) an HVR-H1 comprising the amino acid sequence of SEQ ID NO: 10;
   (b) an HVR-H2 comprising the amino acid sequence of SEQ ID NO: 11;
   (c) an HVR-H3 comprising the amino acid sequence of SEQ ID NO: 16;
   (d) an HVR-L1 comprising the amino acid sequence of SEQ ID NO: 13;
   (e) an HVR-L2 comprising the amino acid sequence of SEQ ID NO: 14; and
   (f) an HVR-L3 comprising the amino acid sequence of SEQ ID NO: 17.
26. The anti-CD3 antibody of para 25, wherein the binding domain comprises (a) a VH domain comprising an amino acid sequence having at least 95% sequence identity to the amino acid sequence of SEQ ID NO: 18; (b) a VL domain comprising an amino acid sequence having at least 95% sequence identity to the amino acid sequence of SEQ ID NO: 19; or (c) a VH domain as in (a) and a VL domain as in (b).
27. The anti-CD3 antibody of para 26, wherein the VH domain comprises the amino acid sequence of SEQ ID NO: 18.
28. The anti-CD3 antibody of para 26, wherein the VL domain comprises the amino acid sequence of SEQ ID NO: 19.
29. The anti-CD3 antibody of paras 27 or 28, wherein VH domain comprises the amino acid sequence of SEQ ID NO: 18 and the VL domain comprises the amino acid sequence of SEQ ID NO: 19.
30. The anti-CD3 antibody of para 24, wherein the binding domain comprises the following six HVRs:
   (a) an HVR-H1 comprising the amino acid sequence of SEQ ID NO: 10;
   (b) an HVR-H2 comprising the amino acid sequence of SEQ ID NO: 11;
   (c) an HVR-H3 comprising the amino acid sequence of SEQ ID NO: 16;
   (d) an HVR-L1 comprising the amino acid sequence of SEQ ID NO: 13;
   (e) an HVR-L2 comprising the amino acid sequence of SEQ ID NO: 14; and
   (f) an HVR-L3 comprising the amino acid sequence of SEQ ID NO: 104.
31. The anti-CD3 antibody of para 30, wherein the binding domain comprises (a) a VH domain comprising an amino acid sequence having at least 95% sequence identity to the amino acid sequence of SEQ ID NO: 107; (b) a VL domain comprising an amino acid sequence having at least 95% sequence identity to the amino acid sequence of SEQ ID NO: 108; or (c) a VH domain as in (a) and a VL domain as in (b).
32. The anti-CD3 antibody of para 31, wherein the VH domain comprises the amino acid sequence of SEQ ID NO: 107.
33. The anti-CD3 antibody of para 31, wherein the VL domain comprises the amino acid sequence of SEQ ID NO: 108.
34. The anti-CD3 antibody of paras 32 or 33, wherein VH domain comprises the amino acid sequence of SEQ ID NO: 107 and the VL domain comprises the amino acid sequence of SEQ ID NO: 108.
35. The anti-CD3 antibody of para 24, wherein the binding domain comprises the following six HVRs:
   (a) an HVR-H1 comprising the amino acid sequence of SEQ ID NO: 10;
   (b) an HVR-H2 comprising the amino acid sequence of SEQ ID NO: 11;
   (c) an HVR-H3 comprising the amino acid sequence of SEQ ID NO: 16;
   (d) an HVR-L1 comprising the amino acid sequence of SEQ ID NO: 13;
   (e) an HVR-L2 comprising the amino acid sequence of SEQ ID NO: 14; and
   (f) an HVR-L3 comprising the amino acid sequence of SEQ ID NO: 105.
36. The anti-CD3 antibody of para 35, wherein the binding domain comprises (a) a VH domain comprising an amino acid sequence having at least 95% sequence identity to the amino acid sequence of SEQ ID NO: 109; (b) a VL domain comprising an amino acid sequence having at least 95% sequence identity to the amino acid sequence of SEQ ID NO: 110; or (c) a VH domain as in (a) and a VL domain as in (b).
37. The anti-CD3 antibody of para 36, wherein the VH domain comprises the amino acid sequence of SEQ ID NO: 109.
38. The anti-CD3 antibody of para 36, wherein the VL domain comprises the amino acid sequence of SEQ ID NO: 110.
39. The anti-CD3 antibody of para 37 or 38, wherein VH domain comprises the amino acid sequence of SEQ ID NO: 109 and the VL domain comprises the amino acid sequence of SEQ ID NO: 110.
40. The anti-CD3 antibody of para 24, wherein the binding domain comprises the following six HVRs:
   (a) an HVR-H1 comprising the amino acid sequence of SEQ ID NO: 10;
   (b) an HVR-H2 comprising the amino acid sequence of SEQ ID NO: 11;
   (c) an HVR-H3 comprising the amino acid sequence of SEQ ID NO: 16;
   (d) an HVR-L1 comprising the amino acid sequence of SEQ ID NO: 13;
   (e) an HVR-L2 comprising the amino acid sequence of SEQ ID NO: 14; and
   (f) an HVR-L3 comprising the amino acid sequence of SEQ ID NO: 106.
41. The anti-CD3 antibody of para 40, wherein the binding domain comprises (a) a VH domain comprising an amino acid sequence having at least 95% sequence identity to the amino acid sequence of SEQ ID NO: 111; (b) a VL domain comprising an amino acid sequence having at least 95% sequence identity to the amino acid sequence of SEQ ID NO: 112; or (c) a VH domain as in (a) and a VL domain as in (b).
42. The anti-CD3 antibody of para 41, wherein the VH domain comprises the amino acid sequence of SEQ ID NO: 111.
43. The anti-CD3 antibody of para 41, wherein the VL domain comprises the amino acid sequence of SEQ ID NO: 112.
44. The anti-CD3 antibody of paras 42 or 43, wherein VH domain comprises the amino acid sequence of SEQ ID NO: 111 and the VL domain comprises the amino acid sequence of SEQ ID NO: 112.
45. The anti-CD3 antibody of any one of paras 1-44, wherein the polypeptide mask inhibits the ability of the anti-CD3 antibody to bind to a human CD3 polypeptide by at least 10%.
46. The anti-CD3 antibody of para 45, wherein the polypeptide mask inhibits the ability of the anti-CD3 antibody to bind to a human CD3 polypeptide by at least 30%.
47. The anti-CD3 antibody of para 46, wherein the polypeptide mask inhibits the ability of the anti-CD3 antibody to bind to a human CD3 polypeptide by at least 60%.
48. The anti-CD3 antibody of para 47, wherein the polypeptide mask inhibits the ability of the anti-CD3 antibody to bind to a human CD3 polypeptide by at least 80%.
49. The anti-CD3 antibody of para 48, wherein the polypeptide mask inhibits the ability of the anti-CD3 antibody to bind to a human CD3 polypeptide by at least 90%.
50. The anti-CD3 antibody of any one of paras 45-49, wherein the human CD3 polypeptide is a human CD3ε polypeptide.
51. The anti-CD3 antibody of any one of paras 1-50, wherein the anti-CD3 antibody comprises an aglycosylation site mutation,
52. The anti-CD3 antibody of para 51, wherein the aglycosylation site mutation is a substitution mutation.
53. The anti-CD3 antibody of para 52, wherein the substitution mutation is at amino acid residue N297, L234, L235, and/or D265 (EU numbering).
54. The anti-CD3 antibody of para 53, wherein the substitution mutation is selected from the group consisting of N297G, N297A, L234A, L235A, and D265A.
55. The anti-CD3 antibody of para 54, wherein the substitution mutation is an N297G mutation.
56. The anti-CD3 antibody of para 55, wherein the aglycosylation site mutation reduces effector function of the anti-CD3 antibody.
57. The anti-CD3 antibody of any one of paras 1-56, wherein the anti-CD3 antibody is monoclonal, human, humanized, or chimeric.
58. The anti-CD3 antibody of any one of paras 1-57, wherein the anti-CD3 antibody is an antibody fragment that binds CD3.
59. The anti-CD3 antibody of para 58, wherein the antibody fragment is selected from the group consisting of Fab, Fab', Fab'-SH, (Fab')2, Fv, scFv, TaFv, diabody, bsDb, scDb, DART, BiTE, and V_{H}H fragments.
60. The anti-CD3 antibody of any one of paras 1-57, wherein the anti-CD3 antibody is a full-length antibody.
61. The anti-CD3 antibody of any one of paras 1-60, wherein the anti-CD3 antibody is an IgG antibody.
62. The anti-CD3 antibody of any one of paras 1-61, wherein the anti-CD3 antibody is a monospecific antibody.
63. The anti-CD3 antibody of any one of paras 1-61, wherein the anti-CD3 antibody is a multispecific antibody.
64. The anti-CD3 antibody of para 63, wherein the multispecific antibody is a bispecific antibody.
65. The anti-CD3 antibody of para 64, wherein the bispecific antibody comprises a second binding domain that binds to a second biological molecule, wherein the second biological molecule is a cell surface antigen.
66. The anti-CD3 antibody of para 65, wherein the cell surface antigen is a tumor antigen.
67. The anti-CD3 antibody of para 66, wherein the tumor antigen is selected from the group consisting of CD20; FcRH5 (Fc Receptor-like 5): HER2; LYPD1; Ly6G6D (lymphocyte antigen 6 complex, locus G61); Ly6-D, MEGT1); PMEL17 (silver homolog; SILV; D12S53E; PMEL17; (SI); (SIL); ME20; gp100); Ly6E (lymphocyte antigen 6 complex, locus E; Ly67, RIG-E, SCA-2, TSA-1); CD19; CD33; CD22 (B-cell receptor CD22-B isoform); CD79a (CD79A, CD79a, immunoglobulin-associated alpha; BMPR1B (bone morphogenetic protein receptor-type IB); CD79b (CD79B, CD79P. 1 Gb (immunoglobulin-associated beta), B29); EDAR (Ectodysplasin A Receptor); GFRA1 (GDNF-Ra1); MRP4 (Multidrug Resistance Protein 4); RET; STEAP1 (six transmembrane epithelial antigen of prostate); TENB2 (putative transmembrane proteoglycan); E16 (LAT1, SLC7A5); 0772P (CA125, MUC16); MPF (MPF, MSLN, SMR, megakaryocyte potentiating factor, mesothelin); Napi3b (NAPI-3B, NPTIIb, SLC34A2, solute carrier family 34 (sodium phosphate), member 2, type II sodium-dependent phosphate transporter 3b); Sema 5b; PSCA hlg (2700050C12Rik, C530008O16Rik, RIKEN cDNA 2700050C12, RIKEN cDNA 2700050C12 gene); ETBR (Endothelin type B receptor); MSG783 (RNF124, hypothetical protein FLJ20315); STEAP2; TrpM4 (BR22450, FLJ20041, TRPM4, TRPM4B, transient receptor potential cation channel, subfamily M, member 4); CRIPTO (CR, CR1, CRGF, CRIPTO, TDGF1, teratocarcinoma-derived growth factor); CD21 (CR2 (Complement receptor 2) or C3DR (C3d/Epstein Barr virus receptor) or Hs.73792); FcRH2 (IFGP4, IRTA4, SPAP1A (SH2 domain containing phosphatase anchor protein 1a), SPAP1B, SPAP1C); NCA; MDP; IL20Rα: Brevican; EphB2R; ASLG659; PSCA; GEDA; BAFF-R (B cell-activating factor receptor, BLyS receptor 3, BR3); CXCR5 (Burkitt's lymphoma receptor 1; HLA-DOB (Beta subunit of MHC class II molecule); P2X5 (Purinergic receptor P2X ligand-gated ion channel 5; CD72 (B-cell differentiation antigen CD72, Lyb-2); LY64 (Lymphocyte antigen 64 (RP105), type I membrane protein of the leucine rich repeat (LRR) family); FcRH1 (Fc receptor-iike protein 1); IRTA2 (Immunoglobulin superfamily receptor translocation associated 2); TMEFF1; TMEM46 (shisa homolog 2 (Xenopus laevis); SHISA2); LGR5 (leucine-rich repeat-containing G protein-coupled receptor 5; GPR49, GPR67); LY6K (lymphocyte antigen 6 complex, locus K; LY6K; HSJ001348; FLJ35226); GPR19 (G protein-coupled receptor 19; Mm 4787); GPR54 (KISS1 receptor; KSSS1R; GPR54; HOT7T175; AXOR12); ASPHD1 (aspartate beta-hydroxylase domain containing 1; LOC253982); Tyrosinase (TYR; OCAIA; OCA1A; tyrosinase; SHEP3); TMEM118 (ring finger protein, transmembrane 2; RNFT2; FLJ14627); GPR172A (G protein-coupled receptor 172A; GPCR41; FLJ11856; D15Ertd747e); GPC3 (Glypican 3); CLL1 (C-Type Lectin-like molecule 1); B7-H4 (B7x; B7S1); RNF43 (Ring finger protein 43); CD70; CXORF61 (Chromosome X open reading frame 61); and SLC53D3.
68. The anti-CD3 antibody of para 67, wherein the tumor antigen is selected from the group consisting of CD20, FcRH5, HER2, LYPD1, LY6G6D, PMEL17, LY6E, CD19, CD33, CD22, CD79A, CD79B, EDAR, GFRA1, MRP4, RET, Steap1, and TenB2.
69. The anti-CD3 antibody of para 68, wherein the tumor antigen is CD20 and the second binding domain comprises the following six HVRs:
   (a) an HVR-H1 comprising the amino acid sequence of SEQ ID NO: 20;
   (b) an HVR-H2 comprising the amino acid sequence of SEQ ID NO: 21;
   (c) an HVR-H3 comprising the amino acid sequence of SEQ ID NO: 22;
   (d) an HVR-L1 comprising the amino acid sequence of SEQ ID NO: 23;
   (e) an HVR-L2 comprising the amino acid sequence of SEQ ID NO: 24; and
   (f) an HVR-L3 comprising the amino acid sequence of SEQ ID NO: 25.
70. The anti-CD3 antibody of para 69, wherein the binding domain comprises (a) a VH domain comprising an amino acid sequence having at least 95% sequence identity to the amino acid sequence of SEQ ID NO: 26; (b) a VL domain comprising an amino acid sequence having at least 95% sequence identity to the amino acid sequence of SEQ ID NO: 27; or (c) a VH domain as in (a) and a VL domain as in (b).
71. The anti-CD3 antibody of para 70, wherein the VH domain comprises the amino acid sequence of SEQ ID NO: 26.
72. The anti-CD3 antibody of para 70, wherein the VL domain comprises the amino acid sequence of SEQ ID NO: 27.
73. The anti-CD3 antibody of para 71 or 72, wherein VH domain comprises the amino acid sequence of SEQ ID NO: 26 and the VL domain comprises the amino acid sequence of SEQ ID NO: 27.
74. The anti-CD3 antibody of any one of paras 1-73, wherein the anti-CD3 antibody comprises one or more heavy chain constant domains, wherein the one or more heavy chain constant domains are selected from a first CH1 (CH1*₁*) domain, a first CH2 (CH2*₁*) domain, a first CH3 (CH3*₁*) domain, a second CH1 (CH1*₂*) domain, second CH2 (CH2*₂*) domain, and a second CH3 (CH3*₂*) domain.
75. The anti-CD3 antibody of para 74, wherein at least one of the one or more heavy chain constant domains is paired with another heavy chain constant domain.
76. The anti-CD3 antibody of para 75, wherein the CH3*₁* and CH3*₂* domains each comprise a protuberance or cavity, and wherein the protuberance or cavity in the CH3*₁* domain is positionable in the cavity or protuberance, respectively, in the CH3*₂* domain.
77. The anti-CD3 antibody of any one of paras 74-76, wherein the CH2*₁* and CH2*₂* domains each comprise a protuberance or cavity, and wherein the protuberance or cavity in the CH2*₁* domain is positionable in the cavity or protuberance, respectively, in the CH2*₂* domain.
78. The anti-CD3 antibody of para 77, wherein the CH2*₁* and CH2*₂* domains meet at an interface between said protuberance and cavity.
79. An isolated nucleic acid encoding the anti-CD3 antibody of any one of paras 1-78.
80. A vector comprising the isolated nucleic acid of para 79.
81. A host cell comprising the vector of para 80.
82. The host cell of para 81, wherein the host cell is a mammalian cell.
83. The host cell of para 82, wherein the mammalian cell is a Chinese hamster ovary (CHO) cell.
84. The host cell of para 81, wherein the host cell is a prokaryotic cell.
85. The host cell of para 84, wherein the prokaryotic cell is *E*. *coli.*
86. A method of producing the anti-CD3 antibody of any one of paras 1-78, the method comprising culturing the host cell of para 81 in a culture medium.
87. The method of para 86, wherein the method further comprises recovering the anti-CD3 antibody from the host cell or the culture medium.
88. An immunoconjugate comprising the anti-CD3 antibody of any one of paras 1-78 and a cytotoxic agent.
89. A composition comprising the anti-CD3 antibody of any one of paras 1-78 or the immunoconjugate of para 88.
90. The composition of para 89, further comprising a pharmaceutically acceptable carrier, excipient, or diluent.
91. The composition of para 90, wherein the composition is a pharmaceutical composition.
92. The composition of any one of paras 89-91, wherein the composition further comprises an additional therapeutic agent
93. A method of treating or delaying the progression of a cell proliferative disorder or an autoimmune disorder in a subject in need thereof, the method comprising administering to the subject the anti-CD3 antibody of any one of paras 1-78.
94. A method of enhancing immune function in a subject having a cell proliferative disorder or an autoimmune disorder, the method comprising administering to the subject an effective amount of the anti-CD3 antibody of any one of paras 1-78.
95. The method of para 93 or 94, wherein the cell proliferative disorder is a cancer.
96. The method of para 95, wherein the cancer is selected from the group consisting of breast cancer, bladder cancer, colorectal cancer, non-small cell lung cancer, non-Hodgkin's lymphoma (NHL), B cell lymphoma, B cell leukemia, multiple myeloma, renal cancer, prostate cancer, liver cancer, head and neck cancer, melanoma, ovarian cancer, mesothelioma, and glioblastoma.
97. The method of para 96, wherein the B cell leukemia is chronic lymphoid leukemia (CLL).
98. The method of para 93 or 94, wherein the autoimmune disorder is selected from the group consisting of rheumatoid arthritis, juvenile rheumatoid arthritis, systemic lupus erythematosus (SLE), Wegener's disease, inflammatory bowel disease, idiopathic thrombocytopenic purpura (ITP), thrombotic thrombocytopenic purpura (TTP), autoimmune thrombocytopenia, multiple sclerosis, psoriasis, IgA nephropathy, IgM polyneuropathies, myasthenia gravis, vasculitis, diabetes mellitus, Reynaud's syndrome, Sjögren's syndrome, glomerulonephritis, Neuromyelitis Optica (NMO) and IgG neuropathy.
99. The method of any one of paras 93-98, wherein the anti-CD3 antibody binds to (a) a CD3 molecule located on an immune effector cell and (b) a second biological molecule located on a target cell other than the immune effector cell.
100. The method of para 99, wherein the anti-CD3 antibody binds to the second biological molecule prior to the CD3 molecule.
101. The method of para 100, wherein the anti-CD3 antibody accumulates at the surface of the target cell.
102. The method of any one of paras 93-101, wherein the anti-CD3 antibody is capable of providing a cytotoxic effect and/or an apoptotic effect on the target cell.
103. The method of para 102, wherein the cytotoxic effect and/or the apoptotic effect on the target cell is independent of activation of the immune effector cell.
104. The method of para 102, wherein the cytotoxic effect and/or the apoptotic effect on the target cell is dependent of activation of the immune effector cell.
105. The method of any one of paras 93-104, wherein the anti-CD3 antibody is administered to the subject in a dosage of about 0.01 mg/kg to about 30 mg/kg.
106. The method of para 105, wherein the anti-CD3 antibody is administered to the subject in a dosage of about 0.1 mg/kg to about 30 mg/kg.
107. The method of para 106, wherein the anti-CD3 antibody is administered to the subject in a dosage of about 1 mg/kg to about 30 mg/kg.
108. The method of any one of paras 93-107, further comprising administering to the subject a PD-1 axis binding antagonist or an additional therapeutic agent.
109. The method of para 108, wherein the PD-1 axis binding antagonist or additional therapeutic agent is administered prior to or subsequent to the administration of the anti-CD3 antibody.
110. The method of para 108, wherein the PD-1 axis binding antagonist additional therapeutic agent is administered concurrently with the anti-CD3 antibody.
111. The method of any one of paras 108-110, wherein the PD-1 axis binding antagonist is selected from the group consisting of a PD-1 binding antagonist, a PD-L1 binding antagonist, and a PD-L2 binding antagonist.
112. The method of para 111, wherein the PD-1 axis binding antagonist is a PD-1 binding antagonist.
113. The method of para 112, wherein the PD-1 binding antagonist is selected from the group consisting of MDX-1106 (nivolumab), MK-3475 (lambrolizumab), CT-011 (pidilizumab), and AMP-224.
114. The method of para 111, wherein the PD-1 axis binding antagonist is a PD-L1 binding antagonist.
115. The method of para 114, wherein the PD-L1 binding antagonist is selected from the group consisting of: YW243.55.S70, MPDL3280A, MDX-1105. and MEDI4736.
116. The method of para 111, wherein the PD-1 axis binding antagonist is a PD-L2 binding antagonist.
117. The method of para 116, wherein the PD-L2 binding antagonist is an antibody or an immunoadhesin.
118. The method of any one of paras 93-117, further comprising administering to the subject a glucocorticoid,
119. The method of para 118, wherein the glucocorticoid is dexamethasone,
120. The method of any one of paras 93-119, further comprising administering to the subject rituximab.
121. The method of any one of paras 93-120, wherein the anti-CD3 antibody is administered subcutaneously, intravenously, intramuscularly, topically, orally, transdermally, intraperitoneally, intraorbitally, by implantation, by inhalation, intrathecally, intraventricularly, or intranasally.
122. The method of para 121, wherein the anti-CD3 antibody is administered subcutaneously.
123. The method of para 121, wherein the anti-CD3 antibody is administered intravenously.
124. The method of any one of paras 93-123, wherein the subject is a human.
125. A kit comprising:
   (a) the composition of any one of paras 89-92; and
   (b) a package insert comprising instructions for administering the composition to a subject to treat or delay progression of a cell proliferative disorder or an autoimmune disorder.

## Claims

1. A multispecific antibody comprising an anti-cluster of differentiation 3 (CD3) binding domain that binds to CD3ε and a polypeptide mask, wherein the polypeptide mask comprises
a masking moiety (MM) including an amino acid sequence comprising an N-terminal fragment of a human CD3ε polypeptide, wherein said amino acid sequence is at least the first three amino acids of a processed human CD3ε polypeptide (amino acid residues 1-3 of SEQ ID NO: 1) and has a maximum length of 27 amino acids; wherein said masking moiety reduces or inhibits the ability of the binding domain to specifically bind CD3ε, and
a linker moiety (LM),
wherein the binding domain comprises a heavy chain variable (VH) domain and a light chain variable (VL) domain and the polypeptide mask is joined to the VH domain or the VL domain; and
wherein the MM, LM and the binding domain are joined relative to each other in an N-terminal to C-terminal direction as (MM)-(LM)-(binding domain).

2. The multispecific antibody of claim 1 wherein b) the MM is extended at the C-terminus by all or a portion of the remaining sequence of SEQ ID NO: 1.

3. The multispecific antibody of claim 1 or 2, wherein the polypeptide mask further comprises a cleavable moiety (CM), wherein the binding domain, MM, CM and LM are positioned relative to each other in an N-terminal to C-terminal direction as (MM)-(LM)-(CM)-(binding domain).

4. The multispecific antibody of claim 3, wherein the CM is capable of being cleaved by an enzyme; optionally wherein the enzyme is selected from the group consisting of matrix metalloprotease (MMP)-2, MMP-9, legumain asparaginyl endopeptidase, thrombin, fibroblast activation protease (FAP), MMP-1, MMP-3, MMP-7, MMP-8, MMP-12, MMP-13, MMP-14, membrane type 1 matrix metalloprotease (MT1-MMP), plasmin, transmembrane protease, serine (TMPRSS-3/4), cathepsin A, cathepsin B, cathepsin D, cathepsin E, cathepsin F, cathepsin H, cathepsin K, cathepsin L, cathepsin L2, cathepsin O, cathepsin S, caspase 1, caspase 2, caspase 3, caspase 4, caspase 5, caspase 6, caspase 7, caspase 8, caspase 9, caspase 10, caspase 11, caspase 12, caspase 13, caspase 14, human neutrophil elastase, urokinase/urokinase-type plasminogen activator (uPA), a disintegrin and metalloprotease (ADAM)10, ADAM12, ADAM17, ADAM with thrombospondin motifs (ADAMTS), ADAMTS5, beta secretase (BACE), granzyme A, granzyme B, guanidinobenzoatase, hepsin, matriptase, matriptase 2, meprin, neprilysin, prostate-specific membrane antigen (PSMA), tumor necrosis factor-converting enzyme (TACE), kallikrein-related peptidase (KLK)3, KLK5, KLK7, KLK11, NS3/4 protease of hepatitis C virus (HCV-NS3/4), tissue plasminogen activator (tPA), calpain, calpain 2, glutamate carboxypeptidase II, plasma kallikrein, AMSH-like protease, AMSH, γ-secretase component, antiplasmin cleaving enzyme (APCE), decysin 1, apoptosis-related cysteine peptidase, and N-acetylated alpha-linked acidic dipeptidase-like 1; preferably wherein the enzyme is MMP-2, MMP-9, legumain asparaginyl endopeptidase, thrombin, or FAP.

5. The multispecific antibody of any one of claims 1-4, wherein:
(a) the LM is between 5 to 24 amino acids in length or between 5 to 15 amino acids in length; and/or
(b) the LM comprises glycine (G) and serine (S) residues.

6. The multispecific antibody of claim 5, wherein the LM comprises glycine serine (GS) repeats.

7. The multispecific antibody of any one of claims 1-6, wherein the binding domain comprises the following six hypervariable regions (HVRs):
(a) an HVR-H1 comprising the amino acid sequence of SEQ ID NO: 2;
(b) an HVR-H2 comprising the amino acid sequence of SEQ ID NO: 3;
(c) an HVR-H3 comprising the amino acid sequence of SEQ ID NO: 4;
(d) an HVR-L1 comprising the amino acid sequence of SEQ ID NO: 5;
(e) an HVR-L2 comprising the amino acid sequence of SEQ ID NO: 6; and
(f) an HVR-L3 comprising the amino acid sequence of SEQ ID NO: 7.

8. The multispecific antibody of claim 7, wherein the binding domain comprises (a) a VH domain comprising an amino acid sequence having at least 95% sequence identity to the amino acid sequence of SEQ ID NO: 8; (b) a VL domain comprising an amino acid sequence having at least 95% sequence identity to the amino acid sequence of SEQ ID NO: 9; or (c) a VH domain as in (a) and a VL domain as in (b).

9. The multispecific antibody of any one of claims 1-8, wherein the polypeptide mask inhibits the ability of the anti-CD3 antibody to bind to a human CD3ε polypeptide by at least 10%, at least 30%, at least 60%, at least 80%, or at least 90%.

10. The multispecific antibody of any one of claims 1-9, wherein the multispecific antibody comprises an aglycosylation site mutation.

11. The multispecific antibody of any one of claims 1-9, wherein the multispecific antibody comprises a substitution mutation at amino acid residue N297, L234, L235, and/or D265 (EU numbering), and/or (b) reduces effector function of the multispecific antibody.

12. The multispecific antibody of any one of claims 1-11, wherein the anti-CD3 antibody is one or more of:
(a) a monoclonal antibody, a humanized antibody, or a chimeric antibody;
(b) an antibody fragment that binds CD3ε; (c) a full-length antibody; and
(d) an IgG antibody.

13. The multispecific antibody of any one of claims 1-12, wherein the multispecific antibody is a bispecific antibody comprising a second binding domain that binds to a second biological molecule, wherein the second biological molecule is a cell surface antigen.

14. The multispecific antibody of claim 13, wherein the cell surface antigen is a tumor antigen selected from the group consisting of CD20; FcRH5 (Fc Receptor-like 5); HER2; LYPD1; Ly6G6D (lymphocyte antigen 6 complex, locus G61, Ly6-D, MEGT1); PMEL17 (silver homolog; SILV; D12S53E; PMEL17; (SI); (SIL); ME20; gp100); Ly6E (lymphocyte antigen 6 complex, locus E; Ly67, RIG-E, SCA-2, TSA-1); CD19; CD33; CD22 (B-cell receptor CD22-B isoform); CD79a (CD79A, CD79a, immunoglobulin-associated alpha; BMPR1B (bone morphogenetic protein receptor-type IB); CD79b (CD79B, CD79β, 1 Gb (immunoglobulin-associated beta), B29); EDAR (Ectodysplasin A Receptor); GFRA1 (GDNF-Ra1); MRP4 (Multidrug Resistance Protein 4); RET; STEAP1 (six transmembrane epithelial antigen of prostate); TENB2 (putative transmembrane proteoglycan); E16 (LAT1, SLC7A5); 0772P (CA125, MUC16); MPF (MPF, MSLN, SMR, megakaryocyte potentiating factor, mesothelin); Napi3b (NAPI-3B, NPTIIb, SLC34A2, solute carrier family 34 (sodium phosphate), member 2, type II sodium-dependent phosphate transporter 3b); Sema 5b; PSCA hlg (2700050C12Rik, C530008016Rik, RIKEN cDNA 2700050C12, RIKEN cDNA 2700050C12 gene); ETBR (Endothelin type B receptor); MSG783 (RNF124, hypothetical protein FLJ20315); STEAP2; TrpM4 (BR22450, FLJ20041, TRPM4, TRPM4B, transient receptor potential cation channel, subfamily M, member 4); CRIPTO (CR, CR1, CRGF, CRIPTO, TDGF1, teratocarcinoma-derived growth factor); CD21 (CR2 (Complement receptor 2) or C3DR (C3d/Epstein Barr virus receptor) or Hs.73792); FcRH2 (IFGP4, IRTA4, SPAP1A (SH2 domain containing phosphatase anchor protein 1a), SPAP1B, SPAP1C); NCA; MDP; IL20Rα; Brevican; EphB2R; ASLG659; PSCA; GEDA; BAFF-R (B cell-activating factor receptor, BLyS receptor 3, BR3); CXCR5 (Burkitt's lymphoma receptor 1; HLA-DOB (Beta subunit of MHC class II molecule); P2X5 (Purinergic receptor P2X ligand-gated ion channel 5; CD72 (B-cell differentiation antigen CD72, Lyb-2); LY64 (Lymphocyte antigen 64 (RP105), type I membrane protein of the leucine rich repeat (LRR) family); FcRH1 (Fc receptor-like protein 1); IRTA2 (Immunoglobulin superfamily receptor translocation associated 2); TMEFF1; TMEM46 (shisa homolog 2 (Xenopus laevis); SHISA2); LGR5 (leucine-rich repeat-containing G protein-coupled receptor 5; GPR49, GPR67); LY6K (lymphocyte antigen 6 complex, locus K; LY6K; HSJ001348; FLJ35226); GPR19 (G protein-coupled receptor 19; Mm 4787); GPR54 (KISS1 receptor; KISS1R; GPR54; HOT7T175; AXOR12); ASPHD1 (aspartate beta-hydroxylase domain containing 1; LOC253982); Tyrosinase (TYR; OCAIA; OCA1A; tyrosinase; SHEP3); TMEM118 (ring finger protein, transmembrane 2; RNFT2; FLJ14627); GPR172A (G protein-coupled receptor 172A; GPCR41; FLJ11856; D15Ertd747e); GPC3 (Glypican 3); CLL1 (C-Type Lectin-like molecule 1); B7-H4 (B7x; B7S1); RNF43 (Ring finger protein 43); CD70; CXORF61 (Chromosome X open reading frame 61); and SLC53D3; preferably wherein the tumor antigen is selected from the group consisting of CD20, FcRH5, HER2, LYPD1, LY6G6D, PMEL17, LY6E, CD19, CD33, CD22, CD79A, CD79B, EDAR, GFRA1, MRP4, RET, Steap1, and TenB2.

15. The multispecific antibody of claim 14, wherein the tumor antigen is CD20 and the second binding domain comprises the following six HVRs:
(a) an HVR-H1 comprising the amino acid sequence of SEQ ID NO: 20;
(b) an HVR-H2 comprising the amino acid sequence of SEQ ID NO: 21;
(c) an HVR-H3 comprising the amino acid sequence of SEQ ID NO: 22;
(d) an HVR-L1 comprising the amino acid sequence of SEQ ID NO: 23;
(e) an HVR-L2 comprising the amino acid sequence of SEQ ID NO: 24; and
(f) an HVR-L3 comprising the amino acid sequence of SEQ ID NO: 25.

16. The multispecific antibody of claim 15, wherein the binding domain comprises (a) a VH domain comprising an amino acid sequence having at least 95% sequence identity to the amino acid sequence of SEQ ID NO: 26; (b) a VL domain comprising an amino acid sequence having at least 95% sequence identity to the amino acid sequence of SEQ ID NO: 27; or (c) a VH domain as in (a) and a VL domain as in (b).

17. The multispecific antibody of any one of claims 1-16, wherein the multispecific antibody comprises one or more heavy chain constant domains, wherein the one or more heavy chain constant domains are selected from a first CH1 (CH1*₁*) domain, a first CH2 (CH2*₁*) domain, a first CH3 (CH3*₁*) domain, a second CH1 (CH1*₂*) domain, second CH2 (CH2*₂*) domain, and a second CH3 (CH3*₂*) domain, preferably wherein:
(a) at least one of the one or more heavy chain constant domains is paired with another heavy chain constant domain; and/or wherein the CH3*₁* and CH3*₂* domains each comprise a protuberance or cavity, wherein the protuberance or cavity in the CH3*₁* domain is positionable in the cavity or protuberance, respectively, in the CH3*₂* domain; and/or
(b) the CH2*₁* and CH2*₂* domains each comprise a protuberance or cavity, and wherein the protuberance or cavity in the CH2*₁* domain is positionable in the cavity or protuberance, respectively, in the CH2*₂* domain, and optionally wherein the CH2*₁* and CH2*₂* domains meet at an interface between said protuberance and cavity.

18. An isolated nucleic acid encoding the multispecific antibody of any one of claims 1-17.

19. A vector comprising the isolated nucleic acid of claim 18.

20. A host cell comprising the vector of claim 19, wherein the host cell is a mammalian cell, or wherein the host is a prokaryotic cell.

21. A method of producing the multispecific antibody of any one of claims 1-17, the method comprising culturing the host cell of claim 20 in a culture medium.

22. An immunoconjugate comprising the multispecific antibody of any one of claims 1-17 and a cytotoxic agent.

23. A composition comprising the multispecific antibody of any one of claims 1-17 or the immunoconjugate of claim 22, wherein the composition comprises a pharmaceutically acceptable carrier, excipient, or diluent.

24. The composition of claim 23, wherein the composition further comprises an additional therapeutic agent.

25. The multispecific antibody of any one of claims 1 to 17 for use as a medicament.

26. The multispecific antibody of any one of claims 1-17 for use in a method of:
(a) treating or delaying the progression of a cell proliferative disorder or an autoimmune disorder in a subject in need thereof; or
(b) enhancing immune function in a subject having a cell proliferative disorder or an autoimmune disorder.

27. The multispecific antibody for use of claim 26, wherein the cell proliferative disorder is a cancer; optionally wherein the cancer is selected from the group consisting of breast cancer, bladder cancer, colorectal cancer, non-small cell lung cancer, non-Hodgkin's lymphoma (NHL), B cell lymphoma, B cell leukemia, multiple myeloma, renal cancer, prostate cancer, liver cancer, head and neck cancer, melanoma, ovarian cancer, mesothelioma, and glioblastoma, preferably wherein the B cell leukemia is chronic lymphoid leukemia (CLL); or wherein the autoimmune disorder is selected from the group consisting of rheumatoid arthritis, juvenile rheumatoid arthritis, systemic lupus erythematosus (SLE), Wegener's disease, inflammatory bowel disease, idiopathic thrombocytopenic purpura (ITP), thrombotic thrombocytopenic purpura (TTP), autoimmune thrombocytopenia, multiple sclerosis, psoriasis, IgA nephropathy, IgM polyneuropathies, myasthenia gravis, vasculitis, diabetes mellitus, Reynaud's syndrome, Sjögren's syndrome, glomerulonephritis, Neuromyelitis Optica (NMO), and IgG neuropathy.

28. The multispecific antibody for use of claim 26 or 27, wherein the multispecific antibody is formulated to be administered to the subject in a dosage of about 0.01 mg/kg to about 30 mg/kg, about 0.1 mg/kg to about 30 mg/kg, or about 1 mg/kg to about 30 mg/kg; and/or wherein the anti-CD3 antibody is formulated to be administered with a PD-1 axis binding antagonist or an additional therapeutic agent.

29. The multispecific antibody for use of claim 28, wherein the PD-1 axis binding antagonist is:
(a) a PD-1 binding antagonist;
(b) a PD-L1 binding antagonist; or
(c) a PD-L2 binding antagonist.

30. The multispecific antibody for use of claim 29, wherein the PD-1 axis binding antagonist is:
(a) the PD-1 binding antagonist is MDX-1106 (nivolumab), MK-3475 (lambrolizumab), or AMP-224;
(b) the PD-L1 binding antagonist is MPDL3280A, MDX-1105, or MEDI4736; or
(c) the PD-L2 binding antagonist is an antibody or an immunoadhesin.

31. The multispecific antibody for use of any one of claims 26-30, wherein the multispecific antibody is formulated to be administered to the subject in combination with a glucocorticoid and/or rituximab.

32. The multispecific antibody for use of claim 31, wherein the glucocorticoid is dexamethasone.

33. The multispecific antibody of any one of claims 26-31, wherein the multispecific antibody is formulated to be administered subcutaneously, intravenously, intramuscularly, topically, orally, transdermally, intraperitoneally, intraorbitally, by implantation, by inhalation, intrathecally, intraventricularly, or intranasally.

34. A kit comprising:
(a) the composition of claim 23; and
(b) a package insert comprising instructions for administering the composition to a subject to treat or delay progression of a cell proliferative disorder or an autoimmune disorder.
